# EUROPÄISCHE PATENTSCHRIFT

(11) **EP 2 900 644 B1**
(45) Veröffentlichungstag und Bekanntmachung des Hinweises auf die Patenterteilung: **21.09.2016**
(21) Anmeldenummer: 13766517.0
(22) Anmeldetag: 23.09.2013
(51) Int. Cl.: C07D 261/04, C07D 413/12, A01N 43/80

(54) **HERBIZID UND FUNGIZID WIRKSAME 5-OXY-SUBSTITUIERTE 3-PHENYLISOXAZOLIN-5-CARBOXAMIDE UND 5-OXY-SUBSTITUIERTE 3-PHENYLISOXAZOLIN-5-THIOAMIDE**
HERBICIDAL AND FUNGICIDAL 5-OXY-SUBSTITUTED 3-PHENYLISOXAZOLIN-5-CARBOXAMIDES AND 5-OXY-SUBSTITUTED 3-PHENYLISOXAZOLIN-5-THIOAMIDES
3-PHÉNYLISOXAZOLINE-5-CARBOXAMIDES 5-OXY-SUBSTITUÉS ET 3-PHÉNYLISOXAZOLINE-5-THIOAMIDES 5-OXY-SUBSTITUÉS À ACTION HERBICIDE ET FONGICIDE

(30) Priorität: 25.09.2012 EP 12185767
(43) Veröffentlichungstag der Anmeldung: 05.08.2015
(73) Patentinhaber: Bayer CropScience AG, 40789 Monheim am Rhein (DE)
(72) Erfinder: HAAF, Klaus Bernhard, 65779 Kelkheim (DE); WILLMS, Lothar, 65719 Hofheim (DE); DIETRICH, Hansjörg, 65835 Liederbach am Taunus (DE); GATZWEILER, Elmar, 61231 Bad Nauheim (DE); ROSINGER, Christopher Hugh, 65719 Hofheim (DE); SCHMUTZLER, Dirk, 65795 Hattersheim (DE); WACHENDORFF-NEUMANN, Ulrike, 56566 Neuwied (DE); GROSJEAN-COURNOYER, Marie-Claire, F-69250 Curis au Mont d'Or (FR); LACHAISE, Helene, F-69009 Lyon (FR); RINOLFI, Philippe, F-69380 Chatillon d'Azergues (FR); BRUNET, Stephane, F-01390 St Andre de Corcy (FR)
(74) Vertreter: BIP Patents
(86) Internationale Anmeldenummer: PCT/EP2013/069737
(87) Internationale Veröffentlichungsnummer: WO 2014/048882

(56) Entgegenhaltungen:
- EP-A2- 0 174 685
- WO-A1-92/03053
- WO-A1-95/14680
- WO-A1-2005/021516
- WO-A2-98/57937
- PRIYA B S ET AL: "DELTA.2-Isoxazoline derivatives as antimicrobials", HETEROCYCLIC COMMUNICATIONS, FREUND PUBLISHING HOUSE, TEL AVIV, Bd. 12, Nr. 1, 1. Januar 2006 (2006-01-01) , Seiten 35-42, XP009140872, ISSN: 0793-0283
- MIROSAA AW GUCMA ET AL: "Synthesis and biological activity of 3-substituted isoxazolecarboxamides", MONATSHEFTE FÜR CHEMIE - CHEMICAL MONTHLY ; AN INTERNATIONAL JOURNAL OF CHEMISTRY, SPRINGER-VERLAG, AU, Bd. 141, Nr. 4, 23. Februar 2010 (2010-02-23), Seiten 461-469, XP019853437, ISSN: 1434-4475
- GUCMA MIROSLAW ET AL: "Synthesis and fungicidal activity of substituted isoxazolecarboxamides", PESTYCYDY = PESTICIDES : QUARTERLY OF THE INSTITUTE OF INDUSTRIAL ORGANIC CHEMISTRY, INSTYTUT PRZEMYS U ORGANICZNEGO <WARSCHAU> , Bd. 2010, Nr. 1-4 1. Januar 2011 (2011-01-01), Seiten 21-31, XP008159982, ISSN: 0208-8703 Gefunden im Internet: URL:http://www.wydawnictwa.ipo.waw.pl/pest ycydy/Pestycydy1-4-2010/Gucma.pdf

## Beschreibung

Die Erfindung betrifft das technische Gebiet der Herbizide und Fungizide, insbesondere das der Herbizide zur selektiven Bekämpfung von Unkräutern und Ungräsern in Nutzpflanzenkulturen.

Speziell betrifft sie substituierte 5-oxy-substituierte 3-Phenylisoxazolin-5-carboxamide und 5-oxy-substituierte 3-Phenyl-isoxazolin-5-thioamide, Verfahren zu ihrer Herstellung und ihre Verwendung als Herbizide und Fungizide.

Aus DE 4026018 A1, EP 0 520 371 A2 und DE 4017665 sind 3-Phenylisoxazolin-5-carboxamide bekannt, die in 5-Position des Isoxazolin-Rings ein Wasserstoffatom tragen. Diese Verbindungen werden dort als agrochemisch wirksame Safener beschrieben, d.h. als Verbindungen, die die unerwünschte herbizide Wirkung von Herbiziden gegenüber Kulturpflanzen aufheben. Eine herbizide Wirkung dieser Verbindungen ist nicht offenbart. Die prioritätsältere nicht vorveröffentlichte europäische Patentanmeldung Nr. 10170238 offenbart herbizid und fungizid wirksame 3-Phenylisoxazolin-5-carboxamide und 3-Phenylisoxazolin-5-thioamide, die in 5-Position des Isoxazolin-Rings ein Wasserstoffatom tragen. Aus Monatshefte Chemie (2010) 141, 461 und Letters in Organic Chemistry (2010), 7, 502 sind ebenfalls 3-Phenylisoxazolin-5-carboxamide bekannt, die in 5-Position des Isoxazolin-Rings ein Wasserstoffatom tragen. Für einige der genannten Verbindungen wird eine fungizide, jedoch keine herbizide Wirkung offenbart.

Aufgabe der vorliegenden Erfindung ist die Bereitstellung herbizid und fungizid wirksamer Verbindungen.

Es wurde gefunden, daß 5-oxy-substituierte 3-Phenylisoxazolin-5-carboxamide und 5-oxy-substituierte 3-Phenylisoxazolin-5-thioamide als Herbizide und Fungizide besonders gut geeignet sind. Ein Gegenstand der vorliegenden Erfindung sind 5-oxy-substituierte 3-Phenylisoxazolin-5-carboxamide und 5-oxy-substituierte 3-Phenylisoxazolin-5-thioamide der Formel (I), oder deren Salze 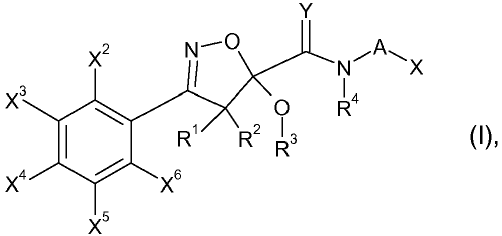 worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy,
   oder
R¹ und R² bilden gemeinsam mit dem Kohlenstoffatom, an das sie gebunden sind, einen gesättigten, teilweise oder vollständig ungesättigten drei-, vier- oder fünfgliedrigen Ring, der aus q Kohlenstoffatomen und p Sauerstoffatomen aufgebaut ist;
R³ bedeutet durch jeweils m Reste aus der Gruppe bestehend aus Fluor, Chlor, Brom, Iod, Cyano, (C₁-C₄)-Alkoxy und Hydroxy substituiertes (C₁-C₆)-Alkyl, (C₃-C₆)-Cycloalkyl, (C₂-C₆)-Alkenyl oder (C₂-C₆)-Alkinyl;
R⁴ bedeutet Wasserstoff, Cyano,
oder durch jeweils m Reste aus der Gruppe bestehend aus Fluor, Chlor, Brom, Iod, Cyano, Hydroxy und (C₁-C₆)-Alkoxy substituiertes (C₁-C₈)-Alkyl, (C₃-C₈)-Cycloalkyl, (C₃-C₈)-Alkenyl oder (C₃-C₈)-Alkinyl,

A bedeutet eine Bindung oder eine divalente Einheit aus der Gruppe bestehend aus 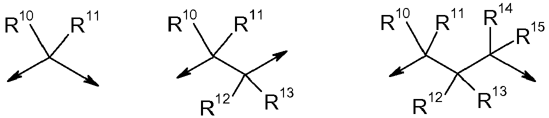 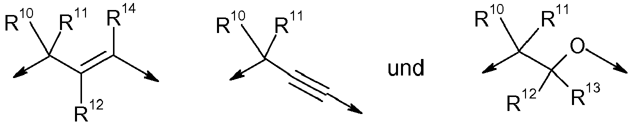
R¹⁰, R¹¹, R¹², R¹³, R¹⁴ und R¹⁵ bedeuten unabhängig voneinanderjeweils Wasserstoff, Fluor, Chlor, Brom, Iod, Hydroxy, Cyano, CO₂R⁸, CONR⁶R⁸, R⁵,
   oder jeweils durch m Reste aus der Gruppe bestehend aus Fluor, Chlor, Brom, Iod, Hydroxy und Cyano substituiertes (C₁-C₆)-Alkyl, (C₃-C₅)-Cyc!oa!ky!, (C₂-C₆)-Alkenyl, (C₂-C₆)-Alkinyl,
   oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
Y bedeutet Sauerstoff oder Schwefel;
X bedeutet Wasserstoff, Cyano, Hydroxy, X¹,
   oder
   durch jeweils m Reste aus der Gruppe bestehend aus Fluor, Chlor, Brom, Iod, Cyano, Hydroxy, OR⁷, X¹, OX¹, NHX¹, S(O)ₙ R⁵, SO₂NR⁶R⁷, SO₂NR⁶COR⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ und C(R⁶)=NOR⁸ substituiertes (C₁-C₁₂)-Alkyl, (C₃-C₈)-Cycloalkyl, (C₂-C₁₂)-Alkenyl oder (C₂-C₁₂)-Alkinyl,
   oder
X, A und R⁴ bilden mit dem Stickstoffatom, an das sie gebunden sind, einen gesättigten, teilweise oder vollständig ungesättigten fünf-, sechs- oder siebengliedrigen Ring, der neben diesem Stickstoffatom k Kohlenstoffatome, n Sauerstoffatome, p Schwefelatome und p Elemente aus der Gruppe bestehend aus NR⁷ und NCOR⁷ als Ringatome enthält, wobei ein Kohlenstoffatom p Oxogruppen trägt;
X¹ bedeutet einen drei, vier, fünf- oder sechsgliedrigen gesättigten, teilweise ungesättigten, vollständig ungesättigten oder aromatischen Ring, der aus r Kohlenstoffatomen, s Stickstoffatomen, n Schwefelatomen und n Sauerstoffatomen aufgebaut ist, und der durch s Reste aus der Gruppe bestehend aus R⁶, R^{6a}, R⁸ und R⁹ substituiert ist, wobei die Schwefelatome und Kohlenstoffatome n Oxogruppen tragen;
   oder X¹ bedeutet durch m Reste aus der Gruppe bestehend aus R⁶, R^{6a}, R⁸ und R⁹ substituiertes Phenyl;
X², X⁴ und X⁶ bedeuten unabhängig voneinander jeweils Wasserstoff, Fluor, Chlor, Brom, Iod, Cyano, Nitro,
   oder jeweils durch m Reste aus der Gruppe bestehend aus Fluor, Chlor, Brom Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Alkylcarbonyl;
X³ und X⁵ bedeuten unabhängig voneinander Wasserstoff, Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸ NR⁶SO₂R⁸, NR⁶SO₂NR⁶R⁸, OCONR⁶R⁸, OSO₂R⁵, S(O)ₙR⁵, SO₂NR⁶R⁸, OSO₂NR⁶R⁸,
   oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
   oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₃-C₆)-Alkenyloxy oder (C₃-C₆)-Alkinyloxy;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷,
R⁹ bedeutet (C₁-C₃)-Alkyl oder (C₁-C₃)-Alkoxy,
k bedeutet 3, 4, 5 oder 6;
m bedeutet 0, 1, 2, 3, 4 oder 5;
n bedeutet 0, 1 oder 2;
p bedeutet 0 oder 1;
q bedeutet 3, 4 oder 5;
r bedeutet 1, 2, 3, 4 oder 5;
s bedeutet 0, 1, 2, 3 oder 4.

Alkyl bedeutet gesättigte, geradkettige oder verzweigte Kohlenwasserstofifreste mit der jeweils angegebenen Anzahl von Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.

Durch Halogen substitiertes Alkyl bedeutet geradkettige oder verzweigte Alkylgruppen, wobei in diesen Gruppen teilweise oder vollständig die Wasserstoffatome durch Halogenatome ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl.

Alkenyl bedeutet ungesättigte, geradkettige oder verzweigte Kohlenwasserstoffreste mit der jeweils angegebenen Anzahl von Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Alkinyl bedeutet geradkettige oder verzweigte Kohlenwasserstoffreste mit der jeweils angegebenen Anzahl von Kohlenstoffatomen und einer Dreifachbindung in einer beliebigen Position, z.B. C₂-C₆-Alkinyl wie Ethinyl, 1-Propinyl, 2-Propinyl (oder Propargyl), 1-Butinyl, 2-Butinyl, 3-Butinyl, 1-Methyl-2-propinyl, 1-Pentinyl, 2-Pentinyl, 3-Pentinyl, 4-Pentinyl, 3-Methyl-1-butinyl, 1-Methyl-2-butinyl, 1-Methyl-3-butinyl, 2-Methyl-3-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 3-Methyl-1-pentinyl, 4-Methyl-1-pentinyl, 1-Methyl-2-pentinyl, 4-Methyl-2-pentinyl, 1-Methyl-3-pentinyl, 2-Methyl-3-pentinyl, 1-Methyl-4-pentinyl, 2-Methyl-4-pentinyl, 3-Methyl-4-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl.

Alkoxy bedeutet gesättigte, geradkettige oder verzweigte Alkoxyreste mit der jeweils angegebenen Anzahl von Kohlenstoffatomen, z.B. C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Dimethylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Durch Halogen substitiertes Alkoxy bedeutet geradkettige oder verzweigte Alkoxyreste mit der jeweils angegebenen Anzahl von Kohlenstoffatomen, wobei in diesen Gruppen teilweise oder vollständig die Wasserstoffatome durch Halogenatome wie vorstehend genannt ersetzt sein können, z.B. C₁-C₂-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlor-fluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor-1,2-difluorethoxy, 2,2-Dichlor-2-fluorethoxy, 2,2,2-Trichlorethoxy, Pentafluor-ethoxy und 1,1,1-Trifluorprop-2-oxy.

Die Verbindungen der Formel (I) können je nach Art und Verknüpfung der Substituenten als Stereoisomere vorliegen. Sind beispielsweise ein oder mehrere asymmetrisch substiuierte Kohlenstoffatome und/oder Sulfoxide vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der Formel (I) umfaßt, jedoch nicht spezifisch definiert sind. Im Folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Je nach Art der oben definierten Substituenten weisen die Verbindungen der Formel (I) saure Eigenschaften auf und können mit anorganischen oder organischen Basen oder mit Metallionen Salze, gegebenenfalls auch innere Salze oder Addukte bilden. Tragen die Verbindungen der Formel (I) Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄-)-Alkyl-Gruppen, Mono-, Di- und Trialkanolamine von (C₁-C₄)-Alkanolen, Cholin sowie Chlorcholin.

Ist eine Gruppe mehrfach durch Reste substituiert, so bedeutet dies, daß diese Gruppe durch einen oder mehrere gleiche oder verschiedene der genannten Reste substituiert ist.

In allen nachfolgend genannten Formeln haben die Substituenten und Symbole, sofern nicht anders definiert, dieselbe Bedeutung wie unter Formel (I) beschrieben. Pfeile in einer chemischen Formel bedeuten die Verknüpfungsorte zum restlichen Molekül.

Bevorzugt sind 5-oxy-substituierte 3-Phenylisoxazolin-5-carboxamide und 5-oxy-substituierte 3-Phenyl-isoxazolin-5-thioamide der Formel (I), worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy,
oder
R¹ und R² bilden gemeinsam mit dem Kohlenstoffatom, an das sie gebunden sind, einen gesättigten, teilweise oder vollständig ungesättigten drei-, vier- oder fünfgliedrigen Ring, der aus q Kohlenstoffatomen und p Sauerstoffatomen aufgebaut ist;
R³ bedeutet durch jeweils m Reste aus der Gruppe bestehend aus Fluor, Chlor, Brom, Iod, Cyano, (C₁-C₄)-Alkoxy und Hydroxy substituiertes (C₁-C₆)-Alkyl, (C₃-C₆)-Cycloalkyl, (C₂-C₆)-Alkenyl oder (C₂-C₆)-Alkinyl,
R⁴ bedeutet Wasserstoff, Cyano,
oder durch jeweils m Reste aus der Gruppe bestehend aus Fluor, Chlor, Brom, Iod, Cyano, Hydroxy und (C₁-C₆)-Alkoxy substituiertes (C₁-C₈)-Alkyl oder (C₃-C₈)-Cycloalkyl;

A bedeutet eine Bindung oder eine divalente Einheit aus der Gruppe bestehend aus 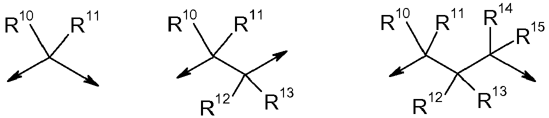 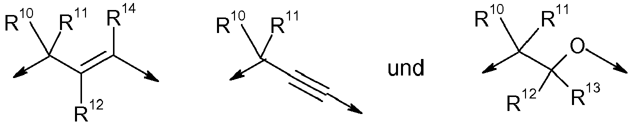
R¹⁰, R¹¹, R¹², R¹³, R¹⁴ und R¹⁵ bedeuten unabhängig voneinanderjeweils Wasserstoff, Fluor, Chlor, Brom, Iod, Hydroxy, Cyano, CO₂R⁸, CONR⁶R⁸, R⁵, oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl, oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
Y bedeutet Sauerstoff oder Schwefel;
X bedeutet Wasserstoff, Cyano, Hydroxy, X¹,
   oder
   durch jeweils m Reste aus der Gruppe bestehend aus Fluor, Chlor, Brom, Iod, Cyano, Hydroxy, OR⁷,X¹, OX¹, NHX¹, S(O)ₙ R⁵, SO₂NR⁶R⁷, SO₂NCOR⁶R⁸, CO₂R⁸, CONR⁶R⁸, COR⁶, C0NR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ und C(R⁶)=NOR⁸ substituiertes (C₁-C₁₂)-Alkyl, (C₃-C₈)-Cycloalkyl, (C₂-C₁₂)-Alkenyl oder (C₂-C₁₂)-Alkinyl,
   oder
X, A und R⁴ bilden mit dem Stickstoffatom, an das sie gebunden sind, einen gesättigten, teilweise oder vollständig ungesättigten fünf-, sechs- oder siebengliedrigen Ring, der neben diesem Stickstoffatom k Kohlenstoffatome, n Sauerstoffatome, p Schwefelatome und p Elemente aus der Gruppe bestehend aus NR⁷ und NCOR⁷ als Ringatome enthält, wobei ein Kohlenstoffatom p Oxogruppen trägt;
X¹ bedeutet einen durch s Reste aus der Gruppe bestehend aus R⁶, R^{6a}, R⁸ und R⁹ substituierten Ring aus der Gruppe bestehend aus 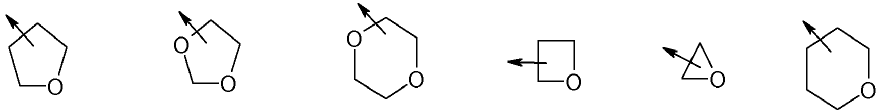 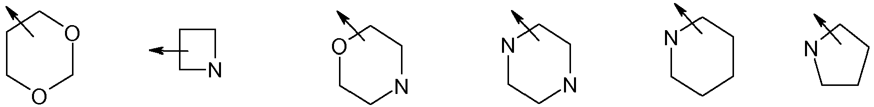 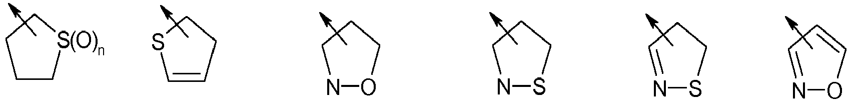 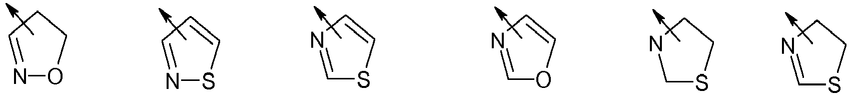 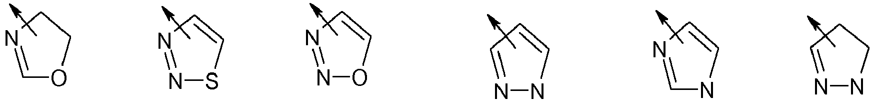 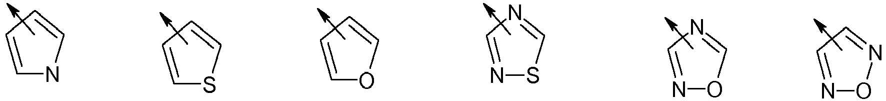 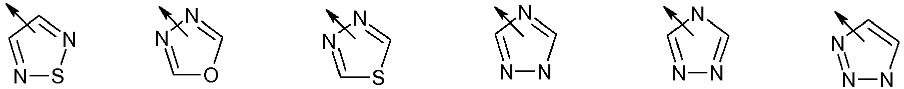 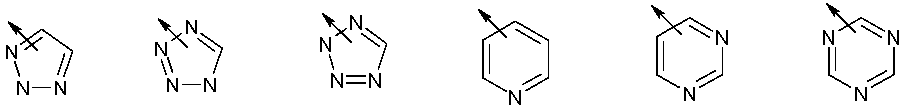 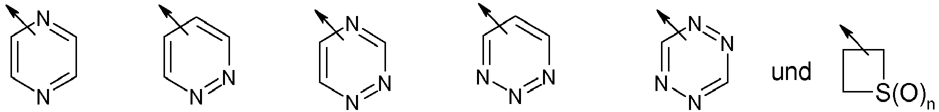 oder X¹ bedeutet durch m Reste aus der Gruppe bestehend aus R⁶, R^{6a}, R⁸ und R⁹ substituiertes Phenyl;
X², X⁴ und X⁶ bedeuten unabhängig voneinander jeweils Wasserstoff, Fluor, Chlor, Brom, Iod, Cyano, Nitro,
   oder jeweils durch m Reste aus der Gruppe bestehend aus Fluor, Chlor, Brom, Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Alkylcarbonyl;
X³ und X⁵ bedeuten unabhängig voneinander Wasserstoff Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸ NR⁶SO₂R⁸, NR⁶SO₂NR⁶R⁸, OCONR⁶R⁸, OSO₂R⁵, S(O)ₙR⁵, SO₂NR⁶R⁸, OSO₂NR⁶R⁸,
   oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
   oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₂-C₆)-Alkenyloxy oder (C₂-C₆)-Alkinyloxy;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷,
R⁹ bedeutet (C₁-C₃)-Alkyl oder (C₁-C₃)-Alkoxy,
k bedeutet 3, 4, 5 oder 6;
m bedeutet 0, 1, 2, 3, 4 oder 5;
n bedeutet 0, 1 oder 2;
p bedeutet 0 oder 1;
q bedeutet 3, 4 oder 5;
s bedeutet 0, 1, 2, 3 oder 4.

Besonders bevorzugt sind 5-oxy-substituierte 3-Phenylisoxazolin-5-carboxamide und 5-oxy-substituierte 3-Phenylisoxazolin-5-thioamide der Formel (I), worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl,
R³ bedeutet durch jeweils m Reste aus der Gruppe bestehend aus Fluor, Chlor, Brom, Cyano, (C₁-C₂)-Alkoxy-substituiertes (C₁-C₄)-Alkyl, (C₃-C₄)-Cycloalkyl, (C₂-C₃)-Alkenyl oder (C₂-C₃)-Alkinyl,
A bedeutet eine Bindung oder eine divalente Einheit aus der Gruppe bestehend aus CH₂, CH₂CH₂, CHCH₃, CH₂CH₂CH₂, CH(CH₂CH₃), CH(CH₃)CH₂, C(CH₃)₂, C(CH₃)₂CH₂, C(iPr)CH₃, CH(CH₂iPr)CH₂, CH₂CH=CH, C(CH₃)₂C=C, CH(CF₃)CH₂, CH(CH₃)CH₂O, CH₂CH₂O, CH(cPr)CH₂O, CH(CH₂OCH₃), CH(CH₂CH₂SCH₃), CH(COOH), CH(COOCH₃), CH(COOH)CH₂, CH(COOCH₃)CH₂, CH₂COH(CF₃), CH(CONHCH₃), CH(CONHCH₃)CH₂ und CH₂CH₂CONHCH₂;
R⁴ bedeutet Wasserstoff oder (C₁-C₈)-Alkyl,
Y bedeutet Sauerstoff oder Schwefel;
X bedeutet Wasserstoff, Cyano, Hydroxy, X¹,
oder
durch jeweils m Reste aus der Gruppe bestehend aus Fluor, Chlor, Cyano, Hydroxy, OR⁷, X¹, OX¹, NHX¹, S(O)ₙR⁵, CO₂R⁸, CONR⁶R⁸, C0NR⁸SO₂R⁵ und POR⁹R⁹ substituiertes (C₁-C₁₂)-Alkyl, (C₃-C₈)-Cycloalkyl, (C₂-C₁₂)-Alkenyl oder (C₂-C₁₂)-Alkinyl;
X¹ bedeutet einen durch s Reste aus der Gruppe bestehend aus R⁶, R^{6a}, R⁸ und R⁹ substituierten Ring aus der Gruppe bestehend aus 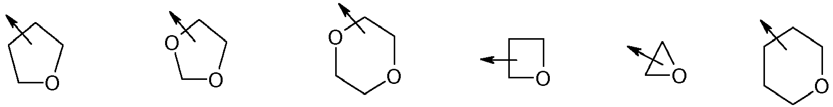 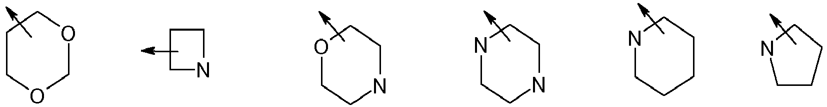 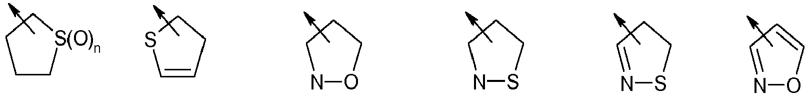 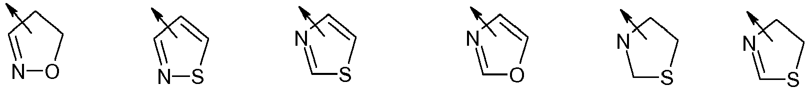 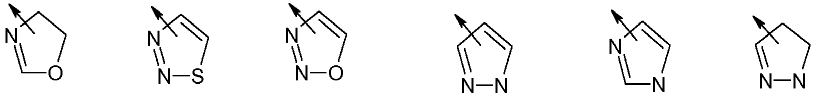 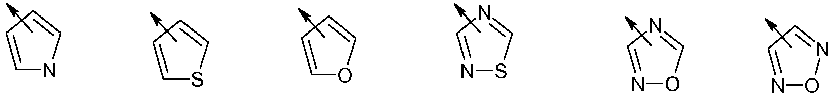 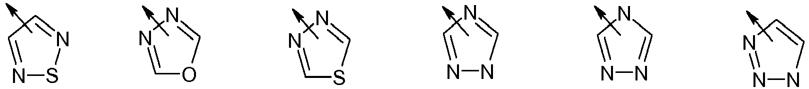 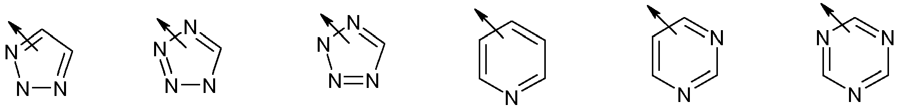 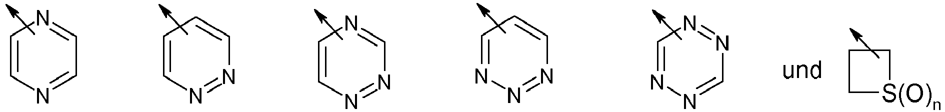 oder X¹ bedeutet durch m Reste aus der Gruppe bestehend aus R⁶, R^{6a}, R⁸ und R⁹ substituiertes Phenyl;
X², X⁴ und X⁶ bedeuten unabhängig voneinander jeweils Wasserstoff, Fluor, oder Chlor,
oder jeweils durch m Reste aus der Gruppe bestehend aus Fluor, Chlor, Cyano und C₁-C₄)-Alkoxy substituiertes (C₁-C₄)-Alkyl oder (C₁-C₄)-Alkoxy,
X³ und X⁵ bedeuten unabhängig voneinander Wasserstoff, Fluor, Chlor, Brom, Cyano,
oder jeweils durch m Reste aus der Gruppe bestehend aus Fluor und Chlor substituiertes (C₁-C₆)-Alkyl,
oder durch m Reste aus der Gruppe bestehend aus Fluor und Chlor substituiertes (C₁-C₆)-Alkoxy;
R⁵ bedeutet Methyl oder Ethyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₂-C₆)-Alkenyloxy oder (C₂-C₆)-Alkinyloxy;
R⁷ bedeutet Wasserstoff oder durch jeweils m Reste aus der Gruppe bestehend aus Fluor und Chlor substituiertes (C₁-C₆)-Alkyl,
R⁸ bedeutet R⁷,
R⁹ bedeutet (C₁-C₃)-Alkoxy,
m bedeutet 0, 1, 2 oder 3;
n bedeutet 0, 1 oder 2;
s bedeutet 0, 1, 2, 3 oder 4.

Geeignete Zwischenstufen zur Herstellung der erfindungsgemässen Verbindungen der Formel (I) sind die Verbindungen der Formel (II): 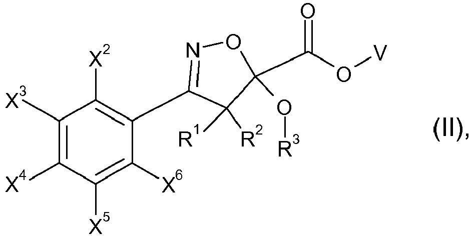 worin die Reste X¹, X², X³, X⁴, X⁵, X⁶, R¹, R², R³ und R⁵ die in der Formel (I) beschriebene Bedeutung haben und V für Wasserstoff oder R⁵ steht. Verbindungen der Formel (II) sind neu und ebenfalls Gegenstand vorliegender Erfindung.

Die erfindungsgemäßen Verbindungen können gemäß dem Fachmann an sich bekannten Reaktionen beispielsweise nach der in Schema 1 genannten Reaktionsfolge hergestellt werden.

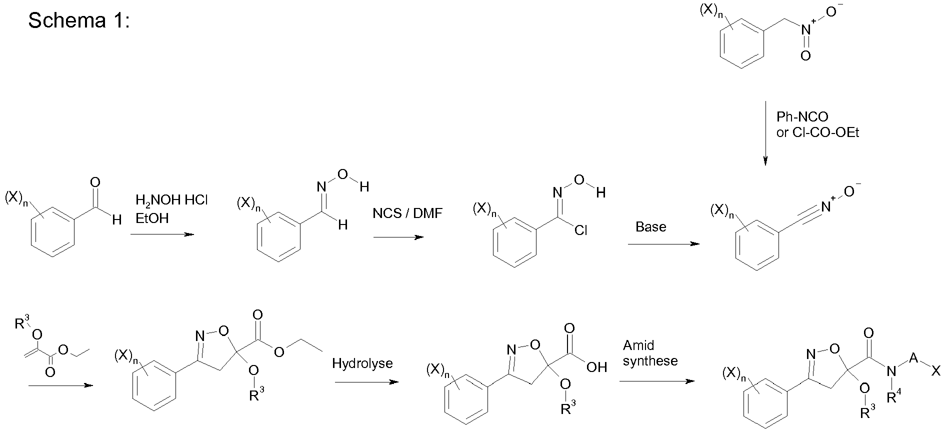

In Schema 1 und den nachfolgenden Schemata steht (X)ₙ für die Substituenten X², X³, X⁴, X⁵ und X⁶. Solche 1,3-dipolaren Cycloadditionen von Nitriloxiden mit geeigneten Dipolarophilen sind beispielsweise beschrieben in Reviews: 1,3 dipolar Cycloaddition Chemistry, Padwa, ed. Wiley, New York, 1984; Kanemasa and Tsuge, Heterocycles 1990, 30, 719. Zur Darstellung von Chloroximen siehe Kim,Jae N., Ryu, Eung K. J. Org. Chem. 1992, 57, 6649).

Erfindungsgemäße Verbindungen, die in der 4- und 5- Position des Isoxazolinringsystems substituiert sind, lassen sich ebenfalls durch 1,3 dipolare Cycloaddition herstellen, indem geeignet 1,2-disubstituierte Olefine als Dipolarophile eingesetzt werden. Zumeist entstehen bei dieser Reaktion Diastereomerengemische, die durch Säulenchromatographie getrennt werden können. Optisch aktive Isoxazoline können durch chirale HPLC geeigneter Vorstufen oder Endstufen erhalten werden, ebenso auch durch enantioselektive Reakionen wie z.B. enzymatische Ester- oder Amidspaltungen oder durch den Einsatz von chiralen Hilfsreagenzien am Dipolarophil wie von Olssen beschrieben, (J. Org.Chem. 1988, 53, 2468).

Die Herstellung von geeignet substituierten 2-Alkoxyacrylsäurestern (Schema 2) ist beispielsweise durch Umsetzung von alpha-Ketoestern zu entsprechenden Ketalen (Lit : Wenkert, E; Alonso, M.E.; Buckwalter B.L., Sanchez E.L. J.Am Chem Soc. 1983, 105, 2021 and Lit.: LaMattina, J.L.; Mularski, C.J., J.Org Chem. 1984, 49, 4800), und deren Eliminierung zu 2-Alkoxyacrylsäureestern möglich (analog Lit.: Esswein A.et al, Helvetica Chimica Acta 1989, 72(2), 213.)

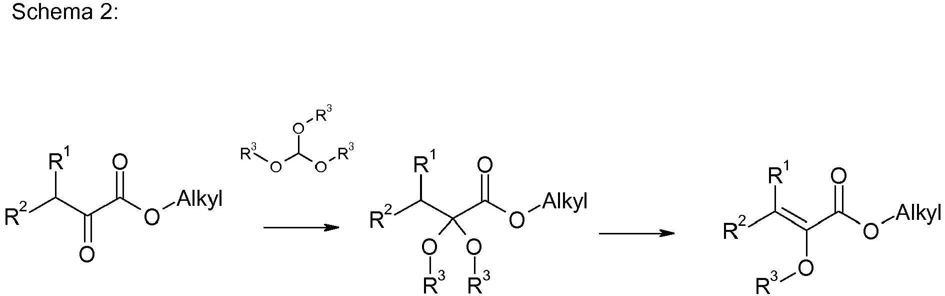

Zur Herstellung der erfindungsgemäßen Verbindungen können auch geeignet substituierte 2-Alkoxyacrylsäureamide eingesetzt werden (Schema 3). Diese sind aus den in Schema 2 beschriebenen Acrylsäureestern nach Hydrolyse und Amidbildung zugänglich.

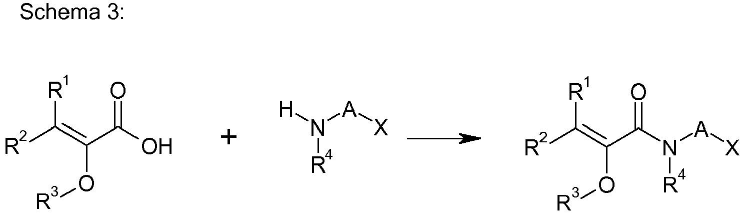

Zur Aktivierung der Alkoxy-Acrylsäure bieten sich dabei Carbodiimide wie zum Beispiel EDCI an (Chen, F. M. F.; Benoiton, N. L. Synthesis 1979, 709). Zur Herstellung von Acrylsäureamiden siehe US2521902, JP60112746, J. of Polymer Science 1979, 17 (6), 1655. Geeignet substituierte 2-Alkoxy-acrylsäureamide können in einer 1,3-Cycloadditionsreaktion mit Nitriloxiden zu den erfindungsgemäßen Verbindungen umgesetzt werden (Schema 4).

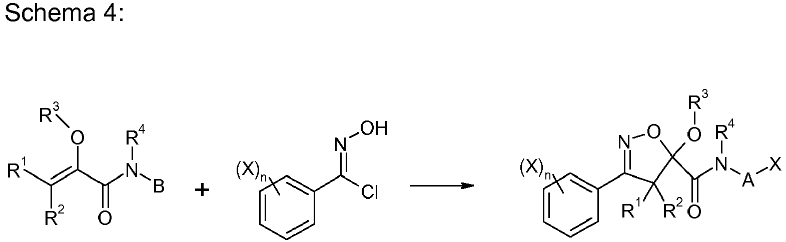

Transformationen der funktionellen Gruppen R³ sind sowohl auf der Stufe der Alkene als auch auf der Stufe der Isoxazoline möglich.

In Schema 5 ist der Zugang zu verschiedenen R³-substituierten Isoxazolinen beschrieben.

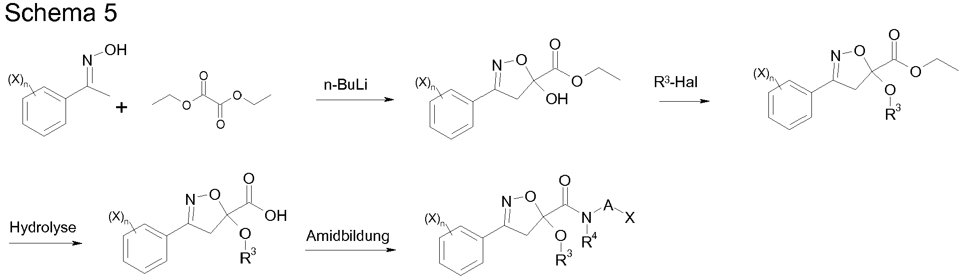

Die Umsetzung von Oxalsäurediestern mit Oximen (Schema 5) führt zu 5-Hydroxy-3-phenylisoxazolinen. (Lit.: Dang, T.T., Albrecht U., Langer P., Synthesis 2006, 15, 2515). Die Hydroxygruppe kann dann unter geeigneten Bedingungen derivatisiert werden. Die Zielverbindungen sind aus den Estern nach Hydrolyse und anschließender Amidbildung zugänglich.

In Schema 6 ist der Zugang zu 5-Alkoxy-3-phenyl-isoxazolin-5-thioamiden durch Umsetzung der 5-Alkoxy-3-phenyl-isoxazolin-5-carboxamiden durch den Einsatz des Lawesson-Reagenz beschrieben. (Lit.:WYETH, WO2003/93277, Lit.: Wishka D.G., Walker D.P., Tetrahedron Letters 2011, 52, 4713-4715)

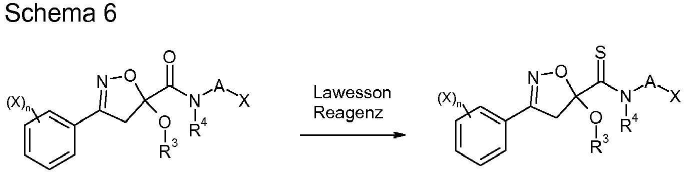

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, Iowa 52004-0797, USA oder Reaktionsstationen (reaction stations) der Firma Radleys, Shirehill, Saffron Waiden, Essex, CB 11 3AZ, England oder MultiPROBE Automated Workstations der Firma Perkin Elmar, Waltham, Massachusetts 02451, USA. Für die parallelisierte Aufreinigung von Verbindungen der Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automations-module beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyl-octenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüber hinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da z.B. die Lagerbildung verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften liegen in einer Toleranz oder Resistenz gegen abiotische Stressoren z.B. Hitze, Kälte, Trockenheit, Salz und ultraviolette Strahlung.

Bevorzugt ist die Anwendung der erfindungsgemäßen Verbindungen der Formel (I) oder deren Salze in wirtschaftlich bedeutenden transgenen Kulturen von Nutz-und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht wurden.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen gentechnische Veränderungen von Kulturpflanzen zwecks Modifikation der in den Pflanzen synthetisierten Stärke (z.B. WO 92/011376 A, WO 92/014827 A, WO 91/019806 A), transgene Kulturpflanzen, welche gegen bestimmte Herbizide vom Typ Glufosinate (vgl. z.B. EP 0242236 A, EP 0242246 A) oder Glyphosate (WO 92/000377 A) oder der Sulfonylharnstoffe (EP 0257993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum™ GAT™ (Glyphosate ALS Tolerant).
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924 A, EP 0193259 A).
- transgene Kulturpflanzen mit modifizierter Fettsäurezusammensetzung (WO 91/013972 A).
- gentechnisch veränderte Kulturpflanzen mit neuen Inhalts- oder Sekundärstoffen z.B. neuen Phytoalexinen, die eine erhöhte Krankheitsresistenz verursachen (EP 0309862 A, EP 0464461 A)
- gentechnisch veränderte Pflanzen mit reduzierter Photorespiration, die höhere Erträge und höhere Stresstoleranz aufweisen (EP 0305398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualität auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen. So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. 2,4 D, Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, oder gegen beliebige Kombinationen dieser Wirkstoffe, resistent sind.

Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung OptimumTM GATTM (Glyphosate ALS Tolerant) eingesetzt werden.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) und der Verbindungen der Formel (la) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl-oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4.

Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxid-addukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen Wirkstoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispiels-weise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylen-oxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen. In Spritzpulvern beträgt die Wirkstoff-konzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Neben den herbiziden Eigenschaften weisen die erfindungsgemäßen Verbindungen auch gute fungizide Eigenschaften auf. Die vorliegende Erfindung betrifft somit auch ein Mittel, zum Bekämpfen von unerwünschten Mikroorganismen, umfassend die erfindungsgemäßen Wirkstoffe. Vorzugsweise handelt es sich um fungizide Mittel, welche landwirtschaftlich verwendbare Hilfsmittel, Solventien, Trägerstoffe, oberflächenaktive Stoffe oder Streckmittel enthalten.
Weiterhin betrifft die Erfindung auch ein Verfahren zum Bekämpfen unerwünschter Mikroorganismen, dadurch gekennzeichnet, dass man die erfindungsgemäßen Wirkstoffe auf die phytopathogenen Pilze und/oder deren Lebensraum ausbringt. Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.
Als feste oder flüssige Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse, feste Düngemittel, Wasser, Alkohole, besonders Butanol, organische Solventien, Mineral- und Pflanzenöle sowie Derivate hiervon. Mischungen solcher Trägerstoffe können ebenfalls verwendet werden. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel.
Als verflüssigte gasförmige Streckmittel oder Trägerstoffe kommen solche Flüssigkeiten infrage, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.
Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat, sowie natürliche Phospholipide, wie Kephaline und Lecithine, und synthetische Phospholipide. Weitere Additive können mineralische und vegetabile Öle sein.
Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Dichlormethan, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels. Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden. Im Allgemeinen enthalten die erfindungsgemäßen Mittel und Formulierungen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent. Die erfindungsgemäßen Wirkstoffe bzw. Mittel können als solche oder in Abhängigkeit von ihren jeweiligen physikalischen und/oder chemischen Eigenschaften in Form ihrer Formulierungen oder den daraus bereiteten Anwendungsformen, wie Aerosole, Kapselsuspensionen, Kaltnebelkonzentrate, Heißnebelkonzentrate, verkapselte Granulate, Feingranulate, fließfähige Konzentrate für die Behandlung von Saatgut, gebrauchsfertige Lösungen, verstäubbare Pulver, emulgierbare Konzentrate, Öl-in-Wasser-Emulsionen, Wasser-in-Öl-Emulsionen, Makrogranulate, Mikrogranulate, Öl dispergierbare Pulver, Öl mischbare fließfähige Konzentrate, Öl mischbare Flüssigkeiten, Schäume, Pasten, Pestizid ummanteltes Saatgut, Suspensionskonzentrate, Suspensions-Emulsions-Konzentrate, lösliche Konzentrate, Suspensionen, Spritzpulver, lösliche Pulver, Stäubemittel und Granulate, wasserlösliche Granulate oder Tabletten, wasserlösliche Pulver für Saatgutbehandlung, benetzbare Pulver, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen eingesetzt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.
Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die Erfindung umfasst weiterhin ein Verfahren zur Behandlung von Saatgut.
Die Erfindung betrifft weiterhin Saatgut, welches gemäß einem der im vorherigen Absatz beschriebenen Verfahren behandelt wurde. Die erfindungsgemäßen Saatgüter finden Anwendung in Verfahren zum Schutz von Saatgut vor unerwünschten Mikroorganismen. Bei diesen wird ein mit wenigstens einem erfindungsgemäßen Wirkstoff behandeltes Saatgut verwendet. Die erfindungsgemäßen Wirkstoffe bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von phytopathogenen Pilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt werden, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.
Die vorliegende Erfindung bezieht sich daher auch auf ein Verfahren zum Schutz von Saatgut und keimenden Pflanzen vor dem Befall von phytopathogenen Pilzen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen. Weiterhin bezieht sich die Erfindung auf Saatgut, welches zum Schutz vor phytopathogenen Pilzen mit einem erfindungsgemäßen Mittel behandelt wurde.

Die Bekämpfung von phytopathogenen Pilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der erfindungsgemäßen Wirkstoffe bzw. Mittel die Behandlung des Saatguts mit diesen Wirkstoffen bzw. Mitteln nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.
Ebenso ist es als vorteilhaft anzusehen, dass die erfindungsgemäßen Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den erfindungsgemäßen Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die erfindungsgemäßen Mittel eignen sich zum Schutz von Saatgut jeglicher Pflanzensorte, die in der Landwirtschaft, im Gewächshaus, in Forsten oder im Garten- und Weinbau eingesetzt wird. Insbesondere handelt es sich dabei um Saatgut von Getreide (wie Weizen, Gerste, Roggen, Triticale, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Raps, Mohn, Olive, Kokosnuss, Kakao, Zuckerrohr, Tabak, Gemüse (wie Tomate, Gurke, Zwiebeln und Salat), Rasen und Zierpflanzen (siehe auch unten). Besondere Bedeutung kommt der Behandlung des Saatguts von Getreide (wie Weizen, Gerste, Roggen, Triticale und Hafer), Mais und Reis zu.
Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus oder Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430, US 5,876,739, US 2003/0176428 A1, WO 2002/080675 A1, WO 2002/028186 A2.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen. Diese Formulierungen werden in bekannter Weise hergestellt, indem man die Wirkstoffe mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate. Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Die erfindungsgemäß verwendbaren Beizmittel-Formulierungen können entweder direkt oder nach vorherigem Verdünnen mit Wasser zur Behandlung von Saatgut der verschiedensten Art, auch von Saatgut transgener Pflanzen, eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die erfindungsgemäßen Wirkstoffe bzw. Mittel weisen eine starke mikrobizide Wirkung auf und können zur Bekämpfung von unerwünschten Mikroorganismen, wie Pilzen und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht wird.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes. Sie können vorzugsweise als Pflanzenschutzmittel eingesetzt werden. Sie sind gegen normal sensible und resistente Arten sowie gegen alle oder einzelne Entwicklungsstadien wirksam.

Als Pflanzen, welche erfindungsgemäß behandelt werden können, seien folgende Hauptanbaupflanzen erwähnt: Mais, Sojabohne, Baumwolle, Brassica Ölsaaten wie Brassica napus (z.B. Canola), Brassica rapa, B. juncea (z.B. (Acker-)Senf) und Brassica carinata, Reis, Weizen Zuckerrübe, Zurckerrohr, Hafer, Roggen, Gerste, Hirse, Triticale, Flachs, Wein und verschiedene Früchte und Gemüse von verschiedenen botanischen Taxa wie z.B. Rosaceae sp. (beispielsweise Kernfrüchte wie Apfel und Birne, aber auch Steinfrüchte wie Aprikosen, Kirschen, Mandeln und Pfirsiche und Beerenfrüchte wie Erdbeeren), Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp. (beispielsweise Bananenbäume und - plantagen), Rubiaceae sp. (beispielsweise Kaffee), Theaceae sp., Sterculiceae sp., Rutaceae sp. (beispielsweise Zitronen, Organen und Grapefruit); Solanaceae sp. (beispielsweise Tomaten, Kartoffeln, Pfeffer, Auberginen), Liliaceae sp., Compositae sp. (beispielsweise Salat, Artischocke and Chicoree - einschließlich Wurzelchicoree, Endivie oder gemeinen Chicoree), Umbelliferae sp. (beispielsweise Karrotte, Petersilie, Stangensellerie und Knollensellerie), Cucurbitaceae sp. (beispielsweise Gurke - einschließlich Gewürzgurke, Kürbis, Wassermelone, Flaschenkürbis und Melonen), Alliaceae sp. (beispielsweise Lauch und Zwiebel), Cruciferae sp. (beispielsweise Weißkohl, Rotkohl, Brokkoli, Blumenkohl, Rosenkohl, Pak Choi, Kohlrabi, Radieschen, Meerrettich, Kresse und Chinakohl), Leguminosae sp. (beispielsweise Erdnüsse, Erbsen, und Bohnen - wie z.B. Stangenbohne und Ackerbohne), Chenopodiaceae sp. (beispielsweise Mangold, Futterrübe, Spinat, Rote Rübe), Malvaceae (beispielsweise Okra), Asparagaceae (beispielsweise Spargel); Nutzpflanzen und Zierpflanzen in Garten und Wald; sowie jeweils genetisch modifizierte Arten dieser Pflanzen.

Wie oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Mitochondriengenom der transformierten Pflanze dadurch neue oder verbesserte agronomische oder sonstige Eigenschaften verleiht, dass es ein interessierendes Protein oder Polypeptid exprimiert oder dass es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, dass die behandelten Pflanzen, wenn sie im Anschluss daran mit unerwünschten phytopathogenen Pilzen inokuliert wurde, einen beträchtlichen Resistenzgrad gegen diese unerwünschten phytopathogenen Pilze aufweisen. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im Allgemeinen von 1 bis 10 Tagen, vorzugsweise 1 bis 7 Tagen, nach der Behandlung der Pflanzen mit den Wirkstoffen. Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Stressfaktoren resistent, d.h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Beispiele für Nematoden-resistente Pflanzen sind z.B. folgenden US Patentanmeldungen beschrieben: 11/765,491, 11/765,494, 10/926,819, 10/782,020, 12/032,479, 10/783,417, 10/782,096, 11/657,964, 12/192,904, 11/396,808, 12/166,253, 12/166,239, 12/166,124, 12/166,209, 11/762,886, 12/364,335, 11/763,947, 12/252,453, 12/209,354, 12/491,396 und 12/497,221.

Pflanzen und Pflanzensorten, die ebenfalls erfindungsgemäß behandelt werden können, sind solche Pflanzen, die gegen einen oder mehrere abiotische Stressfaktoren resistent sind. Zu den abiotischen Stressbedingungen können zum Beispiel Dürre, Kälte- und Hitzebedingungen, osmotischer Stress, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Stress- und Nicht-Stress-Bedingungen) beeinflusst werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im Allgemeinen zu höherem Ertrag, höherer Wüchsigkeit, besserer Gesundheit und besserer Resistenz gegen biotische und abiotische Stressfaktoren führt. Solche Pflanzen werden typischerweise dadurch erzeugt, dass man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d.h. mechanischem Entfernen der männlichen Geschlechtsorgane bzw. der männlichen Blüten), produziert werden; es ist jedoch üblicher, dass die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, dass die Pollenfertilität in Hybridpflanzen, die die für die Pollensterilität verantwortlichen genetischen Determinanten enthalten, völlig restoriert wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden. Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. Pflanzen können mit verschiedenen Methoden tolerant gegenüber Glyphosate gemacht werden. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium Salmonella typhimurium (Comai et al., 1983, Science 221, 370-371), das CP4-Gen des Bakteriums Agrobacterium sp. (Barry et al., 1992, Curr. Topics Plant Physiol. 7, 139-145), die Gene, die für eine EPSPS aus der Petunie (Shah et al., 1986, Science 233, 478-481), für eine EPSPS aus der Tomate (Gasser et al., 1988, J. Biol. Chem. 263, 4280-4289) oder für eine EPSPS aus Eleusine (WO 01/66704) kodieren. Es kann sich auch um eine mutierte EPSPS handeln. Glyphosate-tolerante Pflanzen können auch dadurch erhalten werden, dass man ein Gen exprimiert, das für ein Glyphosate-Oxidoreduktase-Enzym kodiert. Glyphosate-tolerante Pflanzen können auch dadurch erhalten werden, dass man ein Gen exprimiert, das für ein Glyphosate-acetyltransferase-Enzym kodiert. Glyphosatetolerante Pflanzen können auch dadurch erhalten werden, dass man Pflanzen, die natürlich vorkommende Mutationen der oben erwähnten Gene enthalten, selektiert. Pflanzen, die EPSPS Gene, welche Glyphosate-Toleranz verleihen, exprimieren, sind beschrieben. Pflanzen, welche andere Gene, die Glyphosate-Toleranz verleihen, z.B. Decarboxylase-Gene, sind beschrieben. Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricinacetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes oder chimäres HPPD-Enzym kodiert, transformiert werden, wie in WO 96/38567, WO 99/24585, WO 99/24586, WO 2009/144079, WO 2002/046387 oder US 6,768,044 beschrieben. Eine Toleranz gegenüber HPPD-Hemmern kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Solche Pflanzen sind in WO 99/34008 und WO 02/36787 beschrieben. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert, wie in WO 2004/024928 beschrieben ist. Außerdem können Pflanzen noch toleranter gegen HPPD-Hemmern gemacht werden, indem man ein Gen in ihr Genom einfügt, welches für ein Enzym kodiert, das HPPD-Hemmer metabolisiert oder abbaut, wie z.B. CYP450 Enzyme (siehe WO 2007/103567 und WO 2008/150473). Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen wie z.B. in Tranel und Wright (Weed Science 2002, 50, 700-712) beschrieben ist. Die Herstellung von sulfonylharnstofftoleranten Pflanzen und imidazolinontoleranten Pflanzen ist beschrieben. Weitere sulfonylharnstoff- und imidazolinontolerante Pflanzen sind auch beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden (vgl. z.B. für Sojabohne US 5,084,082, für Reis WO 97/41218, für Zuckerrübe US 5,773,702 und WO 99/057965, für Salat US 5,198,599 oder für Sonnenblume WO 01/065922).

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfasst im vorliegenden Zusammenhang jegliche Pflanze, die mindestens ein Transgen enthält, das eine Kodiersequenz umfasst, die für folgendes kodiert:
1) ein insektizides Kristallprotein aus Bacillus thuringiensis oder einen insektiziden Teil davon, wie die insektiziden Kristallproteine, aufgelistet von Crickmore et al. (Microbiology and Molecular Biology Reviews 1998, 62, 807-813), aktualisiert von Crickmore et al. (2005) bei der Bacillus thuringiensis Toxin Nomenclatur, online bei: http://www.lifesci.sussex.ac.uk/Home/Neil Crickmore/Bt/),
   oder insektizide Teile davon, z.B. Proteine der Cry-Proteinklassen Cry1Ab, Cry1Ac, Cry1 B, Cry1 C, Cry1 D, Cry1 F, Cry2Ab, Cry3Aa, or Cry3Bb oder insektizide Teile davon (z.B. EP-A 1999141 und WO 2007/107302), oder solche Proteine, kodiert durch synthetische Gene wie in US Patentanmeldung 12/249,016 beschrieben ist; oder
2) ein Kristallprotein aus Bacillus thuringiensis oder einen Teil davon, der in Gegenwart eines zweiten, anderen Kristallproteins als Bacillus thuringiensis oder eines Teils davon insektizid wirkt, wie das binäre Toxin, das aus den Kristallproteinen Cy34 und Cy35 besteht (Nat. Biotechnol. 2001, 19, 668-72; Applied Environm. Microbiol. 2006, 71, 1765-1774) oder das binäre Toxin, das aus den Cry1A oder Cry1 F Proteinen besteht und die Cry2Aa oder Cry2Ab oder Cry2Ae Proteine (US Patentanmeldung 12/214,022 und EP08010791.5); oder
3) ein insektizides Hybridprotein, das Teile von zwei unterschiedlichen insektiziden Kristallproteinen aus Bacillus thuringiensis umfasst, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus Bacillus thuringiensis oder Bacillus cereus oder einen insektiziden Teil davon, wie die vegetativ wirkenden insektentoxischen Proteine (vegetative insekticidal proteins, VIP), die unter http://www.lifesci.sussex.ac.uk/Home/Neil Crickmore/Bt/vip.html angeführt sind, z. B. Proteine der Proteinklasse VIP3Aa; oder
6) ein sezerniertes Protein aus Bacillus thuringiensis oder Bacillus cereus, das in Gegenwart eines zweiten sezernierten Proteins aus Bacillus thuringiensis oder B. cereus insektizid wirkt, wie das binäre Toxin, das aus den Proteinen VIP1A und VIP2A besteht (WO 94/21795); oder
7) ein insektizides Hybridprotein, das Teile von verschiedenen sezernierten Proteinen von Bacillus thuringiensis oder Bacillus cereus umfasst, wie ein Hybrid der Proteine von 1) oder ein Hybrid der Proteine von 2) oben; oder
8) ein Protein gemäß einem der Punkte 5) bis 7) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102; oder
9) ein sezerniertes Protein aus Bacillus thuringiensis oder Bacillus cereus, das in Gegenwart eines Kristallproteins von Bacillus thuringiensis insektizid wirkt, wie das binäre Toxin, das aus den Proteinen VIP3 und Cry1A oder Cry1 F besteht (US Patentanmeldungen 61/126083 und 61/195019), oder das binäre Toxin, das aus dem VIP3 Protein und den Cry2Aa oder Cry2Ab oder Cry2Ae Proteinen besteht (US Patentanmeldung 12/214,022 und EP 08010791.5); oder
10) ein Protein gemäß Punkt 9) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt). Natürlich zählt zu den insektenresistenten transgenen Pflanzen im vorliegenden Zusammenhang auch jegliche Pflanze, die eine Kombination von Genen umfasst, die für die Proteine von einer der oben genannten Klassen 1 bis 10 kodieren. In einer Ausführungsform enthält eine insektenresistente Pflanze mehr als ein Transgen, das für ein Protein nach einer der oben genannten 1 bis 10 kodiert, um das Spektrum der entsprechenden Zielinsektenarten zu erweitern oder um die Entwicklung einer Resistenz der Insekten gegen die Pflanzen dadurch hinauszuzögern, dass man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Eine "insekten-resistente transgene Pflanze" umfasst im vorliegenden Zusammenhang weiterhin jede Pflanze, die wenigstens ein Transgen enthält, welches eine Sequenz zur Herstellung einer Doppelstrang-RNA umfasst, die nach Nahrungsaufnahme durch einen Insektenschädling das Wachstum dieses Schädlings hindert.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Stressfaktoren tolerant. Solche Pflanzen können durch genetische Transformation oder durch Selektion von Pflanzen, die eine Mutation enthalten, die solch eine Stressresistenz verleiht, erhalten werden. Zu besonders nützlichen Pflanzen mit Stresstoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein stresstoleranzförderndes Transgen enthalten, das die Expression und/oder Aktivität der für PARG kodierenden Gene der Pflanzen oder Pflanzenzellen zu reduzieren vermag;
c. Pflanzen, die ein stresstoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so dass sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.
4) Transgene Pflanzen oder Hybridpflanzen wie Zwiebeln mit bestimmten Eigenschaften wie "hohem Anteil an löslichen Feststoffen" (,high soluble solids content'), geringe Schärfe (,low pungency', LP) und/oder lange Lagerfähigkeit (,long storage', LS).

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten, wie Baumwollpflanzen mit einer erhöhten Expression der Saccharosephosphatsynthase;
c) Pflanzen wie Baumwollpflanzen mit einer erhöhten Expression der Saccharosesynthase;
d) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
e) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Pflanzen oder Pflanzensorten (die nach Methoden der pflanzlichen Biotechnologie, wie der Gentechnik, erhalten werden können), die ebenfalls erfindungsgemäß behandelt werden können, sind Pflanzen wie Kartoffeln, welche Virus-resistent sind z.B. gegen den Kartoffelvirus Y (Event SY230 und SY233 von Tecnoplant, Argentinien), oder welche resistent gegen Krankheiten wie die Kraut- und Knollenfäule (potato late blight) (z.B. RB Gen), oder welche eine verminderte kälteinduzierte Süße zeigen (welche die Gene Nt-Inh, II-INV tragen) oder welche den Zwerg-Phänotyp zeigen (Gen A-20 Oxidase).

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften im Samenausfall (seed shattering). Solche Pflanzen können durch genetische Transformation oder durch Selektion von Pflanzen, die eine Mutation enthalten, die solche veränderten Eigenschaften verleihen, und umfassen Pflanzen wie Raps mit verzögertem oder vermindertem Samenausfall.

Besonders nützliche transgene Pflanzen, die erfindungsgemäß behandelt werden können, sind Pflanzen mit Transformationsevents oder Kombinationen von Transformationsevent, welche in den USA beim Animal and Plant Health Inspection Service (APHIS) of the United States Department of Agriculture (USDA) Gegenstand von erteilten oder anhängigen Petitionen für den nicht-regulierten Status sind. Die Information hierzu ist jederzeit beim APHIS (4700 River Road Riverdale, MD 20737, USA) erhältlich, z.B. über die Internetseite http://www.aphis.usda.gov/brs/not_reg.html. Am Anmeldetag dieser Anmeldung waren beim APHIS die Petitionen mit folgenden Informationen entweder erteilt oder anhängig:
- Petition: Identifikationsnummer der Petition. Die Technische Beschreibung des Transformationsevents kann im einzelnen Petitionsdokument erhältlich von APHIS auf der Website über die Petitionsnummer gefunden werden.
- Erweiterung einer Petition: Referenz zu einer frühere Petition, für die eine Erweiterung oder Verlängerung beantragt wird.
- Institution: Name der die Petition einreichenden Person.
- Regulierter Artikel: die betroffen Pflanzenspecies.
- Transgener Phänotyp: die Eigenschaft ("Trait"), die der Pflanze durch das Transformationsevent verliehen wird.
- Transformationevent oder -linie: der Name des oder der Events (manchmal auch als Linie(n) bezeichnet), für die der nicht-regulierte Status beantragt ist.
- APHIS Documente: verschiedene Dokumente, die von APHIS bzgl. der Petition veröffentlicht warden oder von APHIS auf Anfrage erhalten werden können. Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://cera-gmc.org/index.php?evidcode=&hstIDXCode=&gType=&AbbrCode=&atCode= &stCode=&coIDCode=&action=gm_crop_database&mode=Submit).

Die erfindungsgemäßen Wirkstoffe bzw. Mittel können außerdem im Materialschutz zum Schutz von technischen Materialien gegen Befall und Zerstörung durch unerwünschten Mikroorganismen, wie z.B. Pilzen und Insekten, eingesetzt werden.

Weiter können die erfindungsgemäßen Verbindungen allein oder in Kombinationen mit anderen Wirkstoffen als Antifouling-Mittel eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier, Wandpappe und Karton, Textilien, Teppiche, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen und Gebäuden, z.B. Kühlwasserkreisläufe, Kühl- und Heizsysteme und Belüftungs- und Klimaanlagen, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern. Außerdem können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Das erfindungsgemäße Verfahren zum Bekämpfen von unerwünschten Pilzen kann auch zum Schutz von so genannten Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Beispielhaft, aber nicht begrenzend, seien einige Erreger von pilzlichen Erkrankungen, die erfindungsgemäß behandelt werden können, genannt: Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator; Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi und Phakopsora meibomiae; Puccinia-Arten, wie beispielsweise Puccinia recondita oder Puccinia triticina; Uromyces-Arten, wie beispielsweise Uromyces appendiculatus; Erkrankungen, hervorgerufen durch Erreger der Gruppe der Oomyceten wie z.B. Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum; Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladiosporum-Arten, wie beispielsweise Cladiosporium cucumerinum; Cochliobolus-Arten, wie beispielsweise Cochliobolus sativus (Konidienform: Drechslera, Syn: Helminthosporium); Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola und M. fijiensis; Phaeosphaeria-Arten, wie beispielsweise Phaeosphaeria nodorum; Pyrenophora-Arten, wie beispielsweise Pyrenophora teres; Ramularia-Arten, wie beispielsweise Ramularia collo-cygni; Rhynchosporium-Arten, wie beispielsweise Rhynchosporium secalis; Septoria-Arten, wie beispielsweise Septoria apii; Typhula-Arten, wie beispielsweise Typhula incarnata; Venturia-Arten, wie beispielsweise Venturia inaequalis; Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Rhizoctonia-Arten, wie beispielsweise Rhizoctonia solani; Tapesia-Arten, wie beispielsweise Tapesia acuformis; Thielaviopsis-Arten, wie beispielsweise Thielaviopsis basicola; Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae;
Monographella-Arten, wie beispielsweise Monographella nivalis; Septoria-Arten, wie beispielsweise Septoria nodorum; Erkrankungen, hervorgerufen durch Brandpilze wie z.B. Sphacelotheca-Arten, wie beispielsweise Sphacelotheca reiliana; Tilletia-Arten, wie beispielsweise Tilletia caries, T. controversa; Urocystis-Arten, wie beispielsweise Urocystis occulta; Ustilago-Arten, wie beispielsweise Ustilago nuda, U. nuda tritici; Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum und P. purpurogenum; Sclerotinia-Arten, wie beispielsweise Sclerotinia sclerotiorum; Verticilium-Arten, wie beispielsweise Verticilium alboatrum; Samen- und bodenbürtige Fäulen und Welken, sowie Sämlingserkrankungen, hervorgerufen durch z.B. Fusarium-Arten, wie beispielsweise Fusarium culmorum; Phytophthora Arten, wie beispielsweise Phytophthora cactorum; Pythium-Arten, wie beispielsweise Pythium ultimum; Rhizoctonia-Arten, wie beispielsweise Rhizoctonia solani; Sclerotium-Arten, wie beispielsweise Sclerotium rolfsii; Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Taphrina-Arten, wie beispielsweise Taphrina deformans; Degenerationserkrankungen holziger Pflanzen, hervorgerufen durch z.B. Esca-Arten, wie beispielsweise Phaemoniella clamydospora und Phaeoacremonium aleophilum und Fomitiporia mediterranea; Blüten- und Samenerkrankungen, hervorgerufen durch z.B. Botrytis-Arten, wie beispielsweise Botrytis cinerea; Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani; Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora;
Bevorzugt können die folgenden Krankheiten von Soja-Bohnen bekämpft werden: Pilzkrankheiten an Blättern, Stängeln, Schoten und Samen verursacht durch z.B. Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola). Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Darüber hinaus weisen die erfindungsgemäßen Wirkstoffeauch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die erfindungsgemäßen Wirkstoffe können daher sowohl in medizinischen als auch in nicht-medizinischen Anwendungen eingesetzt werden.

Beim Einsatz der erfindungsgemäßen Wirkstoffe als Fungizide können die Aufwandmengen je nach Applikationsart innerhalb eines größeren Bereiches variiert werden. Die Aufwandmenge der erfindungsgemäßen Wirkstoffe beträgt
- bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
- bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
- bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkornalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-likeorganism) und RLO (Rickettsia-like-organism) verwendet werden. Sie lassen sich gegebenenfalls auch als Zwischen- oder Vorprodukte für die Synthese weiterer Wirkstoffe einsetzen.

Die nachstehenden Beispiele erläutern die Erfindung näher.

### A. Chemische Beispiele

### 1. Herstellung von N-(Cyanmethyl)-3-(3,5-dichlorphenyl)-5-methoxy-4,5-dihydro-1,2-oxazol-5-carboxamid (Beispiel 1.20-40)

### Intermediat 1 : 3,5-Dichlorbenzaldehydoxim

23.82 g (342.8 mmol) Hydroxylaminhydrochlorid wurden mit 80 ml Ethanol versetzt. Nach Zugabe von 28.12 g (342.8 mmo) Natriumacetat tropfte man eine Lösung von 50.00 g (285.7 mmol) 3,5-Dichlorbenzaldehyd in 100 ml Ethanol innerhalb von 30 min zu, ließ für 2h rühren und anschließend über Nacht stehen. Die Reaktions-mischung wurde vollständig eingeengt, dann mit 500 ml CH₂Cl₂ versetzt und mit 400 ml Wasser gewaschen. Die Wasserphase wurde einmal mit 100 ml CH₂Cl₂ gewaschen, die organische Phase über Na₂SO₄ getrocknet, abfiltriert und eingeengt. Der Rückstand wurde ohne weitere Reinigung verwendet. Ausbeute: 56.50 g (98%) ¹ H NMR (CDCl₃) : σ = 7.36 (s, 1 H, Ar-H), 7.47 (s, 2H, Ar-H), 7.63 (s br, 1 H, OH), 8.02 (s, 1 H, HC=NOH).

### Intermediat 2: 3,5-Dichlor-N-hydroxybenzolcarboximidoylchlorid

30.00 g (157.9 mmol) 3,5-Dichlorbenzaldehydoxim wurden in 379 ml 0.5M HCl in DMF vorgelegt und bei Raumtemperatur (RT) portionsweise mit 116.7 g (189.5 mmol) Oxone (Kaliumperoxomonosulfat) versetzt. Damit die Reaktionsmischung sich auf nicht mehr als 50°C Innentemperatur erwärmte, wurde mit einem Eisbad gekühlt. Nach 2h wurde die Reaktionslösung auf 1l Eiswasser gegossen und zweimal mit je 500 ml Ether extrahiert. Die vereinigten organischen Phasen wurden anschließend mit 400 ml 0.5M wässriger HCl und mit 200 ml gesättigter NaCl-Lösung gewaschen, über Na₂SO₄ getrocknet, abfiltriert und eingeengt. Der Rückstand wurde ohne weitere Reinigung verwendet. Ausbeute: 28.40 g (80%)
¹H NMR (CDCl₃) : σ = 7.43 (s, 1H, Ar-H), 7.74 (s, 2H, Ar-H), 8.03 (s br, 1 H, OH).

### Intermediat 3: Methyl-3-(3,5-dichlorphenyl)-5-methoxy-4,5-dihydro-1,2-oxazol-5-carboxylat (Beispiel A-116)

35.00 g (155.9 mmol) wurden in 490 ml 2-Propanol gelöst und mit 31.61 g (171.5 mmol) 2-Methoxyacrylsäuremethylester versetzt. Zu dieser Lösung wurden bei RT 65.49 g (779.6 mmol) NaHCO₃ gegeben und 12 h bei Raumtemperatur gerührt. Anschließend filtrierte man vom Feststoff ab und engte das Filtrat am Rotationsverdampfer ein. Das Rohprodukt wurde in Dichlormethan gelöst, über Na₂SO₄ getrocknet, filtriert und eingeengt.Anschließend löste man den Feststoff in wenig Dichlormethan und gab Isopropanol zu. Aus diesem Lösemittelgemisch kristallisierte das Produkt aus. Ausbeute: 28.0 g (59%)
¹H NMR (CDCl₃) : σ = 3.40 (d H_{A} von AB, J = 19 Hz, 1 H, N=C-CH_{A}H_{B}), 3.48 (s,3H, O-CH₃), 3.78 ( d, H_{B} von AB, J = 19 Hz, 1 H, N=C-CH_{A}H_{B}), 3.90 (s, 3H, OCH₃), 7.43 ( s, 1 H, Ar-H), 7.55 ( s, 2H, Ar-H).

### Intermediat 4: 3-(3,5-Dichlorphenyl)-5-methoxy-4,5-dihydro-1,2-oxazol-5-carbonsäure (Beispiel A-115)

40.00 g (131.5 mmol) Methyl-3-(3,5-dichlorphenyl)-5-methoxy-4,5-dihydro-1,2-oxazol-5-carboxylat wurden in 800 ml THF gelöst und anschließend langsam mit 2.99 g LiOH als 0.5 molare Lösung in Wasser versetzt. Nach 2h rühren bei RT entfernte man das Lösemittel THF i.Vac., gab 200 ml einer halbgesättigten NaHCO3-Lösung zu und extrahierte mit Essigester. Anschließend wurde die wässrige Phase mit 300 ml Methylenchlorid versetzt und dann unter Rühren durch langsame Zugabe von 0.5 n HCl auf pH 1 eingestellt. Die organische Phase wurde über Na₂SO₄ getrocknet, filtriert und eingeengt. Ausbeute: 35.4 g (88%)
¹H NMR (CDCl₃) : σ = 3.45 ( d H_{A} von AB, J = 19 Hz, 1H, N=C-CH_{A}H_{B}), 3.51 (s, 3H,OCH₃), 3.85 ( d H_{B} von AB, J = 19 Hz, 1 H,N=C-CH_{A}H_{B}), 7.45 ( s, 1H, Ar-H), 7.56 (s, 2H, Ar-H), 93 (s br., 1H, COOH).

### Beispiel 1.20-40

N-(Cyanmethyl)-3-(3,5-dichlorphenyl)-5-methoxy-4,5-dihydro-1,2-oxazol-5-carboxamid 200 mg (0.689 mmol) 3-(3,5-Dichlorphenyl)-5-methoxy-4,5-dihydro-1,2-oxazol-5-carbonsäure wurde in 10 ml Dichlormethan vorgelegt und mit 93 mg (0.69 mmol) HOBT und 57 mg (1.0 mmol) 2-Aminoacetonitril versetzt. Anschliessend wurden 172 mg (0.896 mmol) 1-(3-dimethylaminopropyl)-3-ethylcarbodiimidhydrochlorid zugegeben und 30 min gerührt. Zur Aufarbeitung wurde mit Wasser gewaschen und über Kieselgel mit Heptan /Ethylacetat chromatographiert.Ausbeute: 220 mg (92%).
¹H NMR (CDCl₃) : σ = 3.36 ( d H_{A} von AB, J = 19 Hz, 1 H, N=C-CH_{A}H_{B}), 3.37 (s, 3H,OCH₃), 3.81 (d H_{B} von AB, J = 19 Hz, 1 H,N=C-CH_{A}H_{B}), 4.27 (d AB, 2H; J AB = 16 Hz, JAC = 7Hz, 2H, NHc-CH_{A}H_{B}); 7.17 (t br, J=7Hz, 1 H, NH); 7.45 (s, 1 H, Ar-H), 7.56 (s, 2H, Ar-H).

### Intermediat 5

### Methyl-2,2-dimethoxypropanoat

100 g (979 mmol) Methyl-2-oxopropanoat wurden mit 135 g (1273 mmol) Trimethylorthoformiat in 240 ml Methanol versetzt. Nach Zugabe von 0.96 g (9.79 mmol) konzentrierter H₂SO₄ wurde 4h auf Rückfluss erhitzt. Das Lösemittel wurde innerhalb von 2h abdestilliert, das Rohprodukt auf 10°C gekühlt und zu einer Lösung aus 2.4 g KOH in 1200 ml Wasser bei 10°C gegeben. Nach mehrfacher Extraktion mit Diethylether wurde über Na₂SO₄ getrocknet, filtriert und eingeengt. Der Rückstand wurde erneut destilliert. Sdp (10 mbar): 50-55°C. Ausbeute: 118 g (77%)
¹H NMR (CDCl₃) : σ = 1.53 (s,3H,C-CH₃), 3.29 (s, 6H, CH₃-O-C-O-CH₃), 3.82 (s, 3H,COOCH₃).

### Intermediat 6

### Methyl-2-methoxyacrylat

100 g (675 mmol) Methyl-2,2-dimethoxypropanoat wurden in 300 ml DMF vorgelegt und portionsweise unter Rühren mit 52.7 g (371 mmol) P₂O₅ versetzt und anschließend 1 h auf 100°C erwärmt. Die Reaktionslösung wurde dann zu 1l einer auf 10°C gekühlen, gesättigten NaHCO₃-Lösung gegeben. Diese Lösung wurde mit Diethylether extrahiert, die organischen Extrakte dreimal mit gesättigter NaCl-Lösung gewaschen, über Na₂SO₄ getrocknet, filtriert und eingeengt. Das Produkt wurde ohne weitere Reinigung eingesetzt. Ausb.: 66.0 g (81%)
¹H NMR (CDCl₃) : σ = 3.67 (s,3H,C-CH₃), 3.83 (s, 3H, COOCH₃), 4.63 (d, 1 H, J=3Hz,C=CHH), 5.37 (d, 1 H, J=3Hz, C=CHH).

### Intermediat 7

### Ethyl-2-ethoxyacrylat

Synthese analog zu Intermediat 6.
¹H NMR (CDCl₃) : σ = 1.33 (t, 3H, J=7Hz, CH₃CH₂O), 1.40 (t, 3H, J=7Hz, CH₃CH₂O) 3.83 (q, 2H, J=7Hz, CH₃CH₂O), 4.27 (q, 2H, J=7Hz, CH₃CH₂O), 4.58 (d, 1 H, J=3Hz,C=CHH), 5.32 (d, 1H, J=3Hz, C=CHH).

### Beispiel 3.11-9

### 3-(3,5-Difluorphenyl)-5-methoxy-N-(2,2,2-trifluorethyl)-4,5-dihydro-1,2-oxazol-5-carboth ioam id

400 mg (1.182 mmol) 3-(3,5-Difluorphenyl)-5-methoxy-N-(2,2,2-trifluorethyl)-4,5-dihydro-1,2-oxazol-5-carboxamid wurden in 20 ml THF gelöst und portionsweise mit insgesamt 478 mg (1.182 mmol) Lawessons Reagenz versetzt. Die klare Lösung wurde anschließend in der Microwelle auf 80°C für 2h erhitzt. Das Lösemittel wurde im Vakuum entfernt, der Rückstand in Essigester aufgenommen und mit gesättigter Natriumchloridlösung extrahiert. Die organische Phase wurde über Na₂SO₄getrocknet, filtriert und eingeengt. Anschließend erfolgte die chromatographische Reinigung mit Essigester / n-Heptan über Kieselgel. Ausb.: 150 mg (34%)
1 HNMR (CDCl₃) : σ = 3.41 (s,3H, O-CH₃); 3.57 (d H_{A} von AB, J = 19 Hz, 1 H, N=C-CH_{A}H_{B}), 4.07 ( d H_{B} von AB, J = 19 Hz, 1 H,N=C-CH_{A}H_{B}), 4.26-4.41 (m,1 H, CHH-CF₃); 4.54-4.71 (m,1 H, CHH-CF₃); 6.92 (tt,1 H, pheyl-4H); 7.21 (d,2H, Ar-2,6H); 8.70 (s br, 1H, NH).

In Analogie zu der Herstellung der oben genannten Verbindungen und gemäß den allgemeinen Angaben zur Herstellung sind die in folgenden Tabellen genannten Verbindungen erhältlich. Die NMR-Daten der in diesen Tabellen offenbarten Beispiele werden in der Form (δ-Werte Anzahl der H-Atome, Multiplettauf-spaltung) notiert. Die δ-Wert Signalintensitäts - Zahlenpaare von verschiedenen Signalpeaks werden durch Semikolons voneinander getrennt aufgelistet.

### Tabelle 1.1: Erfindungsgemäße Verbindungen 1.1-1 bis 1.1-266 der allgemeinen Formel (1.1), worin A-X wie nachfolgend definiert ist.

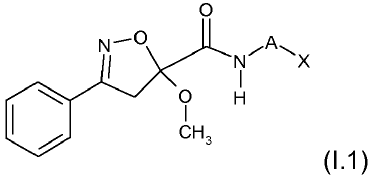

**Tabelle 1.1:**

| Nr. | 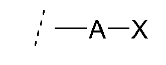 | Nr. | 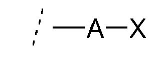 | Nr. | 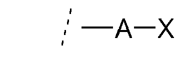 |
|---|---|---|---|---|---|
| 1.1-1 | 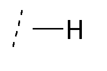 | 1.1-10 | 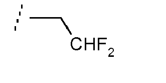 | 1.1-19 | 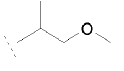 |
| 1.1-2 | 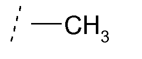 | 1.1-11 | 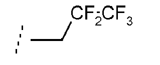 | 1.1-20 | 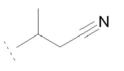 |
| 1.1-3 | 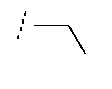 | 1.1-12 | 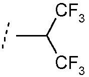 | 1.1-21 | 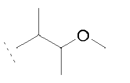 |
| 1.1-4 | 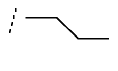 | 1.1-13 | 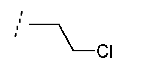 | 1.1-22 | 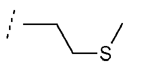 |
| 1.1-5 | 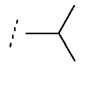 | 1.1-14 | 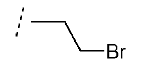 | 1.1-23 | 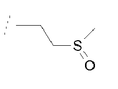 |
| 1.1-6 | 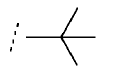 | 1.1-15 | 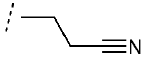 | 1.1-24 | 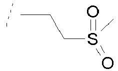 |
| 1.1-7 | 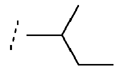 | 1.1-16 | 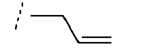 | 1.1-25 | 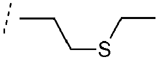 |
| 1.1-8 | 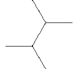 | 1.1-17 | 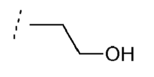 | 1.1-26 | 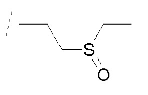 |
| 1.1-9 | 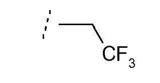 | 1.1-18 | 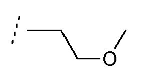 | 1.1-27 | 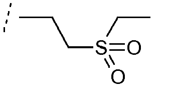 |
| 1.1-28 | 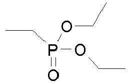 | 1.1-38 | 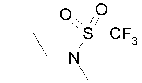 | 1.1-48 | 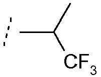 |
| 1.1-29 | 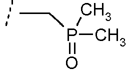 | 1.1-39 | 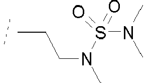 | 1.1-49 | 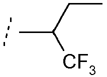 |
| 1.1-30 | 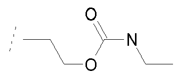 | 1.1-40 | 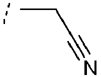 | 1.1-50 | 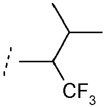 |
| 1.1-31 | 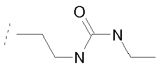 | 1.1-41 | 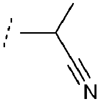 | 1.1-51 | 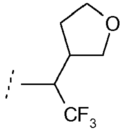 |
| 1.1-32 | 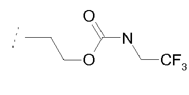 | 1.1-42 | 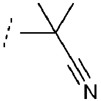 | 1.1-52 | 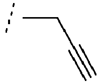 |
| 1.1-33 | 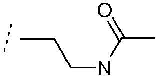 | 1.1-43 | 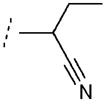 | 1.1-53 | 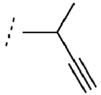 |
| 1.1-34 | 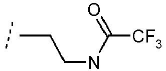 | 1.1-44 | 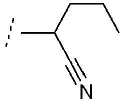 | 1.1-54 | 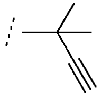 |
| 1.1-35 | 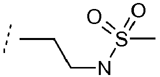 | 1.1-45 | 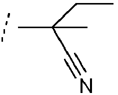 | 1.1-55 | 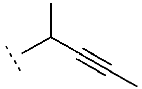 |
| 1.1-36 | 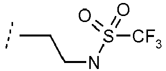 | 1.1-46 | 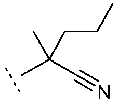 | 1.1-56 | 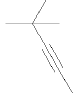 |
| 1.1-37 | 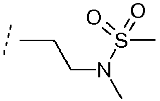 | 1.1-47 | 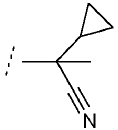 | 1.1-57 | 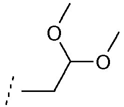 |
| 1.1-58 | 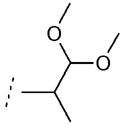 | 1.1-68 | 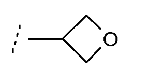 | 1.1-78 | 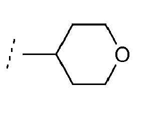 |
| 1.1-59 | 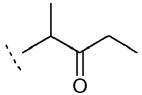 | 1.1-69 | 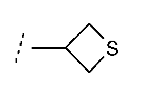 | 1.1-79 | 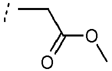 |
| 1.1-60 | 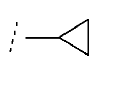 | 1.1-70 | 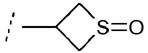 | 1.1-80 | 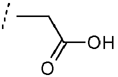 |
| 1.1-61 | 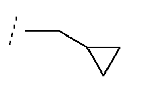 | 1.1-71 | 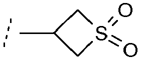 | 1.1-81 | 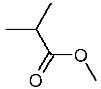 |
| 1.1-62 | 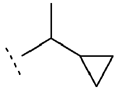 | 1.1-72 | 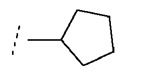 | 1.1-82 | 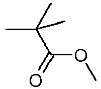 |
| 1.1-63 | 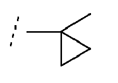 | 1.1-73 | 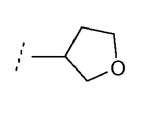 | 1.1-83 | 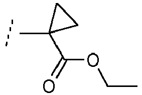 |
| 1.1-64 | 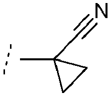 | 1.1-74 | 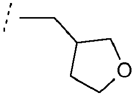 | 1.1-84 | 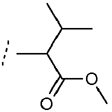 |
| 1.1-65 | 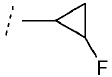 | 1.1-75 | 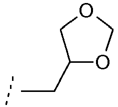 | 1.1-85 | 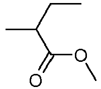 |
| 1.1-66 | 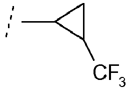 | 1.1-76 | 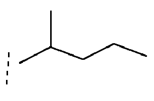 | 1.1-86 | 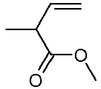 |
| 1.1-67 | 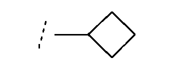 | 1.1-77 | 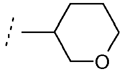 | 1.1-87 | 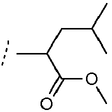 |
| 1.1-88 | 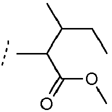 | 1.1-98 | 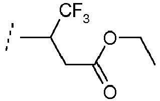 | 1.1-108 | 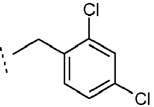 |
| 1.1-89 | 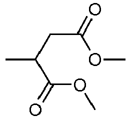 | 1.1-99 | 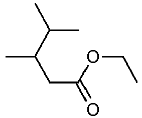 | 1.1-109 | 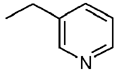 |
| 1.1-90 | 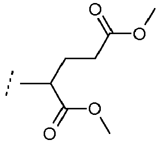 | 1.1-100 | 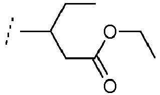 | 1.1-110 | 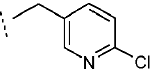 |
| 1.1-91 | 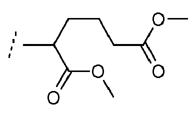 | 1.1-101 | 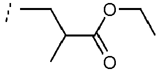 | 1.1-111 | 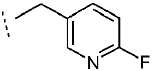 |
| 1.1-92 | 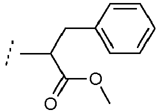 | 1.1-102 | 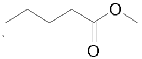 | 1.1-112 | 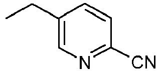 |
| 1.1-93 | 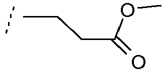 | 1.1-103 | 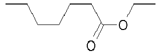 | 1.1-113 | 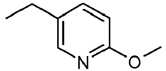 |
| 1.1-94 | 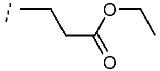 | 1 1.1-04 | 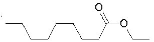 | 1.1-114 | 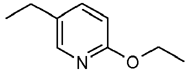 |
| 1.1-95 | 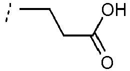 | 1.1-105 | 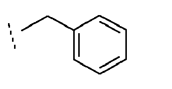 | 1.1-115 | 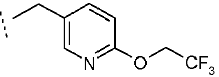 |
| 1.1-96 | 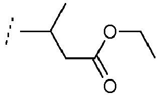 | 1.1-106 | 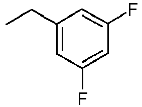 | 1.1-116 | 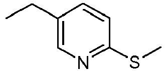 |
| 1.1-97 | 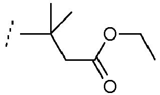 | 1.1-107 | 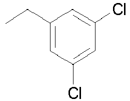 | 1.1-117 | 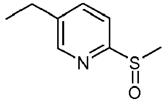 |
| 1.1-118 | 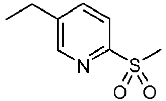 | 1.1-128 | 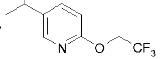 | 1.1-138 | 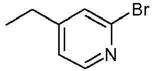 |
| 1.1-119 | 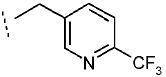 | 1.1-129 | 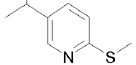 | 1.1-139 | 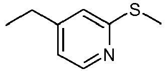 |
| 1.1-120 | 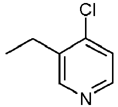 | 1.1-130 | 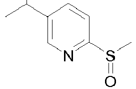 | 1.1-140 | 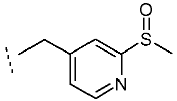 |
| 1.1-121 | 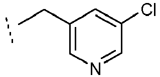 | 1.1-131 | 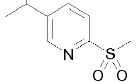 | 1.1-141 | 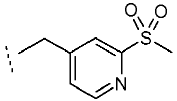 |
| 1.1-122 | 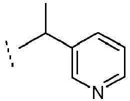 | 1.1-132 | 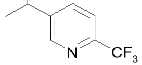 | 1.1-142 | 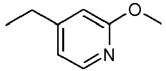 |
| 1.1-123 | 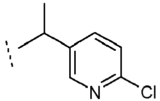 | 1.1-133 | 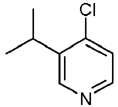 | 1.1-143 | 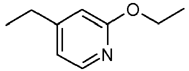 |
| 1.1-124 | 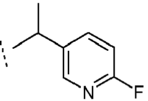 | 1.1-134 | 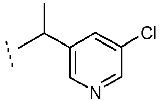 | 1.1-144 | 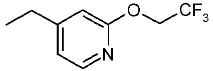 |
| 1.1-125 | 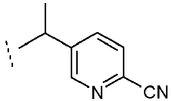 | 1.1-135 | 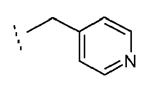 | 1.1-145 | 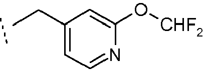 |
| 1.1-126 | 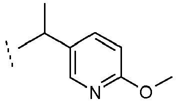 | 1.1-136 | 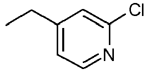 | 1.1-146 | 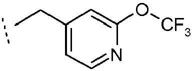 |
| 1.1-127 | 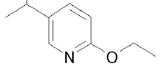 | 1.1-137 | 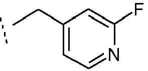 | 1.1-147 | 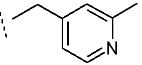 |
| 1.1-148 | 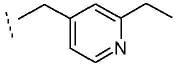 | 1.1-158 | 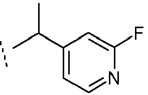 | 1.1-168 | 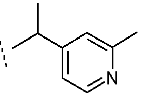 |
| 1.1-149 | 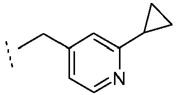 | 1.1-159 | 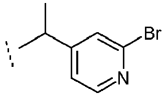 | 1.1-169 | 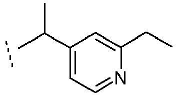 |
| 1.1-150 | 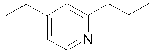 | 1.1-160 | 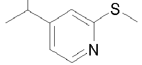 | 1.1-170 | 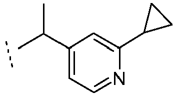 |
| 1.1-151 | 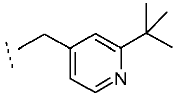 | 1.1-161 | 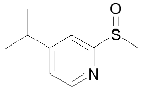 | 1.1-171 | 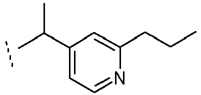 |
| 1.1-152 | 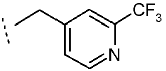 | 1.1-162 | 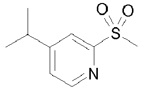 | 1.1-172 | 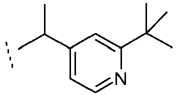 |
| 1.1-153 | 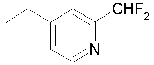 | 1.1-163 | 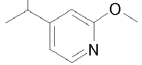 | 1.1-173 | 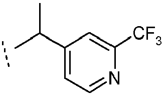 |
| 1.1-154 | 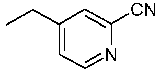 | 1.1-164 | 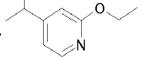 | 1.1-174 | 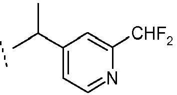 |
| 1.1-155 | 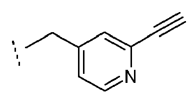 | 1.1-165 | 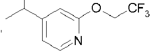 | 1.1-175 | 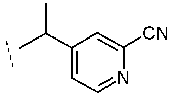 |
| 1.1-156 | 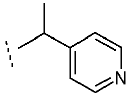 | 1.1-166 | 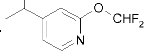 | 1.1-176 | 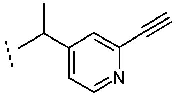 |
| 1.1-157 | 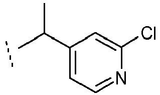 | 1.1-167 | 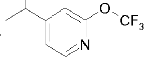 | 1.1-177 | 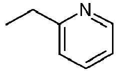 |
| 1.1-178 | 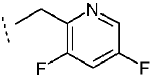 | 1.1-188 | 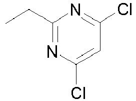 | 1.1-198 | 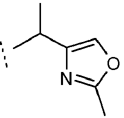 |
| 1.1-179 | 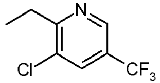 | 1.1-189 | 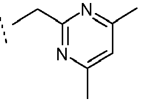 | 1.1-199 | 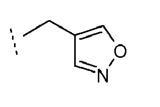 |
| 1.1-180 | 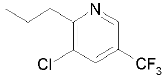 | 1.1-190 | 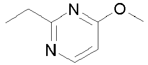 | 1.1-200 | 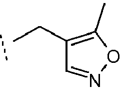 |
| 1.1-181 | 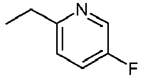 | 1.1-191 | 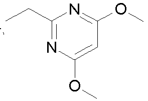 | 1.1-201 | 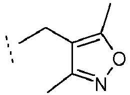 |
| 1.1-182 | 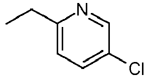 | 1.1-192 | 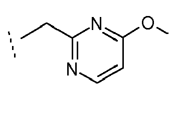 | 1.1-202 | 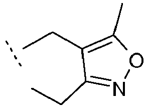 |
| 1.1-183 | 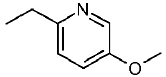 | 1.1-193 | 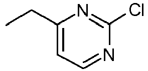 | 1.1-203 | 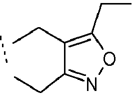 |
| 1.1-184 | 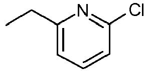 | 1.1-194 | 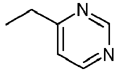 | 1.1-204 | 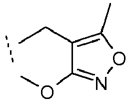 |
| 1.1-185 | 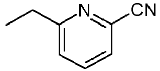 | 1.1-195 | 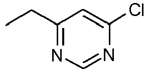 | 1.1-205 | 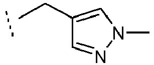 |
| 1.1-186 | 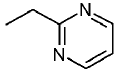 | 1.1-196 | 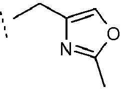 | 1.1-206 | 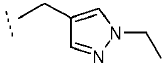 |
| 1.1-187 | 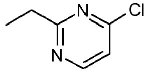 | 1.1-197 | 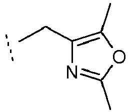 | 1.1-207 | 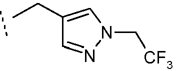 |
| 1.1-208 | 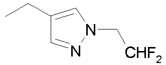 | 1.1-218 | 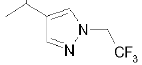 | 1.1-228 | 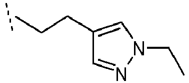 |
| 1.1-209 | 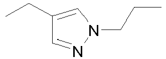 | 1.1-219 | 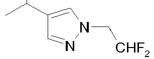 | 1.1-229 | 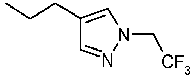 |
| 1.1-210 | 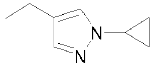 | 1.1-220 | 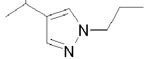 | 1.1-230 | 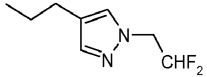 |
| 1.1-211 | 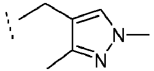 | 1.1-221 | 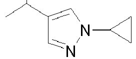 | 1.1-231 | 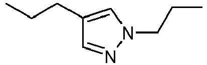 |
| 1.1-212 | 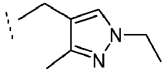 | 1.1-222 | 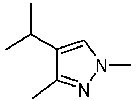 | 1.1-232 | 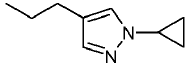 |
| 1.1-213 | 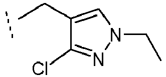 | 1.1-223 | 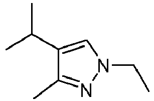 | 1.1-233 | 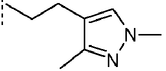 |
| 1.1-214 | 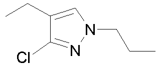 | 1.1-224 | 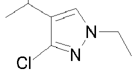 | 1.1-234 | 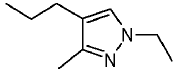 |
| 1.1-215 | 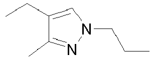 | 1.1-225 | 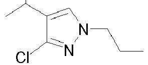 | 1.1-235 | 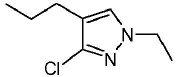 |
| 1.1-216 | 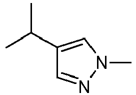 | 1.1-226 | 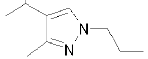 | 1.1-236 | 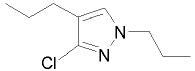 |
| 1.1-217 | 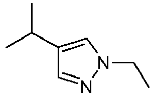 | 1.1-227 | 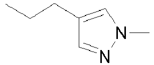 | 1.1-237 | 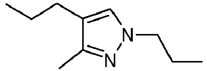 |
| 1.1-238 | 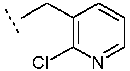 | 1.1-248 | 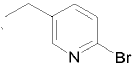 | 1.1-258 | 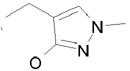 |
| 1.1-239 | 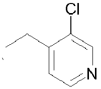 | 1.1-249 | 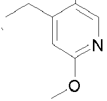 | 1.1-259 | 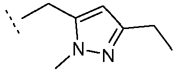 |
| 1.1-240 | 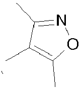 | 1.1-250 | 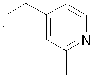 | 1.1-260 | 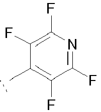 |
| 1.1-241 | 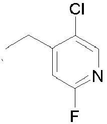 | 1.1-251 | 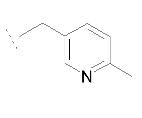 | 1.1-261 | 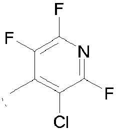 |
| 1.1-242 | 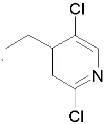 | 1.1-252 | 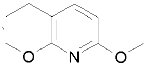 | 1.1-262 | 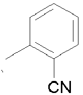 |
| 1.1-243 | 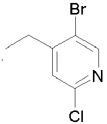 | 1.1-253 | 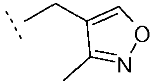 | 1.1-263 | 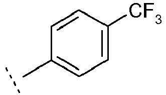 |
| 1.1-244 | 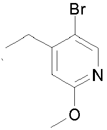 | 1.1-254 | 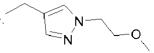 | 1.1-264 | 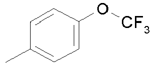 |
| 1.1-245 | 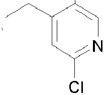 | 1.1-255 | 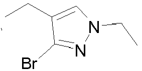 | 1.1-265 | 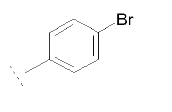 |
| 1.1-246 | 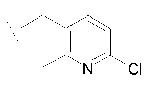 | 1.1-256 | 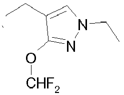 | 1.1-266 | 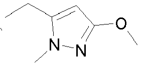 |
| 1.1-247 | 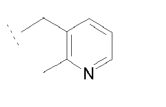 | 1.1-257 | 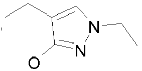 | | |

### Tabelle 1.2: Erfindungsgemäße Verbindungen 1.2-1 bis 1.2-266 der allgemeinen Formel (I.2), worin A-X wie in Tabelle 1.1 definiert ist.

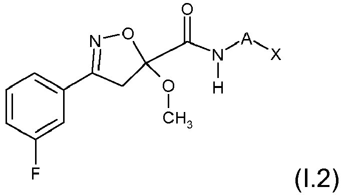

### Tabelle 1.3: Erfindungsgemäße Verbindungen 1.3-1 bis 1.3-266 der allgemeinen Formel (I.3), worin A-X wie in Tabelle 1.1 definiert ist.

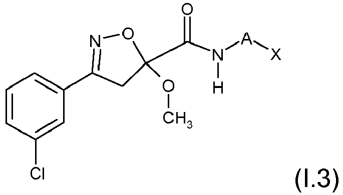

### Tabelle 1.4: Erfindungsgemäße Verbindungen 1.4-1 bis 1.4-266 der allgemeinen Formel (I.4), worin A-X wie in Tabelle 1.1 definiert ist.

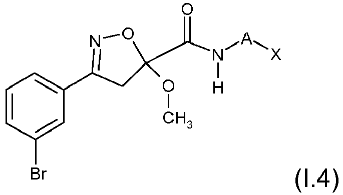

### Tabelle 1.5: Erfindungsgemäße Verbindungen 1.5-1 bis 1.5-266 der allgemeinen Formel (I.5), worin A-X wie in Tabelle 1.1 definiert ist.

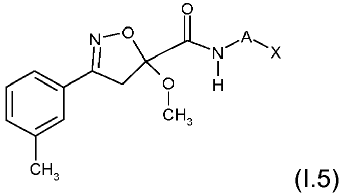

### Tabelle 1.6: Erfindungsgemäße Verbindungen 1.6-1 bis 1.6-266 der allgemeinen Formel (I.6), worin A-X wie in Tabelle 1.1 definiert ist.

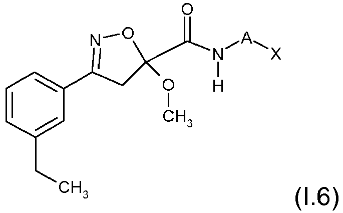

### Tabelle 1.7: Erfindungsgemäße Verbindungen 1.7-1 bis 1.7-266 der allgemeinen Formel (I.7), worin A-X wie in Tabelle 1.1 definiert ist.

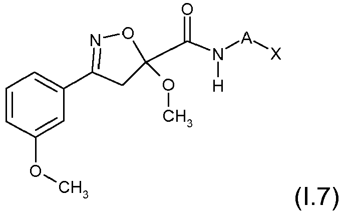

### Tabelle 1.8: Erfindungsgemäße Verbindungen 1.8-1 bis 1.8-266 der allgemeinen Formel (I.8), worin A-X wie in Tabelle 1.1 definiert ist.

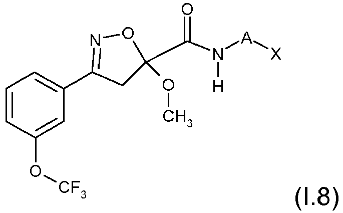

### Tabelle 1.9: Erfindungsgemäße Verbindungen 1.9-1 bis 1.9-266 der allgemeinen Formel (I.9), worin A-X wie in Tabelle 1.1 definiert ist.

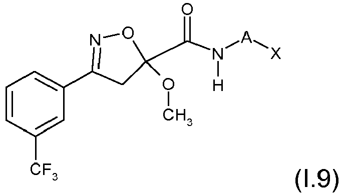

### Tabelle 1.10: Erfindungsgemäße Verbindungen 1.10-1 bis 1.10-266 der allgemeinen Formel (I.10), worin A-X wie in Tabelle 1.1 definiert ist.

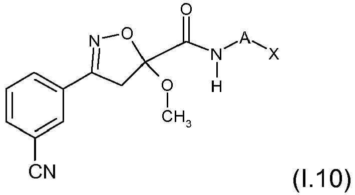

### Tabelle 1.11: Erfindungsgemäße Verbindungen 1.11-1 bis 1.11-266 der allgemeinen Formel (I.11), worin A-X wie in Tabelle 1.1 definiert ist.

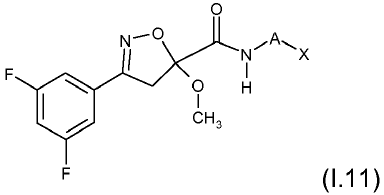

### Tabelle 1.12: Erfindungsgemäße Verbindungen 1.12-1 bis 1.12-266 der allgemeinen Formel (I.12), worin A-X wie in Tabelle 1.1 definiert ist.

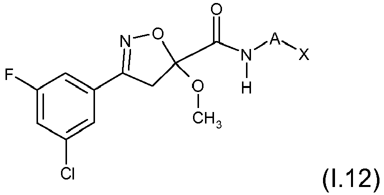

### Tabelle 1.13: Erfindungsgemäße Verbindungen 1.13-1 bis 1.13-266 der allgemeinen Formel (I.13), worin A-X wie in Tabelle 1.1 definiert ist.

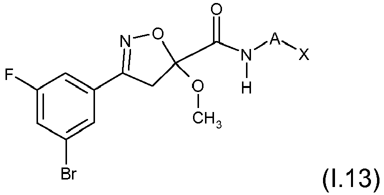

### Tabelle 1.14: Erfindungsgemäße Verbindungen 1.14-1 bis 1.14-266 der allgemeinen Formel (I.14), worin A-X wie in Tabelle 1.1 definiert ist.

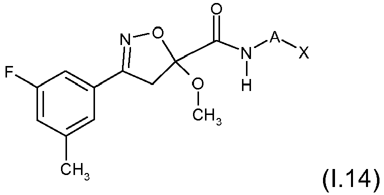

### Tabelle 1.15: Erfindungsgemäße Verbindungen 1.15-1 bis 1.15-266 der allgemeinen Formel (I.15), worin A-X wie in Tabelle 1.1 definiert ist.

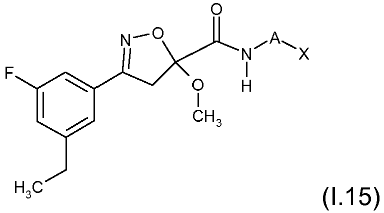

### Tabelle 1.16: Erfindungsgemäße Verbindungen 1.16-1 bis 1.16-266 der allgemeinen Formel (I.16), worin A-X wie in Tabelle 1.1 definiert ist.

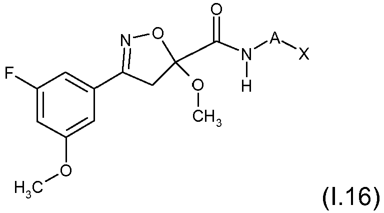

### Tabelle 1.17: Erfindungsgemäße Verbindungen 1.17-1 bis 1.17-266 der allgemeinen Formel (I.17), worin A-X wie in Tabelle 1.1 definiert ist.

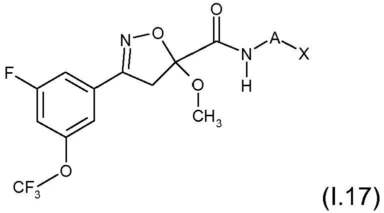

### Tabelle 1.18: Erfindungsgemäße Verbindungen 1.18-1 bis 1.18-266 der allgemeinen Formel (I.18), worin A-X wie in Tabelle 1.1 definiert ist.

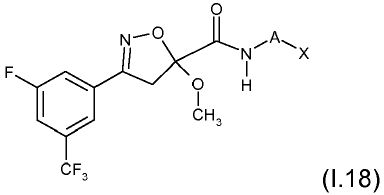

### Tabelle 1.19: Erfindungsgemäße Verbindungen 1.19-1 bis 1.19-266 der allgemeinen Formel (I.19), worin A-X wie in Tabelle 1.1 definiert ist.

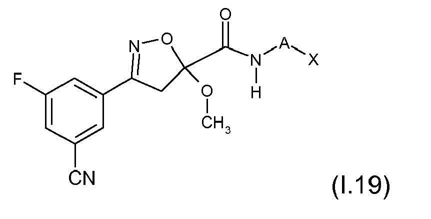

### Tabelle 1.20: Erfindungsgemäße Verbindungen 1.20-1 bis 1.20-266 der allgemeinen Formel (I.20), worin A-X wie in Tabelle 1.1 definiert ist.

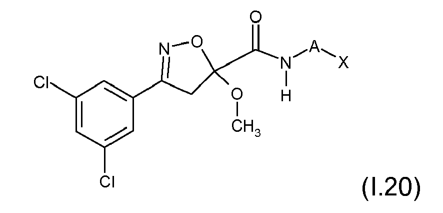

### Tabelle 1.21: Erfindungsgemäße Verbindungen 1.21-1 bis 1.21-266 der allgemeinen Formel (I.21), worin A-X wie in Tabelle 1.1 definiert ist.

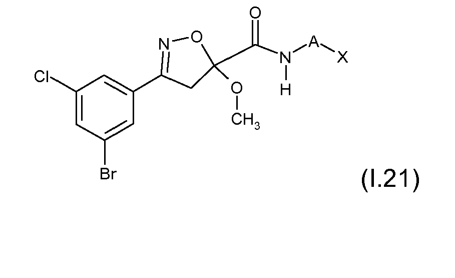

### Tabelle 1.22: Erfindungsgemäße Verbindungen 1.22-1 bis 1.22-266 der allgemeinen Formel (I.22), worin A-X wie in Tabelle 1.1 definiert ist.

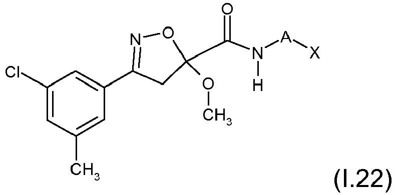

### Tabelle 1.23: Erfindungsgemäße Verbindungen 1.23-1 bis 1.23-266 der allgemeinen Formel (I.23), worin A-X wie in Tabelle 1.1 definiert ist.

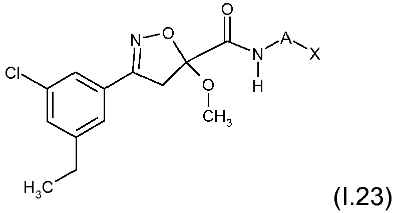

### Tabelle 1.24: Erfindungsgemäße Verbindungen 1.24-1 bis 1.24-266 der allgemeinen Formel (I.24), worin A-X wie in Tabelle 1.1 definiert ist.

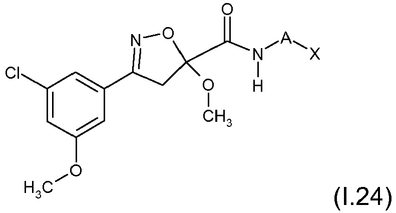

### Tabelle 1.25: Erfindungsgemäße Verbindungen 1.25-1 bis 1.25-266 der allgemeinen Formel (I.25), worin A-X wie in Tabelle 1.1 definiert ist.

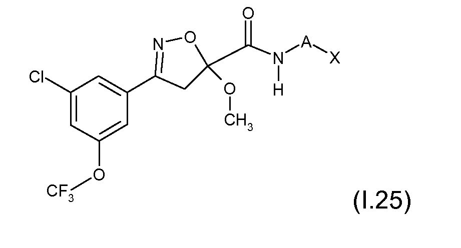

### Tabelle 1.26: Erfindungsgemäße Verbindungen 1.26-1 bis 1.26-266 der allgemeinen Formel (I.26), worin A-X wie in Tabelle 1.1 definiert ist.

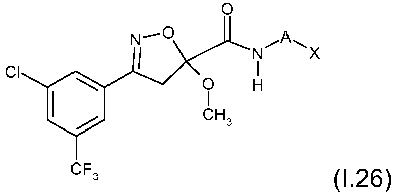

### Tabelle 1.27: Erfindungsgemäße Verbindungen 1.27-1 bis 1.27-266 der allgemeinen Formel (I.27), worin A-X wie in Tabelle 1.1 definiert ist.

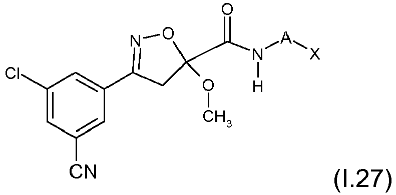

### Tabelle 1.28: Erfindungsgemäße Verbindungen 1.28-1 bis 1.28-266 der allgemeinen Formel (I.28), worin A-X wie in Tabelle 1.1 definiert ist.

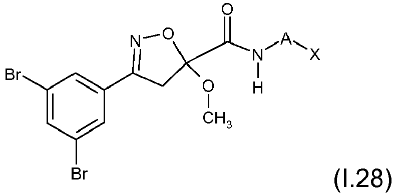

### Tabelle 1.29: Erfindungsgemäße Verbindungen 1.29-1 bis 1.29-266 der allgemeinen Formel (I.29), worin A-X wie in Tabelle 1.1 definiert ist.

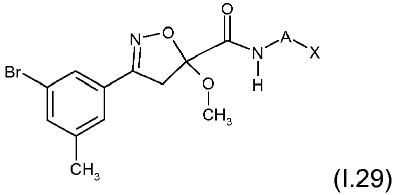

### Tabelle 1.30: Erfindungsgemäße Verbindungen 1.30-1 bis 1.30-266 der allgemeinen Formel (1.30), worin A-X wie in Tabelle 1.1 definiert ist.

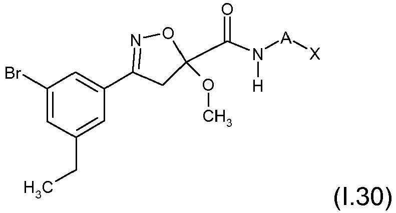

### Tabelle 1.31: Erfindungsgemäße Verbindungen 1.31-1 bis 1.31-266 der allgemeinen Formel (I.31), worin A-X wie in Tabelle 1.1 definiert ist.

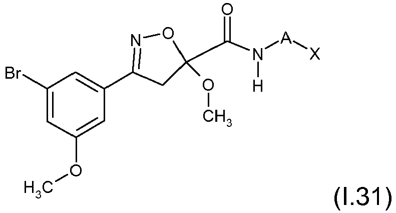

### Tabelle 1.32: Erfindungsgemäße Verbindungen 1.32-1 bis 1.32-266 der allgemeinen Formel (I.32), worin A-X wie in Tabelle 1.1 definiert ist.

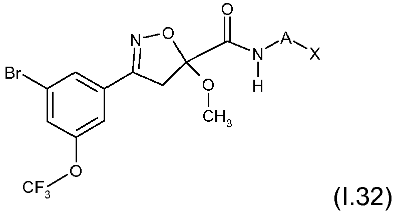

### Tabelle 1.33: Erfindungsgemäße Verbindungen 1.33-1 bis 1.33-266 der allgemeinen Formel (I.33), worin A-X wie in Tabelle 1.1 definiert ist.

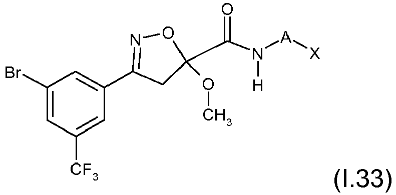

### Tabelle 1.34: Erfindungsgemäße Verbindungen 1.34-1 bis 1.34-266 der allgemeinen Formel (I.34), worin A-X wie in Tabelle 1.1 definiert ist.

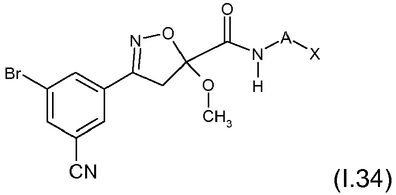

### Tabelle 1.35: Erfindungsgemäße Verbindungen 1.35-1 bis 1.35-266 der allgemeinen Formel (I.35), worin A-X wie in Tabelle 1.1 definiert ist.

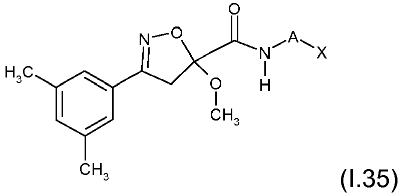

### Tabelle 1.36: Erfindungsgemäße Verbindungen 1.36-1 bis 1.36-266 der allgemeinen Formel (I.36), worin A-X wie in Tabelle 1.1 definiert ist.

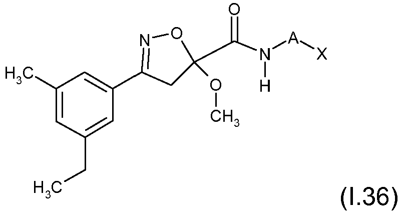

### Tabelle 1.37: Erfindungsgemäße Verbindungen 1.37-1 bis 1.37-266 der allgemeinen Formel (I.37), worin A-X wie in Tabelle 1.1 definiert ist.

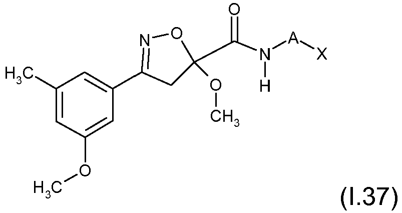

### Tabelle 1.38: Erfindungsgemäße Verbindungen 1.38-1 bis 1.38-266 der allgemeinen Formel (I.38), worin A-X wie in Tabelle 1.1 definiert ist.

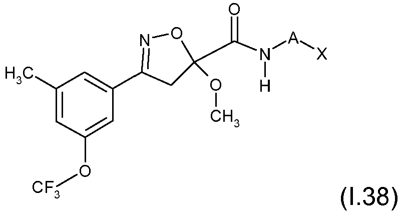

### Tabelle 1.39: Erfindungsgemäße Verbindungen 1.39-1 bis 1.39-266 der allgemeinen Formel (I.39), worin A-X wie in Tabelle 1.1 definiert ist.

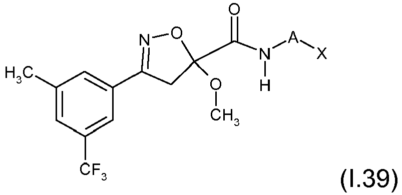

### Tabelle 1.40: Erfindungsgemäße Verbindungen 1.41-1 bis 1.41-266 der allgemeinen Formel (I.41), worin A-X wie in Tabelle 1.1 definiert ist.

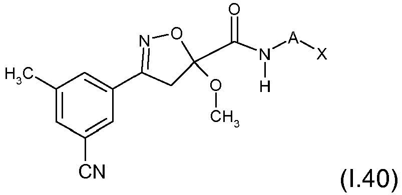

### Tabelle 1.41: Erfindungsgemäße Verbindungen 1.41-1 bis 1.41-266 der allgemeinen Formel (I.41), worin A-X wie in Tabelle 1.1 definiert ist.

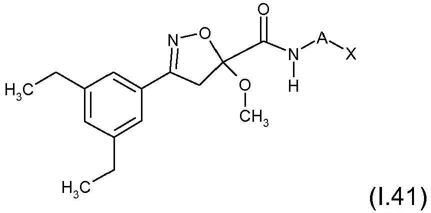

### Tabelle 1.42: Erfindungsgemäße Verbindungen 1.42-1 bis 1.42-266 der allgemeinen Formel (I.42), worin A-X wie in Tabelle 1.1 definiert ist.

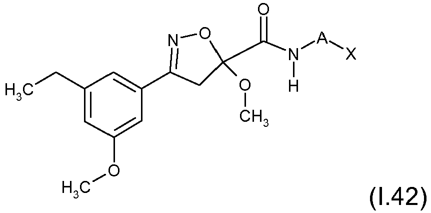

### Tabelle 1.43: Erfindungsgemäße Verbindungen 1.43-1 bis 1.43-266 der allgemeinen Formel (I.43), worin A-X wie in Tabelle 1.1 definiert ist.

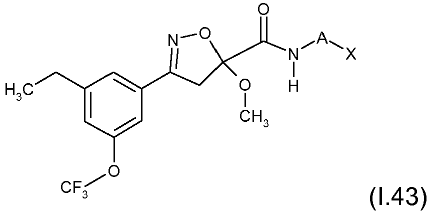

### Tabelle I.44: Erfindungsgemäße Verbindungen 1.44-1 bis I.44-266 der allgemeinen Formel (I.44), worin A-X wie in Tabelle 1.1 definiert ist.

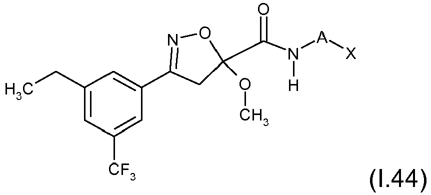

### Tabelle I.45: Erfindungsgemäße Verbindungen 1.45-1 bis I.45-266 der allgemeinen Formel (I.45), worin A-X wie in Tabelle 1.1 definiert ist.

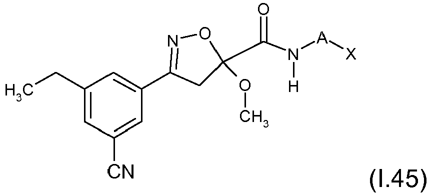

### Tabelle 1.46: Erfindungsgemäße Verbindungen 1.46-1 bis I.46-266 der allgemeinen Formel (I.46), worin A-X wie in Tabelle 1.1 definiert ist.

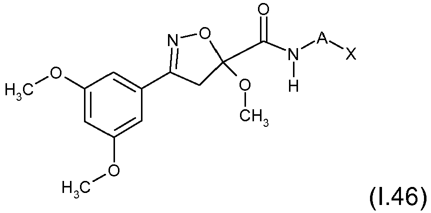

### Tabelle 1.47: Erfindungsgemäße Verbindungen 1.47-1 bis 1.47-266 der allgemeinen Formel (I.47), worin A-X wie in Tabelle 1.1 definiert ist.

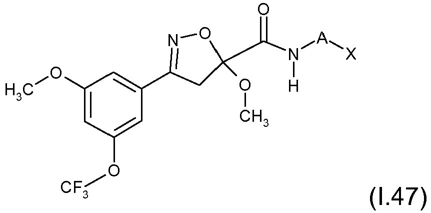

### Tabelle 1.48: Erfindungsgemäße Verbindungen 1.48-1 bis 1.48-266 der allgemeinen Formel (I.48), worin A-X wie in Tabelle 1.1 definiert ist.

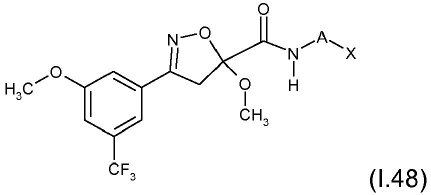

### Tabelle 1.49: Erfindungsgemäße Verbindungen 1.49-1 bis 1.49-266 der allgemeinen Formel (I.49), worin A-X wie in Tabelle 1.1 definiert ist.

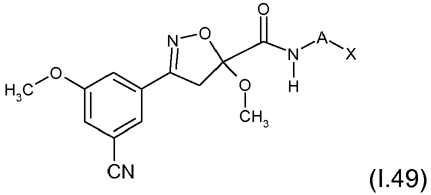

### Tabelle 1.50: Erfindungsgemäße Verbindungen 1.50-1 bis 1.50-266 der allgemeinen Formel (1.50), worin A-X wie in Tabelle 1.1 definiert ist.

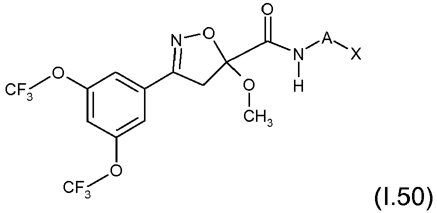

### Tabelle 1.51: Erfindungsgemäße Verbindungen 1.51-1 bis 1.51-266 der allgemeinen Formel (1.51), worin A-X wie in Tabelle 1.1 definiert ist.

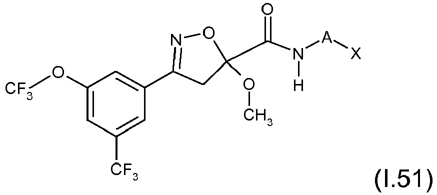

### Tabelle 1.52: Erfindungsgemäße Verbindungen 1.52-1 bis 1.52-266 der allgemeinen Formel (I.52), worin A-X wie in Tabelle 1.1 definiert ist.

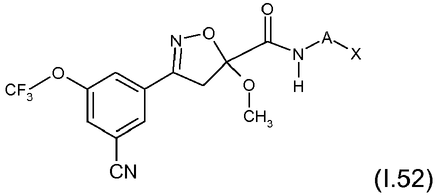

### Tabelle 1.53: Erfindungsgemäße Verbindungen 1.53-1 bis 1.53-266 der allgemeinen Formel (I.53), worin A-X wie in Tabelle 1.1 definiert ist.

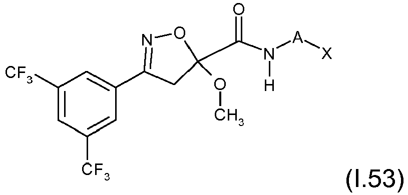

### Tabelle 1.54: Erfindungsgemäße Verbindungen 1.54-1 bis 1.54-266 der allgemeinen Formel (I.54), worin A-X wie in Tabelle 1.1 definiert ist.

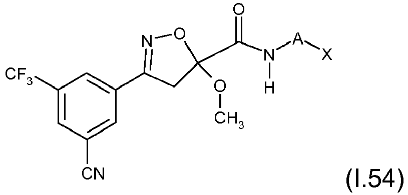

### Tabelle 1.55: Erfindungsgemäße Verbindungen 1.55-1 bis 1.55-266 der allgemeinen Formel (I.55), worin A-X wie in Tabelle 1.1 definiert ist.

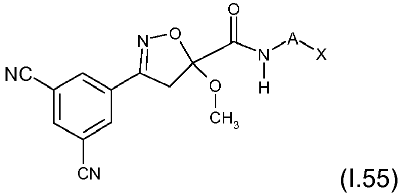

### Tabelle 1.56: Erfindungsgemäße Verbindungen 1.56-1 bis 1.56-266 der allgemeinen Formel (I.56), worin A-X wie in Tabelle 1.1 definiert ist.

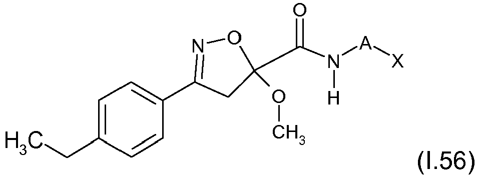

### Tabelle 1.57: Erfindungsgemäße Verbindungen 1.57-1 bis 1.57-266 der allgemeinen Formel (I.57), worin A-X wie in Tabelle 1.1 definiert ist.

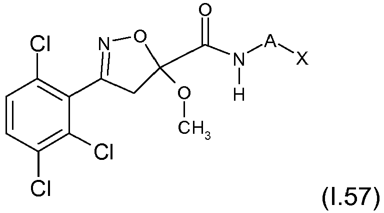

Analog zu den in den Tabellen 1.1 bis 1.57 genannten erfindungsgemäßen Verbindungen ergeben sich entsprechend die erfindungsgemäßen Verbindungen der Tabellen 2.1 bis 2.57, worin Y für Sauerstoff steht, R³ für Ethyl steht, und die anderen Substituenten jeweils die in den Tabellen 1.1 bis 1.57 angegebenen Bedeutungen haben.

Analog zu den in den Tabellen 1.1 bis 1.57 beschriebenen erfindungsgemäßen Verbindungen, ergeben sich entsprechend die erfindungsgemässen Verbindungen der

Tabellen 3.1 bis 3.57, worin Y für Schwefel steht, R³ für Methyl steht, und die anderen Substituenten jeweils die in den Tabellen 1.1 bis 1.57 angegebenen Bedeutungen haben.

Analog zu den in den Tabellen 1.1 bis 1.57 beschriebenen erfindungsgemässen Verbindungen, ergeben sich entsprechend die erfindungsgemässen Verbindungen der Tabellen 4.1 bis 4.57, worin Y für Schwefel steht, R³ für Ethyl steht, und die anderen Substituenten jeweils die in den Tabellen 1.1 bis 1.57 angegebenen Bedeutungen haben.

Die verwendeten Abkürzungen bedeuten:

| | | | | | | | |
|---|---|---|---|---|---|---|---|
| Ac | Acetoxy | Bu | Butyl | Et | Ethyl | Me | Methyl |
| Pr | Propyl | Pen | Pentyl | Hex | Hexyl | Ph | Phenyl |
| c | cyclo | s | sekundär | i | iso | t | tertiär |
| THF | Tetrahydrofuran | | | | | | |

Mit E1, E2, E3, E4 werden enantiomerenreine Verbindungen bezeichnet. D1, D2, D3, D4 bezeichnen Diastereomere eines Diastereomerenpaares, die als Racemate zweier Enantiomere vorliegen.

**Analytische Daten Tabelle 1.2**

| Nr. | NMR |
|---|---|
| 1.2-7 | [CDCl₃] 0.89-0.97 (m,3H); 1.17-1.21 (m,3H); 1.52 (m,2H); 3.38 (s,3H); 3.42 (m,1H); 3.84 (m,1H); 3.96 (m,1H); 6.58 (d,1H); 7.16 (m,1H); 7.42 (m,3H). |
| 1.2-8 | [CDCl₃] 0.93 (m,6H); 1.14 (m,3H); 1.76 (m,1H); 3.38 (s,3H); 3.42 (m,1H); 3.78-3.86 (m,1H), 3.91 (m,1H); 6.61 (d,1H); 7.16 (m,1H); 7.41 (m,3H). |
| 1.2-10 | [CDCl₃] 3.38 (s,3H); 3.44 (d,1H); 3.62-3.79 (m,2H); 3.90 (d,1H); 5.77-6.05 (tt,1H); 7.06 (s,1H); 7.15 (m,1H); 7.42 (m,3H). |
| 1.2-11 | [CDCl₃] 3.39 (s,3H); 3.44 (d,1H); 3.85 (d,1H), 3.90-4.00 (m,1H); 4.10-4.20 (m,1H); 7.05 (s,1H); 7.17 (m,1H); 7.42 (m,3H). |
| 1.2-15 | [CDCl₃] 2.67-2.73 (m,2H); 3.38 (s,3H); 3.42 (d,1H); 3.59-3.68 (m,2H); 3.91 (d,1H); 7.18 (m,2H); 7.42 (m,3H). |
| 1.2-16 | [CDCl₃] 3.39 (s,3H); 3.44 (d,1H); 3.89 (d,1H); 3.92-4.03 (m,2H); 5.18-5.27 (m,2H); 5.82-5.91 (m,1H); 6.88 (s,1H); 7.15 (m,1H); 7.41 (m,3H). |
| 1.2-20 | [CDCl₃] 1.43 (m,3H); 2.57-2.67 (m,1H); 2.75-2.90 (m,1H); 3.38 (s,3H); 3.40 (m,1H); 3.88 (m,1H); 4.29 (m,1H); 6.86 (t,1H); 7.16 (m,1H); 7.42 (m,3H). |
| 1.2-21 | [CDCl₃] 1.13 (m,6H); 1.22 (m,1H); 3.34-3.44 (m,7H); 3.77-3.87 (m,1H); 4.05 (m,1H); 7.02 (t,1H); 7.13 (m,1H); 7.41 (m,3H). |
| 1.2-24 | [CDCl₃] 3.00 (s,3H); 3.26-3.38 (m,6H); 3.81-3.95 (m,3H); 7.16 (m,1H); 7.41 (m,4H). |
| 1.2-25 | [CDCl₃] 1.28 (t,3H); 2.58 (q,2H); 2.72 (t,2H); 3.39 (s,3H); 3.42 (d,1H); 3.55 (m,2H); 3.88 (d,1H); 7.13 (m,2H); 7.41 (m,3H). |
| 1.2-40 | [CDCl₃] 3.38 (s,3H); 3.44 (d,1 H); 3.94 (d, 1 H); 4.18-4.24 (m,1 H); 4.32-4.38 (m, 1 H); 7.19 (m,2H); 7.41 (m,3H). |
| 1.2-41 D1 | [CDCl₃] D1: 1.65 (d,3H); 3.37 (s,3H); 3.45 (d,1H); 3.89 (d,1H); 4.95 (m,1H); 7.09 (d,1H); 7.17 (m,1H); 7.42 (m,3H). |
| 1.2-41 D2 | [CDCl₃] D2: 1.63 (d,3H); 3.39 (s,3H); 3.41 (d,1H); 3.97 (d,1H); 4.93 (m,1H); 7.08 (d,1H); 7.17 (m,1H); 7.43 (m,3H). |
| 1.2-42 | [CDCl₃] 1.78 (s,6H); 3.38 (s,3H); 3.40 (d,1H); 3.99 (d,1H); 6.73 (s,1H); 7.17 (m,1H); 7.42 (m,3H). |
| 1.2-45 | [CDCl₃] 1.09-1.17 (m,3H); 1.73 (s,3H); 1.96-2.12 (m,2H); 3.38 (s,3H); 3.40 (m,1H); 3.94-4.00 (m,1 H); 6.80 (d, 1 H); 7.16 (m, 1 H); 7.43 (m,3H). |
| 1.2-46 | [CDCl₃] 1.03 (m,3H); 1.53-1.59 (m,2H); 1.75 (s,3H); 1.88-2.07 (m,2H); 3.38 (s,3H); 3.40 (m,1H); 3.98 (m,1H); 6.81 (d,1H); 7.16 (m,1H); 7.42 (m,3H). |
| 1.2-47 | [CDCl₃] 0.71-0.78 (m,4H); 1.31 (m,1H); 1.83 (d,3H); 3.38 (s,3H); 3.41 (m,1H); 3.90-4.01 (m,1H), 7.01 (d,1H); 7.17 (m,1H); 7.42 (m,3H). |
| 1.2-48 D1 | [CDCl₃] D1: 1.40 (d,3H); 3.39 (s,3H); 3.45 (d,1H); 3.83 (d,1H); 4.73 (m,1H); 6.83 (d,1H); 7.18 (m,1H); 7.42 (m,3H). |
| 1.2-48 D2 | [CDCl₃] D2: 1.39 (d,3H); 3.38 (s,3H); 3.41 (d,1H); 3.87 (d,1H); 4.73 (m,1H); 6.89 (d,1H); 7.18 (m,1H); 7.42 (m,3H). |
| 1.2-49 D1 | [CDCl₃] D1: 1.04 (t,3H); 1.58-1.65 (m,1H); 1.90-1.98 (m,1H); 3.40 (s,3H); 3.49 (d,1H); 3.80 (d,1H); 4.55 (m,1H); 6.73 (d,1H); 7.17 (m,1H); 7.42 (m,3H). |
| 1.2-49 D2 | [CDCl₃] D2: 1.02 (t,3H); 1.56-1.65 (m,1H); 1.90-1.98 (m,1H); 3.39 (s,3H); 3.45 (d,1H); 3.92 (d,1H); 4.55 (m,1H); 6.78 (d,1H); 7.18 (m,1H); 7.43 (m,3H). |
| 1.2-50 | [CDCl₃] 1.00-1.07 (m,6H); 2.22 (m,1H); 3.39 (d,3H); 3.45 (m,1H); 3.75-3.92 (dd,1H); 4.53 (m,1H); 6.88 (m,1H); 7.17 (m,1H); 7.42 (m,3H). |
| 1.2-51 | [CDCl₃] 1.61-1.66 (m,1H); 1.91 (m,2H); 2.09 (m,1H); 3.38 (s,3H); 3.42 (d,1H); 3.85-3.99 (m,3H); 4.30 (t,1H), 4.65 (quint,1H); 7.17 (m,1H); 7.42 (m,3H). |
| 1.2-53 | [CDCl₃] 1.49 (t,3H); 2.30 (dd,1H); 3.38 (d,3H); 3.43 (m,1H); 3.85 (m,1H); 4.84 (m,1H); 6.96 (s,1H); 7.15 (m,1H); 7.41 (m,3H). |
| 1.2-55 | [CDCl₃] 1.43 (m,3H); 1.81 (m,3H); 3.38 (d,3H); 3.41 (m,1H); 3.83 (m,1H); 4.78 (m,1H); 6.91 (s,1H); 7.17 (m,1H); 7.40 (m,3H). |
| 1.2-58 | [CDCl₃] 1.19 (m,3H); 3.38 (s,3H); 3.41-3.47 (m,7H); 3.88 (m,1H), 4.21 (m,2H), 6.89 (s,1H), 7.15 (m,1H); 7.41 (m,3H). |
| 1.2-59 | [CDCl₃] 1.12 (q,3H); 1.42 (m,3H); 2.49-2.68 (m,2H); 3.37 (s,3H); 3.42 (m,1H); 3.84 (m,1H); 4.63 (m,1H); 7.15 (m,1H); 7.39-7.47 (m,4H). |
| 1.2-60 | [CDCl₃] 0.57-0.61 (m,2H); 0.82-0.87 (m,2H); 2.82 (m,1H); 3.36 (s,3H); 3.41 (d,1H); 3.88 (d,1H); 6.82 (s,1H); 7.14 (m,1H); 7.41 (m,3H). |
| 1.2-62 | [CDCl₃] 0.22-0.30 (m,1H); 0.32-0.40 (m,1 H); 0.44-0.56 (m,2H); 0.87 (m, 1 H); 1.27 (m,3H); 3.38-3.48 (m,5H); 3.78-3.84 (m,1H); 6.79 (s,1H); 7.15 (m,1H); 7.42 (m,3H). |
| 1.2-64 | [CDCl₃] 1.33 (m,2H); 1.62 (m,2H); 3.35 (s,3H); 3.40 (d,1H); 3.99 (d,1H); 7.17 (m,1H); 7.23 (s,1H); 7.41 (m,3H). |
| 1.2-69 | [CDCl₃] 3.36 (d,1H); 3.37 (s,3H); 3.40(m,4H); 3.87(d,1H); 5.22-5.33 (m,1H);7.12-7.20 (m,1H); 7.22(s br,1H); 7.42 (m,3H). |
| 1.2-70 | [CDCl₃] 3.24-3.34(m,2H); 3.40 (s,3H); 3.41 (d,1H); 3.49-3.59 (m,1H); 3,67-3.74(m,1 H); 3.91 (dd,1 H); 4.09-4.19 (m,2H); 4.42-4.54 (m,1 H); 5.15-5.25 (m,1 H); 7.13-7.20(m,1 H); 7.32 (s br,1H); 7.40 (m,1H). |
| 1.2-71 | [CDCl₃] 3.36 (d,1H); 3.37 (s,3H); 3.94 (d,1H); 4.02-4.13 (m, 2H); 4.53-4.13(m,2H); 4.70-4.80 (m,1H); 7.13-7.20 (m,1H); 7.38 (d br,1H); 7.42 (m,3H). |
| 1.2-73 | [CDCl₃] 1.83 -1.92 (m,1 H); 2.26-2.38 (m,1H); 3.36 (s(3H); 3.38 (dd,1H); 3.70-3.76 (m,1H); 3.78-3.91(m,3H); 3.94-4.02(m,1H); 4.51-4.61 (m,1H); 6.95 (s br,1H); 7.18 (m,1H); 7.42 (m,3H). |
| 1.2-76 | [CDCl₃] 0.89-0.96 (m,3H); 1.19 (m,3H); 1.32-1.40 (m,2H); 1.42-1.50 (m,2H); 3.38 (s,3H); 3.41 (m,1H), 3.79-3.86 (m,1H); 4.04 (m,1H); 6.54 (d,1H); 7.15 (m,1H); 7.41 (m,3H). |
| 1.2-94 | [CDCl₃] 1.28 (t,3H); 2.59 (t,2H); 3.36 (s,3H); 3.40 (d,1H); 3.62 (m,2H); 3.83 (d,1H); 4.17 (q,2H); 7.15 (m,1H); 7.28 (s,1H); 7.41 (m,3H). |
| 1.2-96 | [CDCl₃] 1.24-1.32 (m,6H); 2.57 (m,2H); 3.37 (s,3H); 3.40 (d,1H); 3.84 (d,1H); 4.12-4.20 (m,2H); 4.37-4.44 (m,1H); 7.14 (m,1H); 7.41 (m,3H). |
| 1.2-102 | [CDCl₃] 1.88-1.95 (quint,2H); 2.40 (t,2H); 3.37-3.43 (m,6H); 3.69 (s,3H); 3.84 (d,1H); 6.94 (s,1H); 7.15 (m,1H); 7.41 (m,3H). |
| 1.2-119 | [CDCl₃] 3.38 (s,3H); 3.42 (d,1H); 3.96 (d,1H); 4.62 (m,2H); 7.17 (m,1H); 7.28 (s,1H); 7.41 (m,3H); 7.68 (d,1H); 7.86 (d,1H); 8.69 (s,1H). |
| 1.2-138 | [CDCl₃] 3.39 (s,3H), 3.44 (d,1H), 3.99 (d,1H); 4.47-4.58 (m,2H); 7.19 (m,3H); 7.43 (m,4H); 8.34 (d,1H). |
| 1.2-142 | [CDCl₃] 3.39 (s,3H); 3.42 (d,1H), 3.93 (s,3H); 3.93 (d,1H); 4.42-4.56 (m,2H); 6.65 (s,1H); 6.30 (d,1H); 7.13-7.20 (m,2H); 7.43 (m,3H); 8.13 (d,1H). |
| 1.2-147 | [CDCl₃] 2.58 (s,3H); 3.39 (s,3H); 3.42 (d,1H); 3.95 (d,1H), 4.51 (m,2H); 7.05 (s,1H); 7.10 (s,1H); 7.18 (m,2H); 7.41 (m,3H); 7.48 (d,1H). |
| 1.2-148 | [CDCl₃] 1.30 (t,3H); 2.83 (q,2H); 3.39 (s,3H); 3.42 (d,1H); 3.95 (d,1H); 4.51 (m,2H); 7.05 (d,1H); 7.09 (s,1H); 7.17 (m,2H); 7.42 (m,3H); 8.50 (d,1H). |
| 1.2-149 | [CDCl₃] 1.01 (m,4H); 2.03 (m,1H); 3.39 (s,3H); 3.42 (d,1H); 3.97 (d,1H); 4.50 (m,2H); 6.95 (d,1H); 7.06 (s,1H); 7.17 (m,2H); 7.41 (m,3H); 8.40 (d,1H). |
| 1.2-150 | [CDCl₃] 0.97 (t,3H); 1.70-1.80 (m,2H); 2.78 (t,2H); 3.39 (s,3H); 3.42 (d,1H); 3.94 (d,1H); 4.51 (m,2H); 7.05 (d,1H); 7.08 (s,1H); 7.18 (m,2H); 7.41 (m,3H); 8.50 (d,1H). |
| 1.2-200 | [CDCl₃] 2.46 (s,3H); 3.35 (s,3H); 3.39 (d,1H); 3.88 (d,1H); 4.22-4.38 (m,2H); 7.01 (s br,1H); 7.11-7.20 (m,1H); 7.42 (m,3H); 8.20 (s,1H). |
| 1.2-201 | [CDCl₃] 2.27 (s,3H); 2.42 (s,3H); 3.34 (s,3H); 3.40 (d,1H); 3.93 (d,1H); 4.27 (m,2H); 6.81 (s,1H); 7.17 (m, 1H); 7.41 (m, 3H). |
| 1.2-202 | [CDCl₃] 1.30 (t,3H); 2.42 (s,3H); 2.66 (q,2H); 3.34 (s,3H), 3.40 (d,1H); 3.92 (d,1H); 4.28 (m,2H); 6.80 (s,1H); 7.17 (m,1H); 7.41 (m,3H). |
| 1.2-203 | [CDCl₃] 1.30 (m,6H); 2.66 (q,2H); 2.79 (q,2H); 3.34 (s,3H); 3.40 (d,1H); 3.92 (d,1H); 4.28 (m,2H); 6.79 (s,1H); 7.16 (m,1H); 7.42 (m,3H). |
| 1.2-206 | [CDCl₃] 1.48 (t,3H); 3.35 (s,3H); 3.42 (d,1H); 3.89 (d,1H); 4.14 (q,2H); 4.38 (m,2H); 6.96 (s,1H); 7.16 (m,1H); 7.40 (m,4H); 7.46 (s,1H). |
| 1.2-207 | [CDCl₃] 3.35 (s,3H); 3.41 (d,1H); 3.90 (d,1H); 4.37-4.43 (m,2H); 4.68 (q,2H); 7.02 (s,1H); 7.18 (m,1H); 7.41 (m,3H); 7.53 (s,1H); 7.56 (s,1H). |
| 1.2-210 | [CDCl₃] 1.02 (m,2H); 1.11 (m,2H); 3.35 (s,3H); 3.41 (d,1H); 3.57 (m,1H); 3.89 (d,1H); 4.36 (m,2H); 6.95 (s,1H); 7.17 (m,1H); 7.39-7.56 (m,5H). |
| 1.2-211 | [CDCl₃] 2.24 (s,3H); 3.35 (s,3H); 3.39 (d,1H); 3.81 (s,3H); 3.87 (d,1H); 4.26-4.38 (m,2H); 6.83 (s br,1H); 7.15 (m,1H); 7.28 (s,1H); 7.40 (m,3H). |
| 1.2-212 | [CDCl₃] 1.46 (t,3H); 2.26 (s,3H); 3.35 (s,3H); 3.39 (d,1H); 4.09 (q,2H); 4.27-4.39 (m,2H); 6.83 (s br,1H); 7.16 (m,1H); 7.32 (s,1H); 7.41 (m,3H). |
| 1.2-241 | [CDCl₃] 3.40 (s,3H); 3.41 (d,1H); 3.97(d,1H); 4.56-4.68 (m,2H); 6.95(s,1H); 7.16 (m,1H); 7.28 (s br,1H); 7.92 (m,3H); 8.19 (s,1H). |
| 1.2-257 | [CDCl₃] 1.42 (t,3H); 3.36 (s,3H); 3.39 (d,1H); 3.81(d,1H); 3.91 (s,3H); 3.96 (q,2H); 4.20-4.32 (m,2H); 7.03 (s br,1H); 7.15 (m,1H); 7.20 (s,1H); 7.40 (m,3H). |
| 1.2-258 | [CDCl₃] 3.35 (s,3H); 3.39 (d,1H); 3.71 (s,3H); 3.82 (d,1H); 3.90 (s,3H); 4.25 (d,1H); 7.03 (s br,1H); 7.15 (m,1H); 7.17 (s,1H); 7.40 (m,3H). |

**Analytische Daten Tabelle 1.3**

| Nr. | NMR |
|---|---|
| 1.3-1 | [CDCl₃] 3.41 (s,3H); 3.42 (d,1H); 3.88 (d,1H); 5.85 (s,1H); 6.72 (s,1H); 7.38 (t,1H); 7.41 (d,1H); 7.53 (d,1H); 7.69 (s,1H). |
| 1.3-9 | [CDCl₃] 3.39-3.46 (m,4H); 3.85-3.94 (m,2H); 4.06-4.14 (m,1H); 7.07 (s,1H); 7.38 (m,1H); 7.43 (m,1H); 7.56 (m,1H); 7.69 (m,1H). |
| 1.3-16 | [CDCl₃] 3.38 (s,3H); 3.40 (d,1H); 3.89 (d,1H); 3.90-4.03 (m,2H); 5.18-5.27 (m,2H); 5.80-5.91 (m,1H); 6.86 (s,1H); 7.37 (m,1H); 7.42 (m,1H); 7.52 (m,1H); 7.69 (s,1H). |
| 1.3-60 | [CDCl₃] 0.60 (m,2H); 0.85 (m,2H); 2.82 (m,1H); 3.35 (s,3H); 3.39 (d,1H); 3.86 (d,1H); 6.81 (s,1H); 7.37 (t,1H); 7.42 (d,1H); 7.53 (d,1H); 7.69 (s,1H). |
| 1.3-94 | [CDCl₃] 1.28 (m,3H); 2.60 (t,2H); 3.36 (s,3H); 3.39 (d,1H); 3.62 (m,2H); 3.86 (d,1H); 4.18 (q,2H); 7.31 (s,1H); 7.38 (t,1H); 7.41 (d,1H); 7.53 (d,1H); 7.68 (s,1H). |
| 1.3-96 | [CDCl₃] 1.22-1.30 (m,6H); 2.52-2.60 (m,2H); 3.37 (s,3H); 3.39 (d,1H); 3.86 (d,1H); 4.11-4.20 (m,2H); 4.40 (m,1H); 7.25 (s,1H); 7.36 (t,1H); 7.43 (d,1H); 7.52 (d,1H); 7.68 (s,1H). |
| 1.3-102 | [CDCl₃] 1.92 (quint,2H); 2.40 (t,2H); 3.35-3.43 (m,6H); 3.69 (s,3H); 3.88 (d,1H); 6.93 (s,1H); 7.31-7.43 (m,2H); 7.52 (d,1H); 7.68 (s,1H). |
| 1.3-110 | [CDCl₃] 3.36 (s,3H); 3.40 (d,1H); 3.92 (d,1H); 4.52 (m,2H); 7.18 (s,1H); 7.31-7.77 (m,3H); 7.52 (m,1H); 7.67 (m, 2H); 8.35 (s,1H). |
| 1.3-206 | [CDCl₃] 1.48 (t,3H); 3.35 (s,3H); 3.40 (d,1H); 3.88 (d,1H); 4.14 (q,2H), 4.38 (m,2H); 6.95 (s,1H); 7.36-7.46 (m,4H); 7.53 (m,1H); 7.68 (s,1H). |
| 1.3-212 | [CDCl₃] 1.46 (t,3H); 2.25 (s,3H); 3.35 (s,3H); 3.38 (d,1H); 3.88 (d,1H); 4.08 (m,2H); 4.32 (m,2H); 6.81 (s,1H); 7.31 (s,1H); 7.38 (t,1H); 7.41 (t,1H); 7.53 (d,1H); 7.68 (s,1H). |

**Analytische Daten Tabelle 1.5**

| Nr. | NMR |
|---|---|
| 1.5-1 | [CDCl₃] 2.39 (s,3H); 3.41 (s,3H); 3.46 (d,1H); 3,86 (d,1H); 5.73 (s br,1H); 6.72 (s br,1H); 7.27 (d,1H); 7.32 (t,1H); 7.44 (d,1H); 7.51 (s,1H). |
| 1.5-9 | [CDCl₃] 2.39 (s,3H); 3.39 (s,3H); 3.47 (d,1H); 3.88 (d,1H); 3.82-3.97 (m,1H); 4.02-4.17 (m,1H); 7.10 (s br,1H); 7.25 (d,1H); 7.31 (t,1H); 7.44 (d,1H); 7.52 (s,1H). |
| 1.5-16 | [CDCl₃] 2.39 (s,3H); 3.38 (s,3H); 3.45 (d,1H); 3.87 (d,1H); 3.90-4.07 (m,2H); 5.20 (d,1H); 5.24 (d,1H); 5.81-5.92 (m,1H); 6.89 (s br,1H); 7.25 (d,1H); 7.31 (t,1H); 7.45 (d,1H); 7.51 (1H). |
| 1.5-60 | [CDCl₃] 0.54-0.64 (m,2H); 0.79 -0.87 (m,2H); 2.38 (s,3H); 2.77-2.86 (m,1H); 3.35 (s,3H); 3.41 (d,1H); 3.86 (d,1H); 6.83 (s br,1H); 7.25 (d,1H); 7.31 (t,1H); 7.43 (d,1H); 7.50 (s,1 H). |
| 1.5-94 | [CDCl₃] 1.27 (t,3H); 2.38 (s,3H); 2.59 (t,2H); 3.36 (s,3H); 3.41 (d,1H); 3.57-3.66 (m,2H); 3.85 (d,1H); 4.17 (q,2H); 7.21-7.33 (3H); 7.44 (d,1H); 7.51 (s,1H). |
| 1.5-96 | [CDCl₃] D 1: 1.26 (t,3H); 1.30 (d,3H); 2.38 (s,3H); 2.55 (d,2H); 3.36 (s,3H); 3.41 (d,1H); 3.83 (d,1H); 4.15 (q,2H); 4.33-4.46 (m,1H); 7.22 (s br,1H); 7.25 (d,1H); 7.31 (t,1H); 7.45 (d,1H); 7.51 (s,1H). D 2: 1.27 (t,3H); 1.30 (d,3H); 2.38 (s,3H); 2.57 (d,2H); 3.36 (s,3H); 3.41 (d,1H); 3.84 (d,1H); 4.16 (q,2H); 4.33-4.46 (m,1H); 7.22 (s br,1H); 7.25 (d,1H); 7.31 (t,1H); 7.45 (d,1H); 7.51 (s,1H). |
| 1.5-102 | [CDCl₃] 1.91 (pent,2H); 2.38 (q,2H); 2.39 (s,3H); 3.37 (s,3H); 3.30-3.43 (m,2H); 3.42 (d,1H); 3.67 (s,3H); 3.84 (d,1H); 6.94 (s br,1H); 7.25 (d,1H); 7.31 (t,1H); 7.44 (d,1H); 7.51 (s,1H). |
| 1.5-206 | [CDCl₃] 1.48 (t,3H); 2.38 (s,3H); 3.35 (s,3H); 3.43 (d,1H); 3.86 (d,1H); 4.14 (q,2H); 4.31-4.42 (m,2H); 6.97 (s br,1H); 7.25 (d,1H); 7.31 (t,1H); 7.40 s,1H); 7.44 (d,1H); 7.45 (s,1H); 7.50 (s,1H). |
| 1.5-212 | [CDCl₃] 1.26 (t,3H); 2.24 (s,3H); 2.38 (s,3H); 3.35 (s,3H); 3.43 (d,1H); 3.86 (d,1H); 4.06 (q,2H); 4.25-4.38 (m,2H); 6.83 (s br,1H); 7.25 (d,1H); 7.31 (t,1H); 7.44 (d,1H); 7.50 (s,1 H). |

**Analytische Daten Tabelle I.6**

| Nr. | NMR |
|---|---|
| 1.6-1 | [CDCl₃] 1.25 (t,3H); 2.68 (q,2H); 4.41 (s,3H); 3.47 (d,1H); 3.88 (d,1H); 5.66 (s br,1H); 6.72 (s br,1H); 7.29 (d,1H); 7.34 (t,1H); 7.45 (d,1H); 7.54 (s,1H). |
| 1.6-9 | [CDCl₃] 1.25 (t,3H); 2.68 (q,2H); 3.39 (s,3H); 3.47 (d,1H); 3.82-3.97 (m,1H); 3.86 (d,1H); 4.02-4.16 (m,1H); 7.09 (s br,1H); 7.28 (d,1H); 7.34 (t,1H); 7.45 (d,1H); 7.53 (s,1H). |
| 1.6-16 | [CDCl₃] 1.25 (t,,3H); 2.68 (q,1H); 3.39 (s,3H); 3.45 (d,1H); 3.87 (d,1H); 3.90-4.04 (m,2H); 5.18 (d,1H); 5.24 (d,1H); 5.81-5.92 (m,1H); 6.88 (s br,1H); 7.29 (d,1H); 7.33 (t,1H); 7.44 (d,1H); 7.54 (s,1H). |
| 1.6-60 | [CDCl₃] 0.59 (m,2H); 0.84 (m,2H); 1.25 (t,3H); 2.68 (q,2H); 2.81-2.84 (m,1 H); 3.36 (s,3H); 3,43 (d,1H); 3.87 (d,1H); 6.85 (s br,1H); 2.27 (d,1H); 7.33 (t,1H); 7.44 (d,1H); 7.53 (s,1 H). |
| 1.6-94 | [CDCl₃] 1.25 (t,3H); 1.28 (t,3H); 2.59 (m,2H); 2.67 (q,2H); 3.36 (s,3H); 3.42 (d,1H); 3.58-3.66 (m,2H); 3.86 (d,1H); 4.17 (q,2H); 7.27 (d,1H); 7.30 (s br,1H); 7.34 (t,1H); 7.44 (d,1H); 7.54 (s,1H). |
| 1.6-96 | [CDCl₃]1.23-1.33 (m,6H); 2.51-2.63 (m,2H); 2.68 (q,2H); 3.37 (s,3H);3.43 (d,1H); 3.85 (d,1H); 4.12-4.20 (m,2H); 4.36-4.44 (m,1H); 7.25 (t br,1H); 7.27 (d,1H); 7.34 (t,1H); 7.44 (d,1H); 7.54 (s,1H). |
| 1.6-110 | [CDCl₃] 1.25 (t,3H); 2.68 (q,2H); 3.36 (s,3H); 3.44 (d,1H); 3.91 (d,1H); 4.46-4.58 (m,2H); 7.19 (t br,1H); 7.27-7.37 (m,3H); 7.45 (d,1H); 7.53 (s,1H); 7.64 (d,1H); 8.35 (s,1H). |
| 1.6-206 | [CDCl₃] 1.25 (t,3H); 1.48 (t,3H); 2.68 (q,2H); 3.36 (s,3H); 3.44 (d,1H); 4.14 (q,2H); 4.31-4.43 (m,2H); 6.96 (s br,1H); 7.29 (d,1H); 7.33 (t,1H); 7.40 (s,1H); 7.43 (s,1H); 7.44 (s,1H); 7.54 (s,1H). |
| 1.6-212 | [CDCl₃] 1.25 (t,3H); 1.45 (t,3H); 2.26 (s,3H); 2.67 (q,2H); 3.35 (s,3H); 3.44 (d,1 H); 3.87 (d,1H); 4.08 (q,2H); 4.28-4.37 (m,2H); 6.85 (s br,1H); 7.27 (d,1H); 7.33 (s,1H); 7.34 (t,1 H); 7.44 (d,1H); 7.53 (s,1H). |

**Analytische Daten Tabelle 1.7**

| Nr. | NMR |
|---|---|
| 1.7-1 | [CDCl₃] 3.41 (s,3H); 3.46 (d,1H); 3.84 (s,3H); 3.86 (d,1H); 5.79 (s br,1H); 6.72 (s br,1H); 7.00 (d,1H); 7.18 (d,1H); 7.26 (s,1H); 7.34 (t,1H). |
| 1.7-9 | [CDCl₃] 3.39 (s,3H); 3.45 (d,1H); 3.86 (s,3H); 3.87 (d,1H); 3.86-3.95 (m,1H); 4.01-4.18 (m,1H); 7.01 (d,1H);7.09 (s br,1H); 7.18 (d,1H); 7.26 (s,1H); 7.34 (t,1H). |
| 1.7-16 | [CDCl₃] 3.38 (s,3H); 3.43 (d,1H); 3.84 (s,3H); 3.86 (d,1H); 3.80-4.06 (m,2H); 5.19 (d,1H); 5.25 (d,1H); 5.81-5.90 (m,1H); 6.88 (s br,1H); 7.00 (d,1H); 7.17 (d,1H); 7.26 (s,1H); 7.33 (t,1H). |
| 1.7-60 | [CDCl₃] 0.59 (m,2H); 0.84 (m,2H); 2.82 (m,1H); 3.36 (s,3H); 3.41 (d,1H); 3.84 (s,3H); 3.86 (d,1H); 6.85 (s br,1H); 7.00 (d,1H); 7.17 (d,1H); 7.26 (s,1H); 7.33 (t,1H). |
| 1.7-94 | [CDCl₃] 1.28 (t,3H); 2.60 (t,2H); 3.36 (s,3H); 3.40 (d,1H); 3.57-3.68 (m,2H); 3.84 (s,3H); 3.86 (d,1H); 4.16 (q,2H); 6.69 (d,1H); 7.18 (d,1H); 7.30 (s br,1H); 7.31 (t,1H). |
| 1.7-96 | [CDCl₃] D1: 1.24 (t,3H); 1.29 (d,3H); 2.55 (m,2H); 3.37 (s,3H); 3.41 (d,1 H); 3.85 (s,3H); 3.85 (d,1H); 4.10-4.20 (m,2H); 4.35-4.43 (m,1H); 6.99 (d,1H); 7.18 (d,1H); 7.25 (s,1H); 7.26 (s br,1H); 7.33 (t,1H). D2: 1.26 (t,3H); 1.30 (d,3H); 2.58 (m,2H); 3.37 (s,3H); 3.41 (d,1 H); 3.85 (s,3H); 3.85 (d, 1 H); 4.10-4.20 (m,2H); 4.35-4.43 (m,1 H); 6.99 (d, 1 H); 7.18 (d,1H); 7.25 (s,1H); 7.26 (s br,1H); 7.33 (t,1H). |
| 1.7-110 | [CDCl₃] 3.36 (s,3H); 3.43 (d,1H); 3.84 (s,3H); 3.92 (d,1H); 4.47-4.56 (m,2H); 7.00 (d,1H); 7.18 (d,1H); 7.23 (t br,1H); 7.25 (s,1H); 7.33 (t,1H); 7.34 (d,1H); 7.66 (d,1H); 8.35 (s, 1 H). |
| 1.7-206 | [CDCl₃] 1.48 (t,3H); 3.36 (s,3H); 3.43 (d,1H); 3.84 (s,3H); 3.87 (d,1H); 4.15 (q,2H); 4.33-4.42 (m,2H); 6.97 (s br,1H); 7.00 (d,1H); 7.18 (d,1H); 7.26 (s,1H); 7.33 (t,1H); 7.41 (s,1H); 7.46 (s,1H). |
| 1.7-212 | [CDCl₃] 1.46 (t,3H); 2.26 (s,3H); 3.35 (s,3H); 3.42 (d,1H); 3.84 (s,3H); 3.88 (d,1H); 4.08 (q,2H); 4.29-4.37 (m,2H); 6.85 (s br,1H); 7.01 (d,1H); 7.17 (d,1H); 7.26 (s,1H); 7.33 (s,1H); 7.36 (t,1H). |

**Analytische Daten Tabelle 1.11**

| Nr. | NMR |
|---|---|
| 1.11-4 | [CDCl₃] 0.96 (t,3H); 1.54-1.66 (m,2H); 3.22-3.38 (m,2H); 3.34 (d,1H); 3.37 (s,3H); 3.83 (d,1H); 6.78 (s br,1H); 6.90 (t,1H); 7.20 (d,2H). |
| 1.11-7 | [CDCl₃] D1: 0.93 (t,3H); 1.19 (d,3H); 1.52 (m,2H); 3.35 (d,1H); 3.37 (s,3H); 3.81(d,1H); 3.96 (m,1H); 6.55 (d,br,1H); 6.90 (t,1H); 7.19 (d,2H). D2: 0.95 (t,3H); 1.20 (d,3H); 1.52 (m,2H); 3.36 (d,1H); 3.37 (s,3H); 3.83(d,1H); 3.96 (m,1H); 6.55 (d br,1H); 6.90 (t,1H); 7.19 (d,2H). |
| 1.11-8 | [CDCl₃] D1: 0.92 (d,6H); 1.14 (d,3H); 1.75 (sept,1H); 3.34 (d,1H); 3.39 (s,3H); 3.80 (d,1H); 3.86-3.94 (m,1H); 6.60 (d br,1H); 6.90 (t,1H); 7.20 (d,2H). D2: 0.96 (d,6H); 1.15 (d,3H); 1.75 (sept,1H); 3.36 (d,1H); 3.38 (s,3H); 3.83 (d,1H); 3.86-3.94 (m,1H); 6.60 (d br,1H); 6.90 (t,1H); 7.20 (d,2H). |
| 1.11-9 | [CDCl₃] 3.39 (d,1H); 3.39 (s,3H); 3.84 (d,1H); 3.84-3.96 (m,1H); 4.04-4.16 (m,1H); 6.90 (t,1H); 7.1 (t br,1H); 7.20 (d,2H). |
| 1.11-10 | [CDCl₃] 3.36 (d,1H); 3.38 (s,3H); 3.61-3.87 (m,2H); 3.86 (d,1H); 5.91 (tt,1H); 6.92 (t,1H); 7.04 (t br,1H); 7.21 (d,1H). |
| 1.11-11 | [CDCl₃] 3.38 (d,1H); 3.39 (s,3H); 3.84 (d,1H); 3.88-4.03 (m,1H); 4.06-4.22(m,1H); 6.90 (t,1 H); 7.03 (s br,1 H); 7.20 (d,2H). |
| 1.11-15 | [CDCl₃] 2.62-2.80 (m,2H);3.36(d,1H); 3.38 (s,3H); 3.55-3.72 (m,2H); 3.88 (d,1H); 6.90 (t,1H); 7.18 (s br,1H); 7.20 (d,2H). |
| 1.11-16 | [CDCl₃] 3.36 (d,1H); 3.38 (s,3H); 3.87 (d,1H); 3.90-4.06 (m,2H); 5.20 (d,1H); 5.25 (d,1H); 5.81-5.92 (m,1H); 6.86 (s br,1H); 6.90 (t,1H); 7.20 (d,2H). |
| 1.11-19 D1 | [CDCl₃] D1: 1.25 (d,3H); 3.34 (d,1H); 3.40 (s,3H); 3.35-3.44 (m,2H); 3.37 (s,3H); 3.37 (s,3H); 3.82 (d,1H); 4.20 (m,1H); 6.90 (t,1H); 6.94 (d br,1H); 7.20 (d,2H). |
| 1.11-19 D2 | [CDCl₃] D2: 1.24 (d,3H); 3.34 (d,1 H); 3.37 (s,3H); 3,38 (s,3H); 3.41 (m,2H); 3.82 (d,1H); 4.21 (m,1H); 6.90 (t,1H); 6.91(d br,1H); 7.20 (d,2H). |
| 1.11-20 | [CDCl₃] D1: 1.43 (d,3H); 2.55-2.66 (m,1H); 2.73-2.90 (m, 1H); 3.34 (d,1H); 3.37 (s,1H); 3.86 (d,1H); 4.23-4.32 (m,1H); 6.84 (t br,1H); 6.90 (t br,1H); 7.20 (d,2H). D2: 1.44 (d,3H); 2.55-2.66 (m,1H); 2.73-2.90 (m, 1H); 3.34 (d,1H); 3.37 (s,1H); 3.88 (d,1H); 4.23-4.32 (m,1H); 6.84 (t br,1H); 6.90 (t br,1H); 7.20 (d,2H). |
| 1.11-21 | [CDCl₃] 1.10-1.19 (m,7H); 1.20-1.28 (m,1H); 3.30-3.45 (m,8H); 3.74-3.86 (m,1H); 4.00-4.13(m,1H); 6.90 (t,1H); 7.04 (t br,1H); 7.19 (d,2H). |
| 1.11-24 | [CDCl₃] 3.00 (s,3H); 3.23-3.39 (m,2H); 3.32 (d,1H); 3.37 (s,3H); 3.80-3.94 (m,2H); 3.90 (d,1H); 6.91 (t,1H); 7.20 (d,2H); 7.41 (t br,1H). |
| 1.11-25 | [CDCl₃] 1.28 (t,3H); 2.58 (quart,2H); 2.73 (t,2H); 3.35 (d,1H); 3.38 (s,3H); 3.48-3.63 (m,2H); 3.85 (d,1H); 6.91 (t,1H); 7.13 (s br,1H); 7.21 (d,2H). |
| 1.11-40 | [CDCl₃] 3.37 (d,1H); 3.38 (s,3H); 3.92 (d,1H); 4.19-4.39 (m,2H); 6.92 (t,1H); 7.18 (s br,1H); 7.20 (d,2H). |
| 1.11-41 D2 | [CDCl₃] D2: 1.65 (d,3H); 3.37 (d,1H); 3.37 (s,3H); 3.86 (d,1H); 4.90-5.00 (m,1H); 6.92 (t,1 H); 7.07 (s br,1 H); 7.20 (d,2H). |
| 1.11-41 D1 | [CDCl₃] D1: 1.64 (d,3H); 3.34 (d,1H); 3.38 (s,3H); 3.94 (d,1H); 4.88-4.98 (m,1H); 6.92 (t,1H); 7.04 (s br,1H); 7.20 (d,2H). |
| 1.11-42 | [CDCl₃] 1.78 (s,6H); 3.33 (d,1H); 3.37 (s,3H); 3.96 (d,1H); 6.82 (s,1H); 6.92 (t,1H); 7.20 (d,2H). |
| 1.11-45 | [CDCl₃] D1: 1.11 (t,3H); 1.73 (s,3H); 1.90-2.19 (m,2H); 3.32 (d,1H); 3.37 (s,3H); 3.94 (d,1H); 6.28 (d br,1H); 6.90 (t,1H); 7.20 (d,2H). D2: 1.15 (t,3H); 1.74 (s,3H); 1.90-2.19 (m,2H); 3.34 (d,1H); 3.37 (s,3H); 3.97 (d,1H); 6.28 (d br,1H); 6.90 (t,1H); 7.20 (d,2H). |
| 1.11-46 D1 | [CDCl₃] D1: 1.04 (t,3H); 1.50-1.63 (m,2H); 1.75 (s,3H); 1.82-2.06 (m,2H); 3.33 (d,1H); 3.37 (s,3H); 3.97 (d,1H); 6.82 (s br,1H); 6.92 (t,1H); 7.21 (d,2H). |
| 1.11-46 D2 | [CDCl₃] D2: 1.01 (t,3H); 1.48-1.60 (m,2H); 1.74 (s,3H); 1.85-1.88 (m,1 H); 2.01-2.11 (m,1H); 3.33 (d,1H); 3.37 (s,3H); 3.96 (d,1H); 6.79 (s br,1H); 6.92 (t,1H); 7.21 (d,2H). |
| 1.11-47 | [CDCl₃] D1: 0.75 (m,4H);1.31(m,1H); 1.83 (s,3H); 3.32 (d,1H); 3.38 (s,3H); 3.91 (d,1H); 6.92 (t,1H); 7.01 (s br,1H); 7.20 (d,1H). D2: 0.75 (m,4H);1.31(m,1H); 1.82 (s,3H); 3.36 (d,1H); 3.38 (s,3H); 3.99 (d,1H); 6.92 (t,1H); 7.01 (s br,1H); 7.20 (d,1H). |
| 1.11-48 D1 | [CDCl₃] D1: 1.41 (d,3H); 3.37 (d,1H); 3,38 (s,3H); 3.80 (d,1H); 4.65-4.79 (m,1H); 6.83 (d br,1H); 6.91 (t,1H); 7.20 (d,2H). |
| 1.11-48 D2 | [CDCl₃] D2: 1.40 (d,3H); 3.37 (d,1H); 3.38 (s,3H); 3.87 (d,1H); 4.66-4.78 (m,1H); 6.87 (d br,1H); 6.90 (t,1H); 7.20 (d,2H). |
| 1.11-49 D1 | [CDCl₃] D1 1.04 (t,3H); 1.54-1.68 (m,1H); 1.90-2.01(m,1H); 3.39 (s,3H); 3.42 (d,1H); 3.77 (d,1H); 4.46-4.60 (m,1H); 6.7 (d br,1H); 6.91 (t,1H); 7.20 (d,2H). |
| 1.11-49 D2 | [CDCl₃] D2: 1.02 (t,3H); 1.55-1.68 (m,1H); 1.89- 2.00(m,1H); 3.39 (s,3H); 3.37 (d,1H); 3.89 (d,1H); 4.47-4.60 (m,1H); 6.75 (d br,1H); 6.91 (t,1H); 7.20 (d,2H). |
| 1.11-50 | [CDCl₃] D1: 1.02 (m,6H); 2.16-2.28 (m,1H); 3.38 (d,1H); 3.39 (s,3H); 3.78 (d,1H); 4.46-4.59 (m,1H); 6.83 (d br,1H); 6.91 (t,1H); 7.21 (d,2H). D2: 1.05 (m,6H); 2.16-2.28 (m,1H); 3.43 (d,1H); 3.39 (s,3H); 3.89 (d,1H); 4.46-4.59 (m,2H); 6.88 (d br,1H); 6.91 (t,1H); 7.21 (d,2H). |
| 1.11-51 | [CDCl₃] 1.58-1.69 (m,1 H); 1.34-1.98 (m,2H); 2.05-2.15 (m, 1 H); 3.36 (d, 1 H); 3.38 (s,3H); 3.81-3.96 (m, 2H); 3.95 (d,1H); 4.25-4.33 (m,1H); 4.57-4.68 (m,1H); 6.91 (t,1H); 7.21 (d,2H); 7.21 (s br,1H). |
| 1.11-53 | [CDCl₃] D1: 1.47 (d,3H); 2.31 (d,1H); 3.34 (d,1H); 3.37 (s,3H); 3.81 (d,1H); 4.78-4.88 (m,1H); 6.90 (t,1H); 6.95 (s br,1H); 7.20 (d,2H). D2: 1.50 (d,3H); 2.32 (d,1H); 3.36 (d,1 H); 3.37 (s,3H); 3.86 (d, 1 H); 4.78-4.88 (m, 1 H); 6.90 (t,1 H); 6.95 (s br,1 H); 7.20 (d,2H). |
| 1.11-55 | [CDCl₃] D1: 1.41 (d,3H); 1.82 (d,3H); 3.34 (d,1H); 3.38 (s,3H); 3.80 (d,1H); 4.72-4.82 (m,1H); 6.86-6.94 (m,2H); 7.20 (d,2H). D2: 1.44 (d,3H); 1.82 (d,3H); 3.36 (d,1H); 3.39 (s,3H); 3.85 (d,1H); 4.72-4.82 (m,1H); 6.86-6.94 (m,2H); 7.20 (d,2H). |
| 1.11-58 | [CDCl₃] D1: 1.19 (d,3H); 3.34 (d,1H); 3.38(s,3H); 3.43 (s,3H); 3.46 (s,3H); 3.80 (d,1H); 4.16-4.28 (m,2H); 6.85 (s br,1H); 6.90 (t,1H); 7.20 (d,2H). D2: 1.21 (d,3H); 3.35 (d,1H); 3.38(s,3H); 3.44 (s,3H); 3.46 (s,3H); 3.81 (d,1H); 4.16-4.28 (m,2H); 6.85 (s br,1H); 6.90 (t,1H); 7.20 (d,2H). |
| 1.11-59 | [CDCl₃] D1: 1.12 (t,3H); 1.42 (d,3H); 2.44-2.69 (m,2H); 3.33 (d,1H); 3.38 (s,3H); 3.81 (d,1H); 4.59-4.68 (m,1H); 6.90 (t,1H); 7.20 (d,2H); 7.45 (d br,1H). D2: 1.13 (t,3H); 1.41 (d,3H); 2.44-2.69 (m,2H); 3.36 (d,1H); 3.38 (s,3H); 3.84 (d,1H); 4.59-4.68 (m,1H); 6.90 (t,1H); 7.20 (d,2H); 7.45 (d br,1H). |
| 1.11-60 | [CDCl₃] 0.56-0.63 (m,2H); 0.82-0.88 (m,2H); 2.78-2.86 (m,1H); 3.33 (d,1H); 3.37 (s,3H); 3.85 (d,1H); 6.80 (s br,1H); 6.90 (t,1H); 7.20 (d,2H). |
| 1.11-62 | [CDCl₃] D1: 0.22-0.32 (m,2H); 0.43-0.54 (m,2H); 0.83-0.93 (m,1H); 1.27 (d,3H); 3.35 (d,1H); 3.39 (s,3H); 3.38-3.48 (m,1H); 3.81 (d,1H); 6.26 (s br,1H); 6.90 (t,1H); 7.21 (d,2H). D2: 0.32-0.43 (m,2H); 0.54-0.63 (m,2H); 0.83-0.93 (m,1H); 1.28 (d,3H); 3.35 (d,1H); 3.38 (s,3H); 3.38-3.48 (m,1H); 3.82 (d,1H); 6.26 (s br,1H); 6.90 (t,1H); 7.21 (d,2H). |
| 1.11-64 | [CDCl₃] 1.29-1.40 (m,2H); 1.57-1.68 (m,2H); 3.34 (d,1H); 3.35 (s,3H); 3.96 (d,1H); 6.92 (t,1H); 7.20 (d,2H); 7.24 (s br,1H). |
| 1.11-70 | [CDCl₃] 3.19-3.28 (m,2H); 3.32 (d,1H); 3.37 (s,3H); 3.90 (d,1H); 4.10-4.19 (m,2H); 4.40-4.54 (m,1H)6.91 (t,1H); 7.23 (d br,1H); 7.21(d,2H). |
| 1.11-71 | [CDCl₃] 3.32 (d,1H); 3.37 (s,3H); 3.92 (d,1H); 4.02-4.11 (m,2H); 4.54-4.62 (m,2H); 4.71-4.80 (m,1H); 6.90 (t,1H); 7.20 (d,2H); 7.37 (d br,1H). |
| 1.11-73 | [CDCl₃] 1.83 -1.92 (m,1H); 2.26-2.38 (m,1H); 3.33 (dd,1H); 3.36 (s(3H); 3.70-3.76 (m,1H); 3.78-3.91(m,3H); 3.94-4.02(m,1H); 4.51-4.61 (m,1H); 6.91 (t, 1H); 6.94 (s br,1H); 7.21 (d,2H). |
| 1.11-74 | [CDCl₃] 1.60-1.70 (m,1H); 2.02-2.13 (m,1 H); 2.48-2.60 (m,1 H); 3.34 (d, 1 H); 3.38 (s,3H); 3.32-3.44 (m,1H); 3.54-3.58 (m,1H); 3.71-3.95 (m, 3H); 3.85 (d,1H); 6.90 (t,1H); 6.91(s br,1H); 7.20 (d,2H). |
| 1.11-76 | [CDCl₃] D1: 0.92 (t,3H); 1.18 (d,3H); 1.29-1.43 (m,2H); 3.34 (d,1H); 3.37 (s,3H); 3.81 (d,1H); 3.97-4.10 (m,1H); 6.53 (d br,1H); 6.90 (t,1H); 7.21 (d,2H). D2: 0.94 (t,3H); 1.20 (d,3H); 1.43-1.53 (m,2H); 3.35 (d,1H); 3.37 (s,3H); 3.83 (d,1H); 3.97-4.10 (m,1H); 6.53 (d br,1H); 6.90 (t,1H); 7.21 (d,2H). |
| 1.11-93 | [CDCl₃] 2.61 (t,2H); 3.33 (d,1H); 3.36 (s,3H); 3.62 (q,2H); 3.72 (s,3H); 3.84 (d,1H); 6.90 (t,1H); 7.20 (d,2H); 2.29 (s br,1H). |
| 1.11-96 | [CDCl₃] D1: 1.25 (t,3H); 1.30 (d,3H); 2.50-2.62 (m,2H); 3.32 (d,1H); 3.36 (s,3H); 3.83 (d,1H); 4.17 (q,2H); 4.40 (m,1H); 6.91 t,1H); 7.20 (d,2H); 7.25 (s br,1H). D2: 1.26 (t,3H); 1.31 (d,3H); 2.50-2.62 (m,2H); 3.32 (d,1H); 3.36 (s,3H); 3.84 (d,1H); 4.18 (q,2H); 4.40 (m,1H); 6.91 (t,1H); 7.20 (d,2H); 7.25 (s br,1H) |
| 1.11-102 | [CDCl₃] 1.92 (pent,2H); 2.40 (t,2H); 3.34 (d,1H); 3.39 (s,3H); 3.38-3.42 (m,2H); 3.69 (s,3H); 3.85 (d,1H); 6.90 (t,1H); 6.94 (s br,1H); 7.20 (d,2H). |
| 1.11-110 | [CDCl₃] 3.35 (d,1H); 3.36 (s,3H); 3.92 (d,1H); 4.49-4.56 (m,2H); 6.92 (t,1H); 7.19 (d,2H); 7.33 (d,1H); 7.65 (d,1H); 8.35 (s,1H). |
| 1.11-111 | [CDCl₃] 3.34 (d,1H); 3.35 (s,3H); 3.92 (d,1H); 4.45-4.58 (m,2H); 6.88-6.96 (m,2H); 7.18 (s br,1H); 7.20 (d,2H); 7.79 (m,1H); 8.18 (s,1H). |
| 1.11-113 | [CDCl₃] 3.35 (d,1H), 3.35 (s,3H); 3.88 (d,1H); 3.93 (s,3H); 4.40-4.52 (m2H); 6.73 (d,1H); 6.91 (t,1H); 7.04 (t br,1H); 7.20 (d,1H); 7.56 (dd,1H); 8.11 (d,1H). |
| 1.11-119 | [CDCl₃] 3.35 (d,1 H); 3.37 (s,3H); 4.58 - 4.67 (m,2H); 6.92 (t,1 H); 7.20 (d,2H); 7.22 (s br,1H); 7.68 (d,1H); 7.84 (d,1H); 8.69 (s,1H). |
| 1.11-135 | [CDCl₃] 3.37 (d,1H); 3.38 (s,3H); 3.95 (d,1H); 4.49-4.61 (m,2H); 6.92 (t,1H); 7.20 (d,2H); 7.28 (s,2H); 8.59 (s,2H). |
| 1.11-136 | [CDCl₃] 3.37 (d,1H); 3.39 (s,3H); 3.97 (d,1H); 4.51-4.62 (m,2H); 6.87 (s,1H); 6.93 (t,1H); 7.12 (d,1H); 7.22 (d,2H); 7.25 (s br,1H); 8.20 (d,1H). |
| 1.11-137 | [CDCl₃] 3.37 (d,1H); 3.39 (s,3H); 3.97 (d,1H); 4.51-4.62 (m,2H); 6.87 (s,1H); 6.93 (t,1H); 7.12 (d,1H); 7.22 (d,2H); 7.25 (s br,1H); 8.20 (d,1H). |
| 1.11-138 | [CDCl₃] 3.36 (d,1H); 3.39 (s,3H); 3.97 (d,1H); 4.44-4.59 (m,2H);6.92 (t,1H); 7.19 (d,1H); 7.20 (s br,1H), 7.20 (d,2H); 7.42 (s,1H); 8.34 (d,1H). |
| 1.11-142 | [CDCl₃] 3.36 (d,1H); 3.38 (s,3H); 3.92 (d,1H); 3.93 (s,3H); 4.42-4.56 (m,2H); 6.65 (s,1H); 6.79 (d,1H); 6.91 (t,1H); 7.12 (t br,1H); 7.20 (d,2H); 8.13 (d,1H). |
| 1.11-147 | [CDCl₃] 2.56 (s,3H); 3.36 (d,1H); 3.39 (s,3H); 3.95 (d,1H); 4.43-4.58 (m,2H); 6.91 (t,1H); 7.02 (d,1H); 7.08 (s,1H); 7.16 (t br,1H); 7.21 (d,2H); 8.47 (d,1H). |
| 1.11-148 | [CDCl₃] 1.31 (t,3H); 2.83 (q,2H); 3.37 (d,1H); 3.39 (s,3H); 3.94 (d,1H); 4.45-4.58 (m,2H); 6.90 (t,1H); 7.03 (d,1H); 7.08 (s,1H); 7.16 (s br,1H); 7.20 (d,2H); 8.50 (d,1H). |
| 1.11-149 | [CDCl₃] 1.21 (m,2H); 1.42 (m,2H); 2.57 (m,1H); 3.35 (d,1H); 3.39 (s,3H); 4.01 (d,1H); 4.55-4.73 (m,2H); 6.92 (t,1H); 7.15 (s,1H); 7.22 (d,2H); 7.41(d,1H); 7.63 (t br,1H); 8.57 (d,1 H). |
| 1.11-150 | [CDCl₃] 0.97 (t,3H); 1.70-1.82 (m,2H); 2.77 (t,2H); 3.37 (d,1H); 3.39 (s,3H); 3.94 (d,1H); 4.44-4.58 (m,2H); 6.92 (t,1H); 7.04 (d,1H); 7.07 (s,1H); 7.16 (t br,1H); 7.21 (d,2H); 8.50 (d,1 H). |
| 1.11-152 | [CDCl₃] 3.37 (d,1H); 3.39 (s,3H); 3.97 (d,1H); 4.55-4.69 (m,2H); 6.91 (t,1H); 7.21 (d,2H); 7.30 (t br,1H); 7.44 (d,1H); 7.60 (s,1H); 8.71 (d,1H). |
| 1.11-179 | [CDCl₃] 3.41 (d,1H); 3.44 (s,3H); 3.88 (d,1H); 4.74-4.86 (m,2H); 6.91 (t,1H); 7.22 (d,2H); 7.97 (s,1H); 8.20 (s br,1H); 8.76 (s,1H). |
| 1.11-180 | [CDCl₃] 3.26 (t,2H); 3.33 (d,1H); 3.33 (s,3H); 3.80 (d,1H); 3.84-3.92 (m,2H); 6.90 (t,1H); 7.20 (d,2H); 7.52 (s br,1H); 7.91 (s,1H); 8.72 (s,1H). |
| 1.11-189 | [CDCl₃] 2.48 (s,6H); 3.42 (d,1H); 3.46 (s,3H); 3.86 (d,1H); 4.62-4.73 (m,2H); 6.90 (t,1H); 7.21 (d,2H); 8.04 (s br,1H). |
| 1.11-191 | [CDCl₃] 3.40 (d,1H); 3.43 (s,3H); 3.86 (d,1H); 3.95 (s,6H); 4.51-4.15 (m-2H); 6.90 (t,1H); 7.20 (d,2H); 7.95 (s br,1H). |
| 1.11-194 | [CDCl₃] 3.39 (d,1H); 3.42 (s,3H); 3.92 (d,1H); 4.65 (m,2H); 6.90 (t,1H); 7.21 (d,2H); 7.32 (d,1H); 7.84 (br,1H); 8.71 (d,1H); 9.20 (s,1H). |
| 1.11-197 | [CDCl₃] 2.29 (s,3H); 2.38 (s,3H); 3.33 (d,1H); 3.35 (s,3H); 3.83 (d,1H); 4.28 (d,2H); 6.90 (t,1H); 7.19 (s br,1H); 7.20 (d,2H). |
| 1.11-199 | [CDCl₃] 3,34 (d,1H); 3.35 (s,3H); 3.89 (d,1H); 4.34-4.47 (m,2H); 6.91 (t,1H); 7.09 (t br,1H); 7.20 (d,2H); 8.31 (s,1H); 8.45 (s,1H). |
| 1.11-200 | [CDCl₃] 2.46 (s,3H); 3.33 (d,1H); 3.34 (s, 3H); 3.87 (d,1H); 4.24 - 4.37 (m,2H); 6.91 (t,1 H); 6.93 (t br,1 H); 7.20 (d,2H). |
| 1.11-201 | [CDCl₃] 2.26 (s,3H); 2.42 (s,3H); 3.33 (d,1H); 3.34 (s,3H); 3.89 (d,1H); 4.26 (m,2H); 6.8 (s br,1H); 6.91 (t,1H); 7.19 (d,1H). |
| 1.11-202 | [CDCl₃] 1.30 (t,3H); 2.42 (s,3H); 2.65 (q,2H); 3.33 (d,1H); 3.34 (s,3H); 3.90 (d,1H); 4.21-4.33 (m,2H); 6.77 (t br,1H); 6.90 (t,1H); 7.20 (d,2H). |
| 1.11-203 | [CDCl₃] 1.29 (t,3H); 1.30 (t,3H); 2.66 (q,2H); 2.79 (q,2H); 3.33 (d,1H); 3.34 (s,3H); 3.80 (d,1H); 4.22-4.33 (m,2H); 6.26 (s,1H); 6.91 (t,1H); 7.19 (d,2H). |
| 1.11-206 | [CDCl₃] 1.48 (t,3H); 3.34 (d,1H); 3.35 (s,3H); 3.86 (d,1H); 4.15 (q,2H); 4.33-4.43 (m,2H); 6.88 (t,1H); 6.94 (s br,1H); 7.18 (d,2H); 7.41 (s,1H); 7.46 (s,1H). |
| 1.11-207 | [CDCl₃] 3.34 (d,1H); 3.35 (s,3H); 3.87 (d,1H); 4.33-4.46 (m,2H); 4.69 (q,2H); 6.90 (t,1H); 7.02 (t br,1H); 7.20 (d,2H); 7.53 (s,1H); 7.56 (s,1H). |
| 1.11-208 | [CDCl₃] 3.34 (d,1H); 3.35 (s,3H); 3.87 (d,1H); 4.32-4.48 (m,2H); 6.07 (tt,1H); 6.90 (t,1H);6.98 (t br,1H); 7.20 (d,2H); 7.48 (s,1H); 7.54 (s,1H). |
| 1.11-210 | [CDCl₃] 0.98-1.08 (m,2H); 1.10-1.17 (m,2H); 3.34 (d,1H); 3.35 (s,3H); 3.52-3.59 (m,1H); 3.86 (d,1H); 4.30-4.41 (m,1H); 6.91 (t,1H); 6.93 (s br,1H); 7.20 (d,2H); 7.43 (s,1H); 7.46 (s,1H). |
| 1.11-211 | [CDCl₃] 2.24 (s,3H); 3.33 (d,1H); 3.34 (s,3H); 3.81 (s,3H); 3.87 (d,1H); 4.26-4.38 (m,2H); 6.82 (s br,1H); 6.90 (t,1H); 7.20 (d,2H); 7.28 (s,1H). |
| 1.11-212 | [CDCl₃] 1.45 (t,3H); 2.24 (s,3H); 3.34 (d,1H); 3.35 (s,3H); 3.86 (d,1H); 4.08 (q,2H); 4.27-4.39 (m,2H); 6.80 (s br,1H); 6.91 (t,1H); 7.21 (d,2H); 7.33 (s,1H). |
| 1.11-213 | [CDCl₃] 1.47 (t,3H); 3.33 (d,1H); 3.35 (s,3H); 3.84 (d,1H); 4.08 (q, 2H); 4.33 (d,2H); 6.90 (t,1H); 7.03 (s br,1H); 7.20 (d,1H); 7.72 (s,1H). |
| 1.11-238 | [CDCl₃] 3.34 (d,1H); 3.36 (s,3H); 3.80 (d,1H); 4.60 (d,2H); 6.91 (t,1H); 7.20 (d,2H); 7.26 (d,1H); 7.33 (t br,1H); 7.76 (d,1H); 8.35 (d,1H). |
| 1.11-239 | [CDCl₃] 3.37 (d,1H); 3.40 (s,3H); 3.95 (d,1H); 4.62 (d,2H); 6.91 (t,1H); 7.20 (d,2H); 7,28 (s , 1H); 7.30 (d,1H); 8.48 (d,1H); 8.58 (s,1H). |
| 1.11-240 | [CDCl₃] 2.22 (s,3H); 2.36 (s,3H); 3.40 (d,1H); 3.44 (s,3H); 6.93 (t,1H); 7.21 (d,2H), 7.76 (s br,1 H). |
| 1.11-241 | [CDCl₃] 3.36 (d,1H); 3.39 (s,3H); 3.97(d,1H); 4.56-4.69 (m,2H); 6.91(t,1H); 6.93 (m,1H); 7.21 (d,2H); 7.28 (s br,1H); 8.19 (s,1H). |
| 1.11-242 | [CDCl₃] 3.36 (d,1H); 3.39 (s,3H); 3.95 (d,1H); 4.52-4.66 (m,2H); 6.90 (t,1H); 7.20 (d,2H); 7.23 (s br,1H); 7.32 (s,1H); 8.36 (s,1H). |
| 1.11-243 | [CDCl₃] 3.36 (d,1H); 3.40 (s,3H); 3.96 (d,1H); 4.51- 4.64 (m,2H); 6.91 (t,1H); 7.22 (d,2H), 7.31 (s,1H); 8.49 (s,1H). |
| 1.11-244 | [CDCl₃] 3.35 (d,1H); 3,39 (s,3H); 3.91 (s,3H); 3.92 (d,1H); 4.45-4.60 (m,2H); 6.73 (s,1H); 6.91 (t,1H); 7.20 (s br,1H); 7.21 (d,2H); 8.23 (s,1H). |
| 1.11-245 | [CDCl₃] 2.29 (s,3H); 3.37 (d,1H); 3.40 (s,3H); 3.97 (d,1H); 4.42-4.57 (m,2H); 6.92 (t,1H); 7.10 (s br,1H); 7.18 (s,1H); 7.21 (d,2H); 8.18 (s,1H). |
| 1.11-246 | [CDCl₃] 2.55 (s,3H); 3.34 (d,1H); 3.36 (s,3H); 3.94 (d,1H); 4.50 (d,2H); 6.91 (t,1H); 7.03 (t br,1H); 7.16 (d,1H); 7.20 (d, 2H); 7.53 (d,1H). |
| 1.11-247 | [CDCl₃] 2.58 (s,3H); 3.36 (d,1H); 3.37 (s,3H); 3.94 (d,1H); 4.48-4.60 (m,2H); 6.90 (t,1H); 7.02 (t br,1H); 7.15 (dd,1H); 7.21 (d,1H); 7.56 (d,1H); 8.45 (d,1H). |
| 1.11-248 | [CDCl₃] 3.34 (d,1H); 3.35 (s,3H); 3.90 (d,1H); 4.43-4.55 (m,2H); 6.90 (t,1H); 7.19 (s br,1H); 7.20 (d,2H); 7.48 (d,1H); 7.54 (d,1H); 8.33 (s,1H). |
| 1.11-249 | [CDCl₃] 2.21 (s,3H); 3.36 (d,1H); 3.39 (s,3H); 3.91 (s,3H); 3.94 (d,1H); 4.47 (qd,2H); 6.62 (s,1H); 6.91 (t,1H); 7.03 (t br,1H); 7.21 (d,2H); 7.94 (s,1H). |
| 1.11-250 | [CDCl₃] 2.28 (s,3H); 2.53 (s,3H); 3.36 (d,1H); 3.39 (s,3H); 3.96 (d,1H); 4.43-4.54 (m,2H); 6.90 (t,1H); 7.02 (s br,1H), 7.02 (s,1H); 7.21 (d,2H); 8.30 (s,1H). |
| 1.11-251 | [CDCl₃] 2.56 (s,3H); 3.35 (d,1H); 3.36 (s,3H); 3.88 (d,1H); 4.44-4.57 (m,2H); 6.90 (t,1H); 7.08 (s br,1H); 7.15 (d,1H); 7.20 (d,2H); 7.56 (d,1H); 8.45 (s,1H). |
| 1.11-252 | [CDCl₃] 3.34 (d,1H); 3.34 (s,3H); 3.80 (d,1H); 3.91 (s,3H); 3.98 (s,3H); 4.32-4.47 (m,2H); 6.27 (d,1H); 6.90 (t,1H); 7.17 (s br,1H); 7.19 (d,2H); 7.49 (d,1H). |
| 1.11-253 | [CDCl₃] 2.32 (s,3H); 3.33 (d,1H); 3.35 (s,3H); 3.91 (d,1H); 4.36 (d,2H); 6.91 (t,1H); 6.95 (t br,1H); 7.20 (d,2H); 8.34 (s,1H). |
| 1.11-254 | [CDCl₃] 3,34 (s,3H); 3,35 (s,3H); 3.35 (d,1H); 3.74 (t,2H); 3.85 (d,1H); 4.26 (t,2H); 4.32-4.46 (m,2H); 6.91 (t,1H); 6.93 (s br,1H); 7.20 (d,2H); 7.47 (s,2H). |
| 1.11-255 | [CDCl₃] 1.47 (t,3H); 3.34 (d,1H); 3.35 (s,3H); 3.84 (d,1H); 4.11 (q, 2H); 4.32 (d,2H); 6.90 (t,1H); 7.03 (t br,1H); 7.20 (d,2H); 7.40 (s,1H). |
| 1.11-256 | [CDCl₃] 1.44 (t,3H); 3.33 (d,1H); 3.35 (s,3H); 3.83 (d,1H); 4.00 (q,2H); 4.30 (d,2H); 6.90 (t,1 H); 6.90 (t,1 H); 7.03 (t br,1H); 7.19 (d,2H); 7.32 (s,1H). |
| 1.11-257 | [CDCl₃] 1.42 (t,3H); 3.34 (d,1H); 3.35 (s,3H); 3.80 (d,1H); 3.91 (s,3H); 3.96 (q,2H); 4.21-4.32 (m,2H); 6.90 (t,1H); 7.02 (s br,1H); 7.19 (d,2H); 7.20 (s,1H). |
| 1.11-258 | [CDCl₃] 3.33 (d,1H); 3.35 (s,3H); 3.78 (s,3H); 3.86 (d,1H); 3.90 (s,3H); 4.20-4.30 (m,2H); 6.89 (t,1H); 7.02 (s br,1H); 7.16 (s,1H); 7.19 (d,2H); |
| 1.11-259 | [CDCl₃] 1.25 (t,3H); 2.58 (q,2H); 3.36 (d,1H); 3.37 (s,3H); 3.74 (s,3H); 3.83 (d,1H); 4.40-4.52 (m,2H); 5.98 (s,1H); 6.90 (t,1H); 7.18 (s br,1H); 7.20 (d,2H). |

**Analytische Daten Tabelle 1.14**

| Nr. | NMR |
|---|---|
| 1.14-1 | [CDCl₃] 2.39 (s,3H); 3.40 (s,3H); 3.43 (d,1H); 3.85 (d,1H); 5.90 (s br,1H); 6.72 (s br,1H); 6.98 (d,1H); 7.20 (d,1H); 7.24 (s,1H). |
| 1.14-9 | 2.39 (s,3H); 3.38 (s,3H); 3.42 (d,1H); 3.86 (d,1H); 3.85-3.96 (m,1H); 4.03-4.17 (m,1H); 6.98 (d,1H); 7.08 (t br,1H); 7.20 (d,1H); 7.25 (s,1H). |
| 1.14-16 | [CDCl₃] 2.38 (s,3H); 3.38 (s,3H); 3.40 (d,1H); 3.86 (d,1H); 3.90-4.05 (m,2H); 5.19 (d,1H); 5.24 (d,1H); 5.81-5.92 (m,1H); 6.86 (s br,1H); 6.97 (d,1H); 7.20 (d,1H); 7.24 (s,1H). |
| 1.14-60 | [CDCl₃] 0.60 (m,2H); 0.84 (m,2H); 2.38 (s,3H); 2.82 (s,1H); 3.35 (s,3H); 3.37 (d,1H); 3.84 (d,1H); 6.83 (s br,1H); 6.97 (d,1H); 7.19 (d,1H); 7.23 (s,1H). |
| 1.14-94 | [CDCl₃] 1.27 (t,3H); 2.38 (s,3H); 2.59 m,2H); 3.36 (s,3H); 3.37 (d,1H); 3.57-3.66 (m,2H); 3.84 (d,1H); 4.17 (q,2H); 6.97 (d,1H); 7.19 (d,1H); 7.24 (s,1H); 7.32 (s br,1H). |
| 1.14-96 | [CDCl₃] 1.12-1.34 (m,6H); 2.38 (s,3H); 2.51-2.62 (m,2H); 3.37 (s,3H); 3.37 (d,1H); 3.83 (d,1H); 4.10-4.22 (m,2H); 4.36-4.44 (m,1H); 6.96 (d,1H); 7.20 (d,1H); 7.24 (s,1H); 7.26 (s br,1 H). |
| 1.14-102 | [CDCl₃] 1.91 (quin,2H); 2.38 (m,5H); 3.38 (s,3H); 3.38 (d,1H); 3.33-3.45 (m,2H); 3.69 (s,3H); 3.85 (d,1H); 6.92 (t br,1H); 6.97 (d,1H); 7.20 (d,1H); 7.25 (s,1H). |
| 1.14-110 | [CDCl₃] 2.39 (s,3H); 3.35 (s,3H); 3.38 (d,1H); 4.49-4.56 (m,2H); 6.98 (d,1H); 7.20 (d,1H); 7.21 (s br,1H); 7.24 (s,1H); 7.32 (d,1H); 7.65 (d,1H); 8.35 (s,1H). |
| 1.14-136 | [CDCl₃] 2.39 (s,3H); 3.39 (s,3H); 3.41 (d,1H); 3.97 (d,1H); 4.48-4.59 (m,2H); 6.98 (d,1H); 7.16 (d,1H); 7.21 (d,1H); 7.25 (s,2H); 7.26 (s br,1H); 8.36 (d,1H). |
| 1.14-206 | [CDCl₃] 1.48 (t,3H); 2.38 (s,3H); 3.34 (s,3H); 3.37 (d,1H); 3.85 (d,1H); 4.14 (q,2H); 4.31-4.43 (m,2H); 6.94 (s br,1H); 6.97 (d,1H); 7.18 (d,1H); 7.24 (s,1H); 7.40 (s,1H); 7.45 (s, 1 H). |
| 1.14-212 | [CDCl₃] 1.43 (t,3H); 2.25 (s,3H); 2.38 (s,3H); 3.35 (s,3H); 3.39 (d,1H); 3.86 (d,1H); 4.10 (q,2H); 4.29-4.38(m,2H); 6.83 (s br,1H); 6.97 (d,1H); 7.19 (d,1H); 7.24 (s,1H); 7.41 (s,1H). |

**Analytische Daten Tabelle 1.16**

| Nr. | NMR |
|---|---|
| 1.16-1 | [CDCl₃] 3.39 (d,1H); 3.41 (s,3H); 3.83 (s,3H); 3.84 (d,1H); 5.68 (s br,1H); 6.70 (s br 1H); 6.71 (d,1H); 6.96 (d,1H); 7.01 (s,1H). |
| 1.16-60 | [CDCl₃] 0.60 (m,2H); 0.84 (m,2H); 2.81-2.84 (m,1H); 3.35 (s,3H); 3.36 (d,1H); 3.83 (s,3H); 3.85 (d,1H); 6.69 (d,1H); 6.82 (s br,1H); 6.94 (d,1H); 7.00 s,1H). |
| 1.16-94 | [CDCl₃] 1.28 (t,3H); 2.59 (m,2H); 3.35 (d,1H); 3.36 (s,3H); 3.58-3.67 (m,2H); 3.83 (s,3H); 3.84 (d,1H); 4.17 (q,2H); 6-70 (d,1H); 6.97 (d,1H); 7.01 (s,1H); 7.31 (t br,1H). |
| 1.16-96 | [CDCl₃] 1.24-1.32 (m,6H); 2.52-2.62 (m,2H); 3.35 (d,1H); 3.37 (s,3H); 3.82 (d,1H);3.84 (s,3H); 4.11-4.21 (m,2H); 4.36-4.43 (m,1H);6.69 (d,1H); 6.95 (d,1H); 7.01 (s,1H). |
| 1.16-110 | [CDCl₃] 3.35 (s,3H); 3.37 (d,1H); 3.83 (s,3H); 3.89 (d,1H); 4.47-4.56 (m,2H); 6.70 (d,1H); 6.95 (d,1H); 7.01 (s,1H); 7.21 (t br,1H); 7.32 (d,1H); 7.65 (d,1H); 8.35 (s,1H). |
| 1.16-136 | [CDCl₃] 3.39 (d,1H); 3.39 (s,3H); 3.84 (s,3H); 3.96 (d,1H); 4.48-4.59 (m,2H); 6.71 (d,1H); 6.97 (d,1H); 7.02 (s,1H); 7.16 (d,1H); 7.27 (s br,2H); 8.36 (d,1H). |
| 1.16-212 | [CDCl₃] 1.45 (t,3H); 2.26 (s,3H); 3.35 (s,3H); 3.37 (d,1H); 3.84 (s,3H); 3.86 (d,1H); 4.10 (q,2H); 4.30-4.36 (m,2H); 6.71 (d,1H); 6.82 (t br,1H); 6.94 (d,1H); 7.00 (s,1H); 7.32 (s,1H). |

**Analytische Daten Tabelle 1.18**

| Nr. | NMR |
|---|---|
| 1.18-1 | [CDCl₃] 3.42 (s,3H); 3.43 (d,1H); 3.92 (d,1H); 5.86 (s br,1H); 6.70 (s br,1H); 7.43 (d,1H); 7.61 (d,1H); 7.70 (s,1H). |
| 1.18-9 | [CDCl₃] 3.40 (s,3H); 3.43 (d,1H); 3.83-3.98 (m,1H); 3.82 (d,1H); 4.03-4.19 (m,1H); 7.08 (s br,1H); 7.43 (d,1H); 7.60 (d,1H); 7.69 (s,1H). |
| 1.18-16 | [CDCl₃] 3.39 (s,3H); 3.40 (d,1H); 3.93 (d,1H); 3.92-4.06 (m,2H); 5.19 (d,1H); 5.25 (d,1H); 5.82-5.93 (m,1H); 6.87 (s br,1H); 7.42 (d,1H); 7.60 (d,1H); 7.70 (s,1H). |
| 1.18-60 | [CDCl₃] 0.60 (m,2H); 0.85 (m,2H); 2.80-2.84 (m,1H); 3.36 (s,3H); 3.38 (d,1H); 3.92 (d,1H); 6.82 (s br,1H); 7.41 (d,1H); 7.59 (d,1H); 7.69 (s,1H). |
| 1.18-94 | [CDCl₃] 1.28 (t,3H); 2.60 (m,2H); 3.37 (s,3H); 3.37 (d,1H); 3.63 (q,2H); 3.90 (d,1H); 4.18 (q,2H); 7.30 (t br,1H); 7.42 (d,1H); 7.60 (d,1H); 7.69 (s,1H). |
| 1.18-96 | [CDCl₃] 1.25-1.32 (m,6H); 2.50-2.51 (m,2H); 3.37 (d,1H); 3.38 (s,3H); 3.89 (d,1H); 4.13-4.21 (m,2H); 4.31-4.46 (m,1H); 7.27 (s br,1H); 7.41 (d,1H); 7.60 (d,1H); 7.70 (s,1H). |
| 1.18-102 | [CDCl₃] 1.92 (quin, 2H); 2.41 (t,2H); 3.38 (s,3H); 3.36-3.43 (m,3H); 3.91 (d,1H); 6.96 (s br,1H); 7.41 (d,1H); 7.60 (d,1H); 7.70 (s,1H). |
| 1.18-110 | [CDCl₃] 3.37 (s,3H); 3.40 (d,1H); 3.98 (d,1H); 4.48-4.56 (m,2H); 7.21 (t br,1H); 7.33 (d,1H); 7.42 (d,1H); 7.60 (d,1H); 7.65 (d,1H); 7.69 (s,1H); 8.35 (s,1H). |
| 1.18-136 | [CDCl₃] 3.41 (s,3H) 3.42 (d,1H); 4.04 (d,1H); 4.49-4.59 (m,2H); 7.16 (d,1H); 7.43 (d,1H); 7.62 (d,1H); 7.71 (s,1H); 8.36 (d,1H). |
| 1.18-206 | [CDCl₃] 1.48 (t,3H);3.36 (s,3H); 3.38 (d,1H); 3.92 (d,1H); 4.15 (q,2H); 4.31-4.43 (m,2H); 6.95 (s br,1H); 7.40 (s,1H); 7.41 (d,1H); 7.46 (s,1H); 7.58 (d,1H); 7.69 (s,1H). |
| 1.18-212 | [CDCl₃] 1.46 (t,3H); 2.25 (s,3H); 3.36 (s,3H); 3.38 (d,1H); 3.93 (d,1H); 4.10 (q,2H); 4.28-4.39 (m,2H); 6.81 (s br,1H); 7.32 (s,1H); 7.42 (d,1H); 7.60 (d,1H); 7.69 (s,1H). |

**Analytische Daten Tabelle 1.20**

| Nr. | NMR |
|---|---|
| 1.20-1 | [CDCl₃] 3.38 (d,1H); 3.39 (s,3H); 3.86 (d,1H); 5.85 (s br,1H); 6.70 (s br,1H); 7.44 (s,1H); 7.56 (s,2H). |
| 1.20-4 | [CDCl₃] 0.96 (t,3H); 1.59 (m,2H); 3.27-3.33 (m,3H); 3.37 (s,3H); 3.85 (d,1H); 6.78 (s,1H); 7.43 (s,1H); 7.55 (s,2H). |
| 1.20-5 | [CDCl₃] 1.21 (d,3H); 1.23 (d,3H); 3.33 (d,1H); 3.36 (s,3H); 3.84 (d,1H); 4.04-4.18 (m,1H); 6.58 (d br,1H); 7.43 (s,1H); 7.55 (s,2H). |
| 1.20-7 | [CDCl₃] D1: 0.93 (t,3H); 1.18 (d,3H); 1.48-1.58 (m,2H); 3.34 (d,1H); 3.36 (s,3H); 3.83 (d,1 H); 3.90-4.02 (m, 1 H); 6.55 (d br,1 H); 7.43 (s,1 H); 7.55 (s,2H). D2: 0.96 (t,3H); 1.20 (d,3H); 1.48 -1.58 (m,2H); 3.35 (d,1H); 3.36 (s,3H); 3.86 (d,1H); 3.90-4.02 (m,1H); 6.55 (d br,1H); 7.43 (s,1H); 7.55 (s,2H). |
| 1.20-8 | [CDCl₃] D1: 0.93 (d,6H); 1.13 (d,3H); 1.69-1.81 (m,1H); 3.33 (d,1H); 3.37 (s,3H); 3.82 (d,1H); 3.83-3.96 (m,1H); 6.58 (d br,1H); 7.44 (s,1H); 7.55 (s,2H). D2: 0.95 (d,6H); 1.15 (d,3H); 1.69-1.81 (m,1H); 3.34 (d,1H); 3.38 (s,3H); 3.85 (d,1H); 3.83-3.96 (m,1H); 6.58 (d br,1H); 7.44 (s,1H); 7.55 (s,2H). |
| 1.20-9 | [CDCl₃] 3.36 (d,1H); 3.38 (s,3H); 3.86 (d,1H); 3.84-3.97 (m,1H); 4.02-4.17 (m,1H); 7.05 (s br,1H); 7.45 (s,1H); 7.55 (s,2H). |
| 1.20-10 | [CDCl₃] 3.33 (d,1H), 3.38 (s,3H); 3.66-3.80 (m,2H); 3.89 (d,1H); 5.78-6.05 (tt,1H); 7.02 (s,1H); 7.45 (s,1H); 7.56 (s,2H). |
| 1.20-11 | [CDCl₃] 3.38 (s,3H); 3.40 (d,1H); 3.84 (d,1H); 3.91-4.01 (m,1H); 4.08-4.20 (m,1H); 7.03 (s,1H); 7.45 (s,1H); 7.56 (s,2H). |
| 1.20-15 | [CDCl₃] 2.67-2.73 (m,2H); 3.32 (d,1H); 3.37 (s,3H); 3.59-3.68 (m,2H); 3.89 (d,1H); 7.20 (s,1H); 7.45 (s,1H); 7.55 (s,2H). |
| 1.20-16 | [CDCl₃] 3.34 (d,1H); 3.38 (s,3H); 3.87 (d,1H); 3.90-4.06 (m,2H); 5.20 (d,1H); 5.25 (d,1H); 5.80-5.93 (m,1H); 6.85 (s br,1H); 7.44 (s,1H); 7.55 (s,2H). |
| 1.20-19 D1 | [CDCl₃] D1: 1.26 (d,3H); 3.34 (d,1H); 3.37 (s,3H); 3.37-3.45 (m,2H); 3.83 (d,1H); 4.13-4.26 (m,1H); 6.93 (d br,1H); 7.43 (s,1H); 7.55 (s,2H). |
| 1.20-19 D2 | [CDCl₃] D2: 1.23 (d,3H); 3.34 (d,1H); 3.40 (s,3H); 3.37-3.45 (m,2H); 3.83 (d,1H); 4.14-4.27 (m,1H); 6.91 (d br,1H); 7.43 (s,1H); 7.55 (s,2H). |
| 1.20-20 | [CDCl₃] 1.43 (t,3H); 2.57-2.66 (m,1H); 2.75-2.89 (m,1H); 3.31 (d,1H); 3.37 (s,3H); 3.84-3.91 (m,1H); 4.29 (m,1H); 6.83 (s,1H); 7.44 (s,1H); 7.55 (s,2H). |
| 1.20-21 | [CDCl₃] 1.11-1.20 (m,6H); 3.30-3.42 (m,8H); 3.77-3.85 (m,1H); 4.02 (m,1H); 7.02 (m,1H); 7.42 (m,1H); 7.55 (m,2H). |
| 1.20-24 | [CDCl₃] 3.00 (s,3H); 3.29-3.36 (m,3H); 3.38 (s,3H); 3.86-3.93 (m,3H); 7.43 (m,2H); 7.55 (s,2H). |
| 1.20-25 | [CDCl₃] 1.28 (t,3H); 2.58 (q,2H); 2.73 (t,2H); 3.35 (d,1H); 3.38 (s,3H); 3.48-3.61 (m,2H); 3.85 (d,1H); 7.12 (s br,1H); 7.43 (s,1H); 7.55 (s,2H). |
| 1.20-40 | [CDCl₃] 3.36 (d,1H); 3.37 (s,3H); 3.92 (d,1H); 4.17-4.89(m,2H); 7.18 (t br,1H); 7.45 (s,1H); 7.55 (s,2H). |
| 1.20-41 D1 | [CDCl₃] D1: 1.65 (d,3H); 3.37 (s,3H); 3.37 (d,1H); 3.86 (d,1H); 4.90-4.99 (m,1H); 7.05 (s br,1H); 7.45 (s,1H); 7.55 (s,2H). |
| 1.20-41 D2 | [CDCl₃] D2: 1.64 (d,3H); 3.36 (s,3H); 3.34 (d,1H); 3.95 (d,1H); 4.89-4.98 (m,1H); 7.05 (s br,1H); 7.45 (s,1H); 7.55 (s,2H). |
| 1.20-42 | [CDCl₃] 1.78 (s,6H); 3.35 (d,1H); 3.38 (s,3H); 3.99 (d,1H); 6.80 (s,1H); 7.45 (s,1H); 7.55 (s,2H). |
| 1.20-45 | [CDCl₃] 1.09-1.18 (m,3H); 1.73 (d, 3H); 1.98-2.13 (m,2H); 3.30 (d,1H); 3.37 (s,3H); 3.92-4.00 (m,1H); 6.79 (d,1H); 7.45 (s,1H); 7.56 (s,2H). |
| 1.20-46 D1 | [CDCl₃] D1 1.04 (t,3H); 1.59 (m,2H); 1.75 (s,3H); 1.89 (m,1H); 2.00 (m,1H); 3.34 (d,1H); 3.36 (s,3H); 3.98 (d,1H); 6.80 (s,1H); 7.45 (s,1H); 7.56 (s,2H). |
| 1.20-46 D2 | [CDCl₃] D2 1.01 (t,3H); 1.52 (m,2H); 1.74 (s,3H); 1.92 (m,1H); 2.05 (m,1H); 3.35 (d,1H); 3.36 (s,3H); 3.97 (d,1H); 6.78 (s,1H); 7.45 (s,1H); 7.56 (s,2H). |
| 1.20-47 | [CDCl₃] 0.70-0.80 (m,4H); 1.26-1.33 (m,1H); 1.82 (d,3H); 3.30-3.34 (m,1H); 3.38 (s,3H); 3.90-4.02 (m,1H); 7.00 (m,1H); 7.45 (s,1H); 7.56 (s,2H). |
| 1.20-48 D1 | [CDCl₃] D1 1.40 (d,3H); 3.38 (s,3H), 3.39 (d,1H), 3.88 (d,1H); 4.73 (m,1H); 6.85 (d,1H); 7.45 (s,1H); 7.56 (s,2H). |
| 1.20-48 D2 | [CDCl₃] D2 1.40 (d,3H); 3.38 (s,3H), 3.39 (d,1H), 3.80 (d,1H); 4.72 (m,1H); 6.80 (d,1H); 7.45 (s,1H); 7.56 (s,2H). |
| 1.20-49 D1 | [CDCl₃] D1 1.03 (t,3H); 1.58-1.64 (m,1H); 1.90-2.00 (m,1H); 3.38 (s,3H); 3.42 (d,1H); 3.80 (d,1H); 4.55 (m,1H); 6.70 (d,1H); 7.45 (s,1H); 7.55 (s,2H). |
| 1.20-49 D2 | [CDCl₃] D2 1.02 (t,3H); 1.58-1.64 (m,1H); 1.90-2.00 (m,1H); 3.38 (s,3H); 3.40 (d,1H); 3.90 (d,1H); 4.55 (m,1H); 6.75 (d,1H); 7.45 (s,1H); 7.56 (s,2H). |
| 1.20-50 | [CDCl₃] D1: 0.98-1.07 (m,6H); 2.16-2.28 (m,1H); 3.39 (s,3H); 3.36 (d,1H); 3.78 (d,1H); 3.46-4.58 (m,1H); 6.84 (d br,1H); 7.44 (s,1H); 7.56 (s,2H). D2: 0.98-1.07 (m,6H); 2.16-2.28 (m,1H); 3.40 (s,3H); 3.42 (d,1H); 3.90 (d,1H); 3.46-4.58 (m,1H); 6.88 (d br,1H); 7.44 (s,1H); 7.56 (s,2H). |
| 1.20-51 D1 | [CDCl₃] D1 1.80 (m,1H); 1.95 (m,2H); 2.09 (m,1H); 3.38 (s,3H), 3.39 (d,1H); 3.80-3.91 (m,3H); 4.18 (q,1H), 4.72 (m,1H); 7.00 (m,1H); 7.45 (s,1H); 7.56 (s,2H). |
| 1.20-51 D2 | [CDCl₃] D2 1.60 (m,1H); 1.93 (m,2H); 2.14 (m,1H); 3.38 (s,3H), 3.42 (d,1H); 3.80 (d,1H); 3.83-3.97 (m,2H); 4.30 (m,1H), 4.65 (m,1H); 7.20 (d,1H); 7.45 (s,1H); 7.56 (s,2H). |
| 1.20-51 D3 | [CDCl₃] D3 1.62 (m,1H); 1.91 (m,2H); 2.10 (m,1H); 3.37 (s,3H), 3.38 (d,1H); 3.84-3.97 (m,3H); 4.30 (m,1H), 4.64 (m,1H); 7.21 (s,1H); 7.45 (s,1H); 7.56 (s,2H). |
| 1.20-51 D4 | [CDCl₃] D4 1.80 (m,1H); 1.93 (m,2H); 2.08 (m,1H); 3.38 (s,3H), 3.39 (d,1H); 3.81-3.91 (m,2H); 4.18 (q,1H), 4.73 (m,1H); 7.00 (m,1H); 7.45 (s,1H); 7.56 (s,2H) |
| 1.20-53 | [CDCl₃] 1.49 (t,3H); 2.30 (m,1H); 3.33 (m,1H); 3.37 (s,3H); 3.80-3.90 (m,1H); 4.83 (m,1H); 6.93 (s,1H); 7.44 (s,1H); 7.55 (s,2H). |
| 1.20-55 | [CDCl₃] D1: 1.42 (d,3H); 1.81 (s,3H); 3.33 (d,1H); 3.38 (s,3H); 3.80 (d,1H); 4.71-4.81 (m,1H); 6.88 (d br,1H); 7.43 (s,1H); 7.55 (s,2H). D2: 1.45 (d,3H); 1.82 (s,3H); 3.35 (d,1H); 3.38 (s,3H); 3.85 (d,1H); 4.71-4.81 (m,1H); 6.88 (d br,1H); 7.43 (s,1H); 7.55 (s,2H). |
| 1.20-58 | [CDCl₃] D1: 1.18 (d,3H); 3.32 (d,1H); 3.37 (s,3H); 3.43 (s,3H); 3.46 (s,3H); 3.81 (d,1H); 4.16-4.28 (m,2H); 6.87 (t br,1H);7.43 (s,1H); 7.55 (s,2H). D2: 1.20 (d,3H); 3.34 (d,1H); 3.37 (s,3H); 3.43 (s,3H); 3.44 (s,3H); 3.82 (d,1H); 4.16-4.28 (m,2H); 6.87 (t br,1H);7.43 (s,1H); 7.55 (s,2H). |
| 1.20-59 | [CDCl₃] 1.11 (m,3H); 1.42 (m,3H); 2.48-2.63 (m,2H); 3.32 (m,1H); 3.38 (s,3H); 3.79-3.88 (m,1H); 4.62 (m,1H); 7.43-7.50 (m,2H); 7.55 (m,2H). |
| 1.20-60 | [CDCl₃] 0.59 (m,2H); 0.84 (m,2H); 2.82 (m,1H); 3.34 (s,3H); 3.34 (d,1H); 3.88 (d,1H); 6.79 (s,1H); 7.43 (m,1H); 7.54 (m,2H). |
| 1.20-60 E1 | [CDCl₃] 0,56-0.62 (m,2H); 0.82-0.89 (m,2H); 2.77-2.86 (m-1H); 3.33 (d,1H); 3.86 (d,1H); 6.78 (s br,1H); 7.43 (s,1H); 7.54 (s,2H). |
| 1.20-60 E2 | [CDCl₃] 0,56-0.62 (m,2H); 0.82-0.89 (m,2H); 2.77-2.86 (m-1H); 3.33 (d,1H); 3.86 (d,1H); 6.78 (s br,1H); 7.43 (s,1H); 7.54 (s,2H). |
| 1.20-62 | [CDCl₃] D1: 0.27-0.32 (m,2H); 0.43-0.53 (m,2H); 0.82-0.93 (m,1H); 1.26 (d,3H); 3.34 (d,1H); 3.39 (s,3H); 3.37-3.46 (m,1H); 3.82 (d,1H); 6.76 (s br,1H); 7.43 (s,1H); 7.56 (s,2H). D2: 0.33-0.43 (m,2H); 0.53-0.62 (m,2H); 0.82-0.93 (m,1H); 1.28 (d,3H); 3.35 (d,1 H); 3.41 (s,3H); 3.37-3.46 (m, 1 H); 3.83 (d, 1 H); 6.76 (s br,1 H); 7.43 (s, 1 H); 7.56 (s,2H). |
| 1.20-64 | [CDCl₃] 1.26-1.38 (m,2H); 1.58-1.68 (m,2H); 3.30 (d,1H); 3.35 (s,3H); 3.96 (d,1H); 7.23 (s,1H); 7.45 (s,1H); 7.55 (s,2H). |
| 1.20-69 | [CDCl₃] 3.30 (d,1H); 3.36 (s,3H); 3.39(dd,4H); 3.87(d,1H); 5.22-5.33 (m,1H); 7.18 (d br,1H); 7.44 (s,1H); 7.55 (s,1H). |
| 1.20-70 | [CDCl₃] D1 3.20-3.30 (m,2H); 3,32 (d,1H); 3.36 (s,3H); 3.49-3.58 (m,1H); 3.67-3.74 (m,1H); 3.90 (d,1H); 4.10-4.18 (m,2H); 4.41-4.53 (m,1H); 7.19 (s br,1H); 7.45 (s,1H); 7.55 (s,2H). D2 3.20-3.30 (m,2H); 3,32 (d,1H); 3.36 (s,3H); 3.49-3.58 (m,1H); 3.67-3.74 (m,1H); 3.91 (d,1H); 4.10-4.18 (m,2H); 5.14-5.23 (m,1H); 7.12 (s br,1H); 7.45 (s,1H); 7.55 (s,2H). |
| 1.20-71 | [CDCl₃] 3.31 (d,1H); 3.37 (s,3H); 3.93 (d,1H); 4.00-4.11 (m,2H); 4.52-4.63 (m,2H); 4.71-4.79 (m,1H); 7.35 (d br,1H); 7.45 (s,1H); 7.55 (s,2H). |
| 1.20-73 | [CDCl₃] 1.83 -1.92 (m,1H); 2.26-2.38 (m,1H); 3.32 (dd,1H); 3.34 (s,3H); 3.70-3.76 (m,1H); 3.78-3.91(m,3H); 3.93-4.03(m,1H); 4.51-4.61 (m,1H); 6.93 (s br,1H); 7.44 (s,1H); 7.55 (s,2H). |
| 1.20-74 | [CDCl₃] 1.59-1.68(m,1H); 2.02-2.14 (m,1 H); 2.47-2.59 (m, 1 H); 3.34 (d,1 H); 3.37 (s,3H); 3.33-3.45 (m,2H); 3.56 (dd,1H); 3.72-3.79 (m,1H); 3.81-3.94 (m,2H); 3.87 (d,1H); 6.91 (t br,1H); 7.44 (s,1H); 7.55 (s,2H). |
| 1.20-76 | [CDCl₃] D1: 0.92 (t,3H); 1.18 (d,3H); 1.32-1.43 (m,2H); 1.43-1.52 (m,2H); 3.33 (d,1H); 3.40 (s,3H); 3.82 (d,1H); 3.97-4.09 (m,1H); 6.53 (d br,1H); 7.44 (s,1H); 7.55 (s,2H). D2: 0.94 (t,3H); 1.20 (d,3H); 1.32-1.43 (m,2H); 1.43-1.52 (m,2H); 3.34 (d,1H); 3.40 (s,3H); 3.84 (d,1H); 3.97-4.09 (m,1H); 6.53 (d br,1H); 7.44 (s,1H); 7.55 (s,2H). |
| 1.20-93 | [CDCl₃] 2.63 (t,2H); 3.33 (d,1H); 3.36 (s,3H); 3.62 (q,2H); 3.73 (s,3H); 3.85(d,1H); 7.27 (t br,1 H); 7.43 (s,1 H); 7.56 (2H). |
| 1.20-96 | [CDCl₃] 1.28 (m,6H); 2.56 (m,2H); 3.34 (d,1H), 3.36 (s,3H); 3.85 (d,1H); 4.16 (m,2H); 4.39 (m,1H); 7.24 (s,1H); 7.42 (s,1H); 7.55 (s,2H). |
| 1.20-102 | [CDCl₃] 1.91 (quint,2H); 2.40 (t,2H); 3.30 (d,1H); 3.37 (s,3H); 3.39 (m,2H); 3.69 (s,3H); 3.86 (d,1H); 6.90 (s,1H); 7.44 (s,1H); 7.55 (s,2H). |
| 1.20-110 | [CDCl₃] 3.35 (s,3H); 3.36 (d,1H); 3.92 (d,1H); 4.52 (m,2H); 7.20 (s,1H); 7.32 (d,1H); 7.45 (s,1H); 7.55 (s,2H); 7.65 (d,1H); 8.35 (s,1H). |
| 1.20-119 | [CDCl₃] 3.35 (d,1H); 3.36 (s,3H); 3.94 (d,1H); 4.56-4.68 (m,2H); 7.25 (s br,1H); 7.44 (s,1H); 7.55 (s,2H); 7.68 (d,1H); 7.85 (d,1H); 8.68 (s,1H). |
| 1.20-135 | [CDCl₃] 3.34 (d,1H); 3.38 (s,3H); 3.96 (d,1H); 4.55 (d,2H); 7.21 (d,3H); 7.44 (s,1H); 7.56 (s,2H); 8.59 (d,2H). |
| 1.20-136 | [CDCl₃] 3.86 (d,1H); 3.38 (s,3H); 3.95 (d,1H); 4.45-4.60 (m,2H); 7.16 (d,1H); 7.23 (s br,1H); 7.24 (s,1H); 7.44 (s,1H); 7.56 (s,2H); 8.36 (d,1H). |
| 1.20-137 | [CDCl₃] 3.34 (d,1H); 3.38 (s,3H); 3.96 (d,1H); 4.57 (m,2H); 6.86 (m,1H); 7.11 (m,1H); 7.20 (s,1H); 7.47 (s,1H); 7.57 (s,2H); 8.20 (d,1H). |
| 1.20-138 | [CDCl₃] 3.37 (d,1H); 3.38 (s,3H); 3.97 (d,1H); 4.45-4.58 (m,2H); 7.18 (d,1H); 7.23 (t br,1H); 7.41 (s,1H); 7.44 (s,1H); 7.57 (s,2H); 8.34 (d,1H). |
| 1.20-142 | [CDCl₃] 3.36 (d,1H); 3.38 (s,3H); 3.92 (d,1H); 3.93 (s,3H); 4.41-4.56 (m,2H); 6.65 (s,1H); 6.79 (d,1H); 7.13 (t br,1H); 7.44 (s,1H); 7.56 (s,2H); 8.13 (d,1H). |
| 1.20-147 | [CDCl₃] 2.56 (s,3H); 3.35 (d,1H); 3.38 (s,3H); 3.95 (d,1H); 4.44-4.56 (m,2H); 7.02 (d,1H); 7.08 (s,1H); 7.14 (t br,1H); 7.45 (s,1H); 7.56 (s,2H); 8.47 (d,1H). |
| 1.20-148 | [CDCl₃] 1.31 (t,3H); 2.81 (q,2H); 3.36 (d,1H); 3.38 (s,3H); 3.95 (d,1H); 4.45-4.58 (m,2H); 7.03 (d,1H); 7.08 (s,1H); 7.15 (t br,1H); 7.45 (s,1H); 7.56 (s,2H); 8.50 (d,1H). |
| 1.20-149 | [CDCl₃] 0.94-1.06 (m,4H); 1.97-2.06 (m,1H); 3.37 (d,1H); 3.38 (s,3H); 3.95 (d,1H); 4.42-4.56 (m,2H); 6.95 (d,1H); 7.05 (s,1H); 7.13 (t br,1H); 7.44 (s,1H); 7.56 (s,2H); 8.40 (d,1 H). |
| 1.20-150 | [CDCl₃] 0.97 (t,3H); 1.70-1.82 (m,2H); 2.73-2.83 (m,2H); 3.37 (d,1 H); 3.38 (s,3H); 3.94 (d,1H); 4.43-4.58 (m,2H); 7.04 (d,1H); 7.07 (s,1H); 7.15 (s br,1H); 7.45 (s,1H); 7.56 (s,2H); 8.50 (d,1H). |
| 1.20-152 | [CDCl₃] 3.36 (d,1H); 3.38 (s,3H); 3.97 (d,1H); 4.55-4.69 (m,2H); 7.28 (t br,1H);7.42 (d,1H); 7.45 (s,1H); 7.56 (s,2H); 7.61 (s,1H); 8.71 (d,1H). |
| 1.20-191 | [CDCl₃] 3.39 (d,1H); 3.43 (s,3H); 3.87 (d,1H); 3.95 (s,6H); 4.50-4.64 (m,2H); 7.43 (s,1H); 7.56 (s,2H); 7.45 (s br,1H). |
| 1.20-199 | [CDCl₃] 3.33 (d,1H), 3.34 (s,3H); 3.89 (d,1H); 4.33-4.47 (m,2H); 7.08 (t br,1H); 7.44 (s,1H); 7.56 (s,2H); 8.31 (s,1H); 8.45 (s,1H). |
| 1.20-200 | [CDCl₃] 2.46 (s,3H); 3.32 (d,1H); 3.33 (s,3H); 3.88 (d,1H); 4.23-4.37 (m,2H); 7.00 (s br,1H); 7.44 (s,1H); 7.56 (s,2H); 8.19 (s,1H). |
| 1.20-201 | [CDCl₃] 2.26 (s,3H); 2.42 (s,3H); 3.32 (d,1H); 3.33 (s,3H); 3.91 (d,1H); 4.22-4.33 (m,2H); 6.80 (t br,1H); 7.46 (s,1H); 7.55 (s,2H). |
| 1.20-202 | [CDCl₃] 1.30 (t,3H); 2.42 (s,3H); 2.65 (q,2H); 3.32 (d,1H); 3.90 (d,1H); 4.21-4.34 (m,2H); 6.75 (s br,1H); 7.44 (s,1H); 7.55 (s,2H). |
| 1.20-203 | [CDCl₃] 1.29 (t,3H); 1.30 (t,3H); 2.66 (q,2H); 2.78 (q,2H); 3.32 (d,1H); 3.33 (s,3H); 3.89 (d,1H); 4.24-4.33 (m,2H); 7.75 (s br,1H); 7.44 (s,1H); 7.55 (s,2H). |
| 1.20-206 | [CDCl₃] 1.49 (t,3H); 3.34 (s,3H); 3.35 (d,1H); 3.88 (d,1H); 4.14 (m,2H); 4.38 (m,2H); 6.90 (s,1H); 7.40 (s,1H); 7.43 (m,2H); 7.54 (s,2H). |
| 1.20-207 | 3.33 (d,1H); 3.34 (s,3H); 3.88 (d,1H); 4.33-4.46 (m,2H); 4.68 (q,2H); 7.01 (s br,1H); 7.43 (s,1H); 7.53 (s,1H); 7.55 (s,2H); 7.56 (s,1H). |
| 1.20-208 | [CDCl₃] 3.34 (s,3H); 3.36 (d,1H); 3.89 (d,1H); 4.38-4.48 (m,4H); 5.93-6.21 (tt,1H); 6.98 (s,1H); 7.42 (m,1H); 7.49 (s,1H); 7.54 (m,3H). |
| 1.20-210 | [CDCl₃] 0.98-1.08 (m,2H); 1.09-1.14 (m,2H); 3.33 (d,1H); 3.34 (s,3H); 3.53-3.60 (m,1H); 3.86 (d,1H); 4.30-4.42 (m,2H); 6.92 (s br,1H); 7.44 (s,2H); 7.46 (s,1H); 7.55 (s,2H). |
| 1.20-211 | [CDCl₃] 2.24 (s,3H); 3.33 (d,1H); 3.34 (s,3H); 3.81 (s,3H); 3.87 (d,1H); 4.26-4.38 (m,2H); 6.82 (s br, 1H); 7.28 (s,1H); 7.43 (s,1H); 7.54 (s,2H). |
| 1.20-212 | [CDCl₃] 1.46 (t,3H); 2.25 (s,3H); 3.33 (s,3H); 3.36 (d,1H); 3.86 (d,1H); 4.08 (q,2H); 4.33 (m,2H); 6.80 (s,1H); 7.31 (s,1H); 7.43 (s,1H); 7.54 (s,2H). |
| 1.20-213 | [CDCl₃] 1.47 (t,3H); 3.34 (d,1H); 3.35 (s,3H); 3.84 (d,1H); 4.08 (q, 2H); 4.33 (d,2H); 6.90 (t,1H); 7.03 (s br,1H);7.21 (d,2H); 7.42 (s,1H). |
| 1.20-241 | [CDCl₃] 3.36 (d,1H); 3.38 (s,3H); 3.97 (d,1H); 4.54-4.68 (m,2H); 6.94 (d,1H); 7.27 (s br,1 H); 7.45 (s,1 H); 7.57 (s,2H); 8.19 (s,1 H). |
| 1.20-242 | [CDCl₃] 3.36 (d,1H); 3.39 (s,3H); 3.96 (d,1H); 4.51-4.64 (m,2H); 7.23 (s br,1H); 7.31 (s,1H); 7.45 (s,1H); 7.57 (s,2H); 8.36 (s,1H). |
| 1.20-243 | [CDCl₃] 3.36 (d,1H); 3.39 (s,3H); 3.96 (d,1H); 4.51-4.63 (m,2H); 7.25 (s br,1H); 7.30 (s,1H); 7.45 (s,1H); 7.57 (s,2H); 8.46 (s,1H). |
| 1.20-244 | [CDCl₃] 3.36 (d,1H); 3.38 (s,3H); 3.91 (s,3H); 3.92 (d,1H); 4.45-4.59 (m,2H); 6.73 (s,1H); 7.21 (t br,1H); 7.44 (s,1H); 7.56 (s,2H); 8.23 (s,1H). |
| 1.20-255 | [CDCl₃] 1.47 (t,3H); 3.33 (d,1H); 3.34 (s,3H); 3.85 (d,1H); 4.11 (q, 2H); 4.32 (d,2H); 7.03 (s br,1H); 7.40 (s,1H); 7.43 (s,1H), 7.55 (s,2H). |
| 1.20-257 | [CDCl₃] 1.42 (t,3H); 3.33 (d,1H); 3.34 (s,3H); 3.81 (d,1H); 3.91 (s,3H); 3.96 (q,2H); 4.21-4.32 (m,2H); 7.00 (s br,1H); 7.20 (s,1H); 7.43 (s,1H); 7.54 (s,2H). |

**Analytische Daten Tabelle 1.25**

| Nr. | NMR |
|---|---|
| 1.25-1 | [CDCl₃] 3.37 (d,1H); 3.41 (s,3H); 3.87 (d,1H); 5.60 (s br,1H); 6.76 (s br,1H); 7.32 (s,1H); 7.45 (s,1H); 7.58 (s,1H). |
| 1.25-9 | [CDCl₃] 3.39 (s,3H); 3.39 (d,1H); 3.37 (d,1H); 3.33-3.46 (m,1H); 4.02-4.18 (m,1H); 7.05 (t br,1H); 7.32 (s,1H); 7.45 (s,1H); 7.58 (s,1H). |
| 1.25-16 | [CDCl₃] 3.36 (d,1H); 3.38 (s,3H); 3.89 (d,1H); 3.90-4.06 (m,2H); 5.20 (d,1H); 5.25 (d,1H); 5.81-5.92 (m,1H); 6.84 (s br,1H); 7.31 (s,1H); 7.45 (s,1H); 7.58 (s,1H). |
| 1.25-60 | [CDCl₃] 0.57-0.61 (m,2H); 0.81-0.87 (m,2H); 2.78-2.86 (m,1H); 3.34 (d,1H); 3.35 (s,3H); 3.87 (d,1H); 6.78 (s br,1H); 7.31 (s,1H); 7.44 (s,1H); 7.57 (s,1H). |
| 1.25-94 | [CDCl₃] 1.28 (t,3H); 2.59 (t,2H); 3.33 (d,1H); 3.36 (s,3H); 3.62 (q,2H); 3.85 (d,1H); 4.18 (q,1H); 7.28 (s br,1H); 7.31 (s,1H); 7.44 (s,1H); 7.58 (s,1H). |
| 1.25-96 | [CDCl₃] 1.23-1.32 (m,6H); 2.50-2.62 (m,2H); 3.33 (d,1H); 3.37 (s,3H); 3.85 (d,1H); 4.11-4.21 (m,2H); 4.33-4.45 (m, 1 H); 7.25 (s br,1 H); 7.45 (s, 1 H); 7.52 (s,1 H); 7.58 (s,1H). |
| 1.25-102 | [CDCl₃] 1.92 (quin, 2H); 2.40 (t,2H); 3.34 (d,1H); 3.38 (s,3H); 3.36-3.43 (m,2H); 3.69 (s,3H); 3.87 (d,1H); 6.92 (s br,1H); 7.31 (s,1H); 7.44 (s,1H); 7.58 (s,1H). |
| 1.25-110 | [CDCl₃] 3.35 (d,1H); 3.36 (s,3H); 3.92 (d,1H); 4.45-4.57 (m,2H); 7.15 (t br,1H); 7.32 (d,1H); 7.34 (s,1H); 7.45 (s,1H); 7.58 (s,1H); 6.64 (d,1H); 8.35 (s,1H). |
| 1.25-136 | [CDCl₃] 3.37 (d,1H); 3.39 (s,3H); 3.98 (d,1H); 4.46-4.60 (m,2H); 7.17 (d,1H); 7.33 (s,1H); 7.46 (s,1H); 7.59 (s,1H); 8.36 (d,1H). |
| 1.25-206 | [CDCl₃] 1.48 (t,1H); 3.34 (d,1H); 3,35 (s,3H); 3.87 (d,1H); 4.15 (q,2H); 4.32-4.43 (m,2H); 6.92 (s br,1H); 7.31 (s,1H); 7.40 (s,1H); 7.44 (s,1H); 7.46 (s,1H); 7.57 (s,1H). |
| 1.25-212 | [CDCl₃] 1.45 (t,3H); 2.24 (s,3H); 3.32 (d,1H); 3.33 (s,3H); 3.89 (d,1H); 4.08 (q,2H); 4.28-4.38 (m,2H); 6.79 (t br,1H); 7.31 (s,1H); 7.44 (s,1H); 7.57 (s,1H). |

**Analytische Daten Tabelle 1.29**

| Nr. | NMR |
|---|---|
| 1.29-9 | [CDCl₃] 2.37 (s,3H); 3.38 (s,3H); 3.42 (d,1H); 3.83-3.90 (m,2H); 4.02-4.13 (m,1H); 7.08 (s,1H); 7.40 (m,2H); 7.60 (s,1H). |
| 1.29-41 D1 | [CDCl₃] D1: 1.63 (d,3H); 2.37 (s,3H); 3.36 (d,1H); 3.38 (s,3H), 3.81-3.91 (m,1H); 4.95 (quint,1H); 7.03 (d,1H); 7.41 (m,2H); 7.61 (s,1H). |
| 1.29-41 D2 | [CDCl₃] D2: 1.63 (d,3H); 2.37 (s,3H); 3.36 (d,1H); 3.38 (s,3H); 3.95 (d,1H); 4.93 (quint,1H); 7.06 (d,1H); 7.40 (m,2H); 7.61 (s,1H). |
| 1.29-48 | [CDCl₃] 1.40 (t,3H); 2.37 (s,3H); 3.38 (s,3H); 3.41 (d,1H); 3.77-3.89 (m,1H); 4.68-4.76 (m,1H); 6.86 (m,1H); 7.41 (m,2H); 7.61 (1H). |
| 1.29-60 | [CDCl₃] 0.59 (m,2H); 0.83 (m,2H); 2.36 (s,3H); 2.82 (m,1H); 3.34 (s,3H); 3.37 (d,1H); 3.87 (d,1H); 6.80 (s,1H); 7.40 (m,2H); 7.60 (s,1H). |
| 1.29-73 | [CDCl₃] 1.88 (m,1H), 2.28-2.35 (m,1H); 2.37 (s,3H); 3.32 (d,1H); 3.36 (s,3H); 3.72 (m,1H); 3.78-3.88 (m,3H); 3.98 (q,1H); 4.55 (m,1H); 6.93 (d,1H); 7.41 (s,2H); 7.61 (s,1H). |
| 1.29-93 | [CDCl₃] 2.36 (s,3H); 2.61 (t,2H); 3.35 (s,3H); 3.37 (d,1H); 3.62 (q,2H); 3.72 (s,3H); 3.86 (d,1H); 7.40 (m,2H); 7.60 (s,1H). |
| 1.29-96 | [CDCl₃] 1.25-1.31 (m,6H); 2.36 (s,3H); 2.57 (m,2H); 3.32 (d,1H); 3.37 (s,3H); 3.85 (d,1H); 4.11-4.20 (m,2H); 4.39 (m,1H); 7.22 (s,1H); 7.41 (s,2H); 7.61 (s,1H); |
| 1.29-136 | [CDCl₃] 2.37 (s,3H); 3.38 (s,3H); 3.41 (d,1H); 3.98 (d,1H), 4.53 (t,2H); 7.16 (d,1H), 7.26 (m,1 H), 7.42 (s, 2H); 7.61 (s,1 H); 8.36 (d, 1 H). |
| 1.29-137 | [CDCl₃] 2.37 (s,3H); 3.38 (s,3H); 3.41 (d,1H); 3.96 (d,1H); 4.57 (t,2H); 6.86 (s,1H); 7.11 (m,1H); 7.41 (s, 2H); 7.61 (s,1H); 8.20 (d,1H). |
| 1.29-142 | [CDCl₃] 2.37 (s,3H), 3.37 (s,3H), 3.41 (d,1H); 3.89 (d,1H); 3.93 (s,3H); 4.46-4.51 (m,2H); 6.65 (s,1H); 6.80 (d,1H); 7.16 (s,1H); 7.41 (s,2H); 7.61 (s,1H); 8.12 (d,1H). |
| 1.29-148 | [CDCl₃] 1.30 (t,3H); 2.37 (s,3H); 2.82 (q,2H); 3.38 (s,3H); 3.41 (d,1H); 3.63 (d,1H); 4.51 (t,2H); 7.02 (d,1H); 7.08 (s,1H); 7.19 (s,1H); 7.41 (s,2H); 7.62 (s,1H); 8.50 (d,1H). |
| 1.29-149 | [CDCl₃] 0.97-1.03 (m,4H); 2.00 (m,1H); 2.37 (s,3H); 3.38 (s,3H); 3.41 (d,1H); 3.94 (d,1H); 4.49 (m,2H); 6.96 (d,1H); 7.05 (s,1H); 7.14 (s,1H); 7.41 (s,2H); 7.61 (s,1H); 8.40 (d,1H). |
| 1.29-150 | [CDCl₃] 0.96 (t,3H); 1.76 (sext,2H); 2.37 (s,3H); 2.75 (m,2H); 3.38 (s,3H), 3.41 (d,1H); 3.93 (d,1H); 4.50 (t,2H); 7.02 (d,1H); 7.06 (s,1H); 7.18 (s,1H); 7.41 (s,2H); 7.61 (s,1H); 8.50 (d,1H). |
| 1.29-152 | [CDCl₃] 2.37 (s,3H); 3.38 (s,3H); 3.41 (d,1H); 3.96 (d,1H); 4.58-4.64 (m,2H); 7.30 (s,1H); 7.44 (m,3H); 7.61 (d,2H); 8.70 (d,1H). |
| 1.29-206 | [CDCl₃] 1.48 (t,3H); 2.36 (s,3H); 3.34 (s,3H); 3.38 (d,1H); 3.88 (d,1H); 4.16 (q,2H); 4.38 (m,2H); 6.93 (s,1H); 7.40 (s,3H); 7.46 (s,1H); 7.60 (s,1H). |
| 1.29-207 | [CDCl₃] 2.36 (s,3H); 3.34 (s,3H); 3.38 (d,1H); 3.88 (d,1H); 4.40 (t,2H); 4.67 (q,2H); 7.03 (s,1H); 7.41 (m,2H); 7.53 (s,1H); 7.56 (s,1H); 7.61 (s,1H). |
| 1.29-208 | [CDCl₃] 2.36 (s,3H); 3.34 (s,3H); 3.35 (d,1H); 3.85 (d,1H); 4.31-4.50 (m, 4H); 6.07 (tt,1H); 6.99 (t br,1H); 7.39 (s,2H); 7.48 (s,1H); 7.53 (s,1H); 7.60 (s,1H). |
| 1.29-212 | [CDCl₃] 1.46 (t,3H); 2.25 (s,3H); 2.36 (s,3H); 3.34 (s,3H); 3.38 (d,1H); 3.88 (d,1H); 4.07 (q,2H); 4.33 (m,2H); 6.80 (s,1H); 7.31 (s,1H); 7.40 (s,2H); 7.61 (s,1H). |

**Analytische Daten Tabelle 1.32**

| Nr. | NMR |
|---|---|
| 1.32-1 | [CDCl₃] 3.36 (d,1H); 3.41 (s,3H); 3.87 (d,1H); 5.68 (s br,1H); 6.66 (s br,1H); 7.47 (s,1H); 7.49 (s,1H); 7.73 (s,1H). |
| 1.32-9 | [CDCl₃] 3.39 (s,3H); 3.39 (d,1H); 3.88 (d,1H); 3.85-3.97 (m,1H); 4.01-4.18 (m,1H); 7.05 (t br,1H); 7.48 (s,1H); 7.49 (s,1H); 7.73 (s,1H). |
| 1.32-16 | 3.36 (d,1H); 3.38 (s,3H); 3.88 (d,1H); 3.90-4.05 (m,2H); 5.20 (d,1H); 5.24 (d,1H); 5.80-5.92 (m,1H); 6.85 (s br,1H); 7.47 (s,1H); 7.49 (s,1H); 7.73 (s,1H). |
| 1.32-60 | [CDCl₃] 0.60 (m,2H); 0.84 (m,2H), 2.81 (m,1H); 3.32 (d,1H); 3.35 (s,3H); 3.87 (d,1H); 6.78 (s br,1H); 7.46 (s,1H); 7.48 (s,1H); 7.72 (s,1H). |
| 1.32-94 | [CDCl₃] 1.28 (t,3H); 2.59 (t,2H); 3.32 (d,1H); 3.36 (s,3H); 3.62 (q,2H); 3.86 (d,1H); 4.18 (q,2H); 7.28 (s br,1H); 7.46 (s,1H); 7.48 (s,1H); 7.73 (s,1H). |
| 1.32-96 | [CDCl₃] 1.22-1.33 (m,6H); 2.49-2.12 (m,2H); 3.33 (d,1H); 3.37 (d,1H); 3.84 (d,1H); 4.10-4.23 (m,2H); 4.34-4.44 (m,1H); 7.25 (s br,1H); 7.46 (s,1H); 7.49 (s,1H); 7.73 (s,1H). |
| 1.32-102 | [CDCl₃] 1.92 (pent,2H); 2.40 (t,2H); 3.35 (d,1H); 3.40 (s,3H); 3.35-3.44 (m,2H); 3.69 (s,3H); 3.87 (d,1H); 6.92 (s br,1H); 7.46 (s,1H); 7.49 (s,1H); 7.73 (s,1H). |
| 1.32-110 | [CDCl₃] 3.34 (d,1H); 3.37 (s,3H); 3.92 (d,1H); 4.46-4.59 (m,2H); 7.15 (t br,1H); 7.33 (d,1H); 7.47 (s,1H); 7.49 (s,1H); 7.64 (d,1H); 7.73 (s,1H); 8.35 (s,1H). |
| 1.32-136 | [CDCl₃] 3.37 (d,1H); 3.39 (s,3H); 3.99 (d,1H); 4.48-4.58 (m,2H); 7.16 (d,1H); 7.24 (s br,1H); 7.48 (s,1H); 7.51 (s,1H); 7.75 1H); 8.36 (d,1H). |
| 1.32-206 | [CDCl₃] 1.48 (t,3H); 3.34 (d,1H); 3.35 (s,3H); 3.89 (d,1H); 4.15 (q,2H); 4.31-4.43 (m,2H); 6.92 (s br,1H); 7.41 (s,1H); 7.47 (s,2H); 7.48 (s,1H); 7.72 (s,1H). |
| 1.32-212 | [CDCl₃] 1.44 (t,3H); 2.25 (s,3H); 3.34 (d,1H); 3.35 (s,3H); 3.88 (d,1H); 4.03-4.18 (m,2H); 4.28-4.38 (m,2H); 6.79 (s br,1H); 7.32 (s,1H); 7.46 (s,1H); 7.72 (s,1H). |

**Analytische Daten Tabelle 1.33**

| Nr. | NMR |
|---|---|
| 1.33-1 | [CDCl₃] 3.40 (d,1H); 3.41 (s,3H); 3.93 (d,1H); 5.64 (s br,1H); 6.66 (s br,1H); 7.84 (s,1H); 7.85 (s,1H); 8.01 (s,1H). |
| 1.33-9 | 3.38 (s,3H); 3.43 (d,1H); 3.83-3.98 (m,1H); 3.42 (d,1H); 4.03-4.18 (m,1H); 7.06 (s br,1H);7.85 (s,2H); 8.01 (s,1H). |
| 1.33-16 | [CDCl₃] 3.38 (s,3H); 3.39 (d,1H); 3.91 (d,1H); 3.91-4.06 (m,2H); 5.20 (d,1H); 5.28 (d,1 H); 5.80-5.92 (m,1 H); 6.84 (s br,1 H); 7.83 (s,1 H); 7.84 (s,1 H); 8.00 (s,1 H). |
| 1.33-60 | [CDCl₃] 0.60 (m,2H); 0.84 (m,2H); 2.81 (m,1H); 3.35 (s,3H); 3.36 (d,1H); 3.91 (d,1H); 6.79 (s br,1H); 7.83 (s,1H); 7.84 (s,1H); 7.99 (s,1H). |
| 1.33-94 | [CDCl₃] 1.28 (t,3H); 2.56 (t,2H); 3.37 (d,1H); 3.38 (s,3H); 3.63 (q,2H); 3.89 (d,1H); 4.18 (q,2H); 7.28 (s br,1H); 7.83 (s,1H); 7.84 (s,1H); 8.00 (s,1H). |
| 1.33-96 | [CDCl₃] 1.24-1.35 (m,6H); 2.50-2.64 (m,2H); 3.36 (d,1H); 3.38 (s,3H); 3.89 (d,1H); 4.12-4.23 (m,2H); 4.34-4.45 (m,1H); 7.27 (s br, 1H); 7.83 (s,1H); 7.85 (s,1H); 8.00 (s,1H). |
| 1.33-102 | [CDCl₃] 1.92 (pent,2H); 2.40 /t,2H); 3.39 (s,3H); 3.39 (d,1H); 3.39 (t,2H); 3.69 (s,3H); 3.91 (d,1H); 6.93 (s br,1H); 7.83 (s,1H); 7.85 (s,1H); 8.00 (s,1H). |
| 1.33-110 | [CDCl₃] 3.36 (s,3H); 3.38 (d,1H); 3.97 (d,1H); 4.45-4.59 (m,2H); 7.15 (t br,1H); 7.33 (d,1H); 7.65 (dd,1H); 7.84 (s,2H); 8.00 (s,1H); 8.36 (d,1H). |
| 1.33-136 | [CDCl₃] 3.39 (s,3H); 3.41 (d,1H); 4.03 (d,1H); 4.49-4.59(m,2H); 7.17 (d,1H); 7.25 (s br,1H); 7.85 (s,1H); 7,86 (s,1H); 8.02 (s,1H); 8.37 (d,1H). |
| 1.33-206 | [CDCl₃] 1.49 (t,3H); 3.35 (s,3H); 3.37 (d,1H); 3.92 (d,1H); 4.15 (q,2H); 4.32-4.43 (m,2H); 6.92 (s br,1H); 7.41 (s,1H); 7.46 (s,1H); 7.84 (s,2H); 8.00 (s,1H). |
| 1.33-212 | [CDCl₃] 1.46 (t, 3H); 2.25 (s,3H); 3.35 (s,3H); 3.37 (d,1H); 3.43 (d,1H); 4.08 (q, 2H); 4.26-4.38 (m,2H); 6.79 (s br, 1H); 7.31 (s,1H); 7.83 (s,1H); 7.84 (s,1H); 8.00 (s,1H). |

**Analytische Daten Tabelle 1.56**

| Nr. | NMR |
|---|---|
| 1.56-260 | [CDCl₃] 1.26 (t,3H); 2.70 (q,2H); 3.47 (s,3H); 3.53 (d,1H); 4.03 (d,1H); 7.28 (d,2H); 7.61 (d,2H); 8.51 (s br,1H). |
| 1.56-261 | [CDCl₃] 1.26 (t,3H); 2.70 (q,2H); 3.48 (s,3H); 3.55 (d,1H); 4.01 (d,1H); 7.27 (d,2H); 7.61 (d,2H); 8.57 (s br,1H). |
| 1.56-262 | [CDCl₃] 1.26 (t,3H); 2.70 (q,2H); 3.47 (d,1H); 3,48 (s,3H); 4.08 (d,1H); 7.20-7.30 (m,2H); 7.6-7.66 (m,4H); 8.44 (d,1H); 9.20 (s br,1H). |
| 1.56-263 | [CDCl₃] 1.26 (t,3H); 2.69 (q,2H); 3.45 (s,3H); 3.49 (d,1H); 4.00 (d,1H); 7.27 (d,2H); 7.62 (m,4H); 7.75 (d,2H); 8.67 (s br,1H). |

**Analytische Daten Tabelle 1.57**

| Nr. | NMR |
|---|---|
| 1.57-264 | [CDCl₃] 3.37 (d,1H); 3.60 (s,3H); 4.05 (d,1H); 7.23 (d,2H); 7.36 (d,1H); 7.51 (d,1H); 7.67 (d 2H); 8.58 (s br,1H). |
| 1.57-265 | [CDCl₃] 3.36 (d,1H); 3.59 (s,3H); 4.05 (d,1H); 7.36 (d,1H); 7.45-7.56 (m,5H) 8.53 (s br,1 H). |

**Analytische Daten Tabelle 2.2**

| Nr. | NMR |
|---|---|
| 2.2-1 | [CDCl₃] 1.26 (t,3H); 3.42 (d,1H); 3.63 (m,2H); 3.86 (d,1H); 5.70 (s br,1H); 6.70 (s br,1H); 7.16 (m,1H); 7.40 (m,3H). |
| 2.2-9 | [CDCl₃] 1.25 (t,3H); 3.43 (d,1H); 3.60 (q,2H); 3.82 (d,1H); 3.90 (m,1H); 4.03-4.14 (m,1H); 7.10 (s br,1H); 7.13-7.18 (m,1H); 7.40 (m,3H). |
| 2.2-16 | [CDCl₃] 1.25 (t,3H); 3.40 (d,1H); 3.60 (m,2H); 3.85 (d,1H); 3.90-4.02 (m,2H); 5.17-5.27 (m,2H); 5.87 (m,1H); 6.90 (s br,1H); 7.17 (m,1H); 7.40 (m,3H). |
| 2.2-60 | [CDCl₃] 0.60 (m,2H); 0.82 (m,2H); 1.24 (t,3H); 2.80 (m,2H); 3.40 (d,1 H); 3.58 (m,2H); 3.82 (d,1H); 6.82 (s br,1H); 7.15 (m,1H); 7.41 (m,2H). |
| 2.2-94 | [CDCl₃] 1.26 (m,6H); 2.59 (t,2H); 3.39 (d,1H); 3.60 (m,4H); 3.82 (d,1H); 4.18 (q,2H); 7.14 (m,1H); 7.31 (s br,1H); 7.40 (m,3H). |
| 2.2-96 | [CDCl₃] 1.23-1.31 (m,9H); 2.56 (m,2H); 3.40 (d,1H), 3.60 (m,2H); 3.80 (d,1H); 4.18 (m,2H); 4.40 (m,1H); 7.13 (m,1H); 7.27 (s br,1H); 7.40 (m,3H). |
| 2.2-102 | [CDCl₃] 1.25 (t,3H); 1.92 (quin,2H); 2.40 (t,2H); 3.39 (m,3H); 3.57 (m,2H); 3.70 (s,3H); 3.84 (d,1H); 6.98 (s br,1H); 7.16 (m,1H); 7.40 (m,3H). |
| 2.2-206 | [CDCl₃] 1.24 (t,3H); 1.48 (t,3H); 3.40 (d,1H); 3.58 (m,2H); 3.84 (d,1H); 4.16 (q,2H); 4.39 (d,2H); 6.98 (s br,1H); 7.12 (m,1H); 7.40 (m,4H); 7.46 (s,1H). |
| 2.2-212 | [CDCl₃] 1.23 (t,3H); 1.44 (m,3H); 2.26 (s,3H); 3.40 (d,1H); 3.58 (m,2H); 3.82 (d,1H), 4.08 (m,2H); 4.33 (d,2H); 6.85 (s br,1H); 7.15 (m,1H); 7.30 (s,1H); 7.40 (m,3H). |

**Analytische Daten Tabelle 2.5**

| Nr. | NMR |
|---|---|
| 2.5-9 | [CDCl₃] 1.25 (t,3H); 2.40 (s,3H); 3.48 (d,1H); 3.60 (q,2H); 3.83 (d,1H); 3.86-3.95 (m,1H), 4.05-4.12 (m,1H); 7.10 (s br,1H); 7.30 (m,2H); 7.45 (d,1H); 7.51 (s,1H). |
| 2.5-16 | [CDCl₃] 1.25 (t,3H); 2.39 (s,3H); 3.42 (d,1H); 3.61 (q,2H); 3.85 (d,1H); 3.92-4.02 (m,2H); 5.17-5.30 (m,2H); 5.82-5.92 (m,1H); 6.90 (s br,1H); 7.24-7.33 (m,2H); 7.44 (d,1H); 7.51 (s,1H). |
| 2.5-60 | [CDCl₃] 0.58 (m,2H); 0.84 (m,2H); 1.23 (t,3H); 2.39 (s,3H); 2.82 (m1H); 3.40 (d,1H); 3.58 (m,2H); 3.84 (d,1H); 6.85 (s br,1H); 7.24-7.32 (m,2H); 7.43 (d,1H); 7.50 (s,1H). |
| 2.5-94 | [CDCl₃] 1.22-1.29 (m,6H); 2.38 (s,3H); 2.61 (t,2H); 3.42 (d,1H); 3.55-3.63 (m, 4H); 3.84 (d,1H); 4.19 (q,2H); 7.24-7.32 (m,3H); 7.44 (d,1H); 7.51 (s,1H). |
| 2.5-96 | [CDCl₃] 1.22-1.31 (m,9H); 2.38 (s,3H); 2.57 (m,2H); 3.40 (d,1H); 3.60 (m,2H); 3.80 (d,1H); 4.11-4.20 (m,2H); 4.43 (m,1H); 7.24-7.32 (m,2H); 7.45 (d,1H); 7.51 (s,1H). |
| 2.5-102 | [CDCl₃] 1.25 (t,3H); 1.91 (quin,2H); 2.39 (m,5H); 3.36-3.44 (m,3H); 3.57 (m,2H); 3.69 (s,3H); 3.84 (d,1H); 6.98 (s br,1H); 7.24-7.33 (m,2H); 7.45 (d,1H); 7.51 (s,1H). |
| 2.5-206 | [CDCl₃] 1.23 (t,3H); 1.48 (t,3H); 2.39 (s,3H); 3.42 (d,1H); 3.57 (m,2H); 3.83 (d,1H), 4.15 (q,2H); 4.38 (d,2H); 6.99 (s br,1H); 7.22-7.32 (m,2H); 7.40-7.46 (m,3H); 7.50 (s,1H). |
| 2.5-212 | [CDCl₃] 1.22 (t,3H); 1.45 (t,3H); 2.26 (s,3H); 2.39 (s,3H); 3.42 (d,1H); 3.58 (m,2H); 3.84 (d,1H); 4.07 (m,2H); 4.32 (d,2H); 6.86 (s br,1H); 7.22-7.30 (m,3H); 7.43 (m,1H); 7.50 (s,1H). |

**Analytische Daten Tabelle 2.11**

| Nr. | NMR |
|---|---|
| 2.11-4 | [CDCl₃] 0.98 (t,3H); 1.27 (t,3H); 1.62 (q,2H); 3.22-3.38 (m,2H); 3.37 (d,1H); 3.52-3.65 (m,2H); 3.80 (d,1H); 6.80 (s br,1H); 6.89 (t,1H); 7.19 (d,2H). |
| 2.11-9 | [CDCl₃] 1.29 (t,3H); 3.39 (d,1H); 3.53-3.65 (m,2H); 3.82 (d,1H); 3.85-3.98 (m,1H); 4.01-4.15 (m,1H); 6.90 (t,1H); 7.05 (t br,1H); 7.20 (d,2H). |
| 2.11-60 | [CDCl₃] 0.59 (t,2H); 0.87 (m,2H); 1.26 (t,3H); 2.81 (m,1H); 3.32 (d,1H); 3.50-3.63 (m,2H); 3.82 (d,1H); 6.80 (s br,1H); 6.89 (t,1H); 7.18 (d,2H). |
| 2.11-93 | [CDCl₃] 1.25 (t, 3H); 2.60 (t,2H); 3.32 (d,1H); 3.50-3.60 (m,2H); 3.62 (q,2H); 3.71 (s,3H); 3.82 (d,1H); 6.89 (t,1H); 7.18 (d,2H); 7.28 (t br,1H). |
| 2.11-96 | [CDCl₃] 1.22-1.36 (m,9H); 2.50-2.61 (m,2H); 3.34 (d,1H); 3.53-3.65 (m,2H); 3.80 (d,1H); 4.11-4.22 (m,2H);4.34-4.45 (m1H); 6.89 (t,1H); 7.18 (d,2H); 7.25 (s br,1H). |
| 2.11-102 | [CDCl₃] 1.26 (t,3H); 1.93 (q,2H); 2.40 (t,2H); 3.34 (d,1H); 3.40 (q,2H); 3.51-3.65 (m,2H); 3.69 (s,3H); 3.83 (d,1 H); 6.85-6.93 (m, 1 H); 6.95 (t br, 1 H); 7.19 (d,2H). |
| 2.11-135 | [CDCl₃] 1.28 (t,3H); 3.38 (d,1H); 3.50-3.70 (m,2H); 3.95 (d,1H); 4.62 (t,2H); 6.90 (t,1H); 7.20 (d,2H); 7.38 (t br,1H); 7.45 (d,2H); 8.68 (d,2H). |
| 2.11-206 | [CDCl₃] 1.27 (t,3H); 1.49 (t,3H); 3.35 (d,1H); 3.50-3.63 (m,2H); 3.83 (d,1H); 4.15 (q,2H); 4.38 (d,2H); 6.89 (t,1H); 6.95 (t br,1H); 7.18 (d,2H); 7.40 (s,1H); 7.46 (s,1H). |
| 2.11-212 | [CDCl₃] 1.24 (t,3H); 1.48 (t,3H); 2.25 (s,3H); 3.35 (d,1H); 3.50-3.64 (m,2H); 3.85 (d,1H); 4.08 (q,2H); 4.34 (d,2H); 6.82 (t,1H); 6.90 (t,1H); 7.18 (d,2H); 7.32 (s,1H). |

**Analytische Daten Tabelle 2.20**

| Nr. | NMR |
|---|---|
| 2.20-4 | [CDCl₃] 0.96 (t,3H); 1.25 (t,3H); 1.55-1.64 (m,2H); 3.23-3.35 (m,2H); 3.34 (d,1H); 3.51-3.65 (m,2H); 3.80 (d,1H); 6.79 (t br,1H); 7.43 (s,1H); 7.55 (s,2H). |
| 2.20-9 | [CDCl₃] 1.27 (t,3H); 3.39 (d,1H); 3.52-3.65 (m,2H); 3.83 (d,1H); 3.83-3.97 (m,1H); 4.01-4.18 (m,1H); 7.05 (t br,1H); 7.44 (s,1H); 7.55 (s,2H). |
| 2.20-60 | [CDCl₃] 0.60 (m,2H); 0.85 (m,2H); 1.24 (t,3H); 2.80 (m,1H); 3.32 (d,1H); 3.50-3.63 (m,2H); 3.84 (d,1H); 6.80 (s br,1H); 7.42 (s,1H); 7.54 (s,2H). |
| 2.20-93 | [CDCl₃] 1.25 (t,3H); 2.61 (t,2H); 3.36 (d,1H); 3.50-3.65 (m,4H); 3.72 (s,3H); 3.83 (d,1H); 7.30 (t br,1H); 7.42 (s,1H); 7.55 (d,2H). |
| 2.20-96 | [CDCl₃] 1.23-1.31 (m,9H); 2.55 (m,2H); 3.31 (d,1H); 3.57 (m,2H); 3.80 (d,1H); 4.14 (m,2H); 4.39 (m,1H); 7.28 (s br,1H); 7.42 (s,1H); 7.55 (s,2H). |
| 2.20-102 | [CDCl₃] 1.26 (t,3H); 1.92 (quin,2H); 2.40 (t,2H); 3.32 (d,1H); 3.39 (q,2H); 3.50-3.64 (m,2H); 3.69 (s,3H); 3.83 (d,1H); 6.96 (t br,1H); 7.43 (s,1H); 7.55 (s,2H). |
| 2.20-135 | [CDCl₃] 1.27 (t,3H); 3.39 (d,1H); 3.52-3.67 (m,2H); 3.93 (d,1H); 4.55 (d,2H); 7.23 (m,3H); 7.44 (s,1 H); 7.56 (s,2H); 8.60 (d,2H). |
| 2.20-206 | [CDCl₃] 1.25 (t,3H); 1.50 (t,3H); 3.34 (d,1H); 3.50-3.64 (m,2H); 3.85 (d,1H); 4.15 (q,2H); 4.38 (d,2H); 6.95 (t br,1H); 7.40 (s,1H); 7.44 (s,1H); 7.48 (s,1H); 7.55 (s,2H). |
| 2.20-212 | [CDCl₃] 1.23 (t,3H); 1.43 (t,3H); 2.26 (s,3H); 3.34 (d,1H); 3.48-3.64 (m,2H); 3.83 (d,1H); 4.08 (q,2H); 4.33(d,2H); 6.82 (s br,1H); 7.32 (s,1H); 7.42 (s,1H); 7.54 (s,2H). |
| 3.11-9 | [CDCl₃] 3.41 (s,3H); 3.57 (d,1H), 4.08 (d,1H); 4.34 (m,1H); 4.62 (m,1H); 6.91 (m,1H); 7.20 (m,2H); 8.70 (s br,1H). |
| 3.11-60 | [CDCl₃] 0.73 (m,2H); 1.01 (m,2H); 3.30 (m,1H); 3.38 (s,3H); 3.54 (d,1H); 4.10 (d,1H); 6.90 (m,1H); 7.19 (m,2H); 8.50 (s br,1H). |
| 3.11-93 | [CDCl₃] 2.76 (m,2H); 3.38 (s,3H); 3.51 (d,1H); 3.73 (s,3H); 4.02 (m,2H); 4.10 (d,1H), 6.90 (m,1H); 7.20 (m,2H); 9.12 (s br,1H). |
| 3.11-212 | [CDCl₃] 1.47 (t,3H); 2.25 (s,3H), 3.38 (s,3H); 3.55 (d,1H), 4.09-4.14 (m,3H); 4.63 (m,2H); 6.90 (m,1H), 7.19 (m,2H); 7.39 (s,1H); 8.50 (s br,1H). |

Tabelle A beschreibt Verbindungen der allgemeinen Formel (II), worin R¹ und R² jeweils Wasserstoff bedeuten, und die Reste X², X³, X⁴, X⁵, X⁶, R³ und V die in der Tabelle angegebene Bedeutung haben.

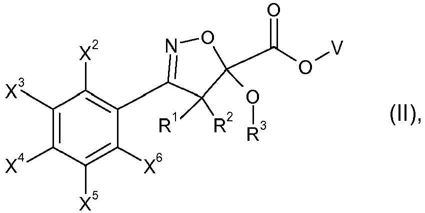

**Tabelle a: Intermediate**

| Nr. | X² | X³ | X⁴ | X⁵ | X⁶ | R³ | V |
|---|---|---|---|---|---|---|---|
| A-1 | H | H | H | H | H | Me | H |
| A-2 | H | H | H | H | H | Me | Me |
| A-3 | H | H | H | H | H | Me | Et |
| A-4 | H | H | H | H | H | Et | H |
| A-5 | H | H | H | H | H | Et | Me |
| A-6 | H | H | H | H | H | Et | Et |
| A-7 | H | H | H | F | H | Me | H |
| A-8 | H | H | H | F | H | Me | Me |
| A-9 | H | H | H | F | H | Me | Et |
| A-10 | H | H | H | F | H | Et | H |
| A-11 | H | H | H | F | H | Et | Me |
| A-12 | H | H | H | F | H | Et | Et |
| A-13 | H | H | H | Cl | H | Me | H |
| A-14 | H | H | H | Cl | H | Me | Me |
| A-15 | H | H | H | Cl | H | Me | Et |
| A-16 | H | H | H | Cl | H | Et | H |
| A-17 | H | H | H | Cl | H | Et | Me |
| A-18 | H | H | H | Cl | H | Et | Et |
| A-19 | H | H | H | Br | H | Me | H |
| A-20 | H | H | H | Br | H | Me | Me |
| A-21 | H | H | H | Br | H | Me | Et |
| A-22 | H | H | H | Br | H | Et | H |
| A-23 | H | H | H | Br | H | Et | Me |
| A-24 | H | H | H | Br | H | Et | Et |
| A-25 | H | H | H | Me | H | Me | H |
| A-26 | H | H | H | Me | H | Me | Me |
| A-27 | H | H | H | Me | H | Me | Et |
| A-28 | H | H | H | Me | H | Et | H |
| A-29 | H | H | H | Me | H | Et | Me |
| A-30 | H | H | H | Me | H | Et | Et |
| A-31 | H | H | H | Et | H | Me | H |
| A-32 | H | H | H | Et | H | Me | Me |
| A-33 | H | H | H | Et | H | Me | Et |
| A-34 | H | H | H | Et | H | Et | H |
| A-35 | H | H | H | Et | H | Et | Me |
| A-36 | H | H | H | Et | H | Et | Et |
| A-37 | H | H | H | OMe | H | Me | H |
| A-38 | H | H | H | OMe | H | Me | Me |
| A-39 | H | H | H | OMe | H | Me | Et |
| A-40 | H | H | H | OMe | H | Et | H |
| A-41 | H | H | H | OMe | H | Et | Me |
| A-42 | H | H | H | OMe | H | Et | Et |
| A-43 | H | H | H | OCF₃ | H | Me | H |
| A-44 | H | H | H | OCF₃ | H | Me | Me |
| A-45 | H | H | H | OCF₃ | H | Me | Et |
| A-46 | H | H | H | OCF₃ | H | Et | H |
| A-47 | H | H | H | OCF₃ | H | Et | Me |
| A-48 | H | H | H | OCF₃ | H | Et | Et |
| A-49 | H | H | H | CF₃ | H | Me | H |
| A-50 | H | H | H | CF₃ | H | Me | Me |
| A-51 | H | H | H | CF₃ | H | Me | Et |
| A-52 | H | H | H | CF₃ | H | Et | H |
| A-53 | H | H | H | CF₃ | H | Et | Me |
| A-54 | H | H | H | CF₃ | H | Et | Et |
| A-55 | H | H | H | CN | H | Me | H |
| A-56 | H | H | H | CN | H | Me | Me |
| A-57 | H | H | H | CN | H | Me | Et |
| A-58 | H | H | H | CN | H | Et | H |
| A-59 | H | H | H | CN | H | Et | Me |
| A-60 | H | H | H | CN | H | Et | Et |
| A-61 | H | F | H | F | H | Me | H |
| A-62 | H | F | H | F | H | Me | Me |
| A-63 | H | F | H | F | H | Me | Et |
| A-64 | H | F | H | F | H | Et | H |
| A-65 | H | F | H | F | H | Et | Me |
| A-66 | H | F | H | F | H | Et | Et |
| A-67 | H | F | H | Cl | H | Me | H |
| A-68 | H | F | H | Cl | H | Me | Me |
| A-69 | H | F | H | Cl | H | Me | Et |
| A-70 | H | F | H | Cl | H | Et | H |
| A-71 | H | F | H | Cl | H | Et | Me |
| A-72 | H | F | H | Cl | H | Et | Et |
| A-73 | H | F | H | Br | H | Me | H |
| A-74 | H | F | H | Br | H | Me | Me |
| A-75 | H | F | H | Br | H | Me | Et |
| A-76 | H | F | H | Br | H | Et | H |
| A-77 | H | F | H | Br | H | Et | Me |
| A-78 | H | F | H | Br | H | Et | Et |
| A-79 | H | F | H | Me | H | Me | H |
| A-80 | H | F | H | Me | H | Me | Me |
| A-81 | H | F | H | Me | H | Me | Et |
| A-82 | H | F | H | Me | H | Et | H |
| A-83 | H | F | H | Me | H | Et | Me |
| A-84 | H | F | H | Me | H | Et | Et |
| A-85 | H | F | H | Et | H | Me | H |
| A-86 | H | F | H | Et | H | Me | Me |
| A-87 | H | F | H | Et | H | Me | Et |
| A-88 | H | F | H | Et | H | Et | H |
| A-89 | H | F | H | Et | H | Et | Me |
| A-90 | H | F | H | Et | H | Et | Et |
| A-91 | H | F | H | OMe | H | Me | H |
| A-92 | H | F | H | OMe | H | Me | Me |
| A-93 | H | F | H | OMe | H | Me | Et |
| A-94 | H | F | H | OMe | H | Et | H |
| A-95 | H | F | H | OMe | H | Et | Me |
| A-96 | H | F | H | OMe | H | Et | Et |
| A-97 | H | F | H | OCF₃ | H | Me | H |
| A-98 | H | F | H | OCF₃ | H | Me | Me |
| A-99 | H | F | H | OCF₃ | H | Me | Et |
| A-100 | H | F | H | OCF₃ | H | Et | H |
| A-101 | H | F | H | OCF₃ | H | Et | Me |
| A-102 | H | F | H | OCF₃ | H | Et | Et |
| A-103 | H | F | H | CF₃ | H | Me | H |
| A-104 | H | F | H | CF₃ | H | Me | Me |
| A-105 | H | F | H | CF₃ | H | Me | Et |
| A-106 | H | F | H | CF₃ | H | Et | H |
| A-107 | H | F | H | CF₃ | H | Et | Me |
| A-108 | H | F | H | CF₃ | H | Et | Et |
| A-109 | H | F | H | CN | H | Me | H |
| A-110 | H | F | H | CN | H | Me | Me |
| A-111 | H | F | H | CN | H | Me | Et |
| A-112 | H | F | H | CN | H | Et | H |
| A-113 | H | F | H | CN | H | Et | Me |
| A-114 | H | F | H | CN | H | Et | Et |
| A-115 | H | Cl | H | Cl | H | Me | H |
| A-116 | H | Cl | H | Cl | H | Me | Me |
| A-117 | H | Cl | H | Cl | H | Me | Et |
| A-118 | H | Cl | H | Cl | H | Et | H |
| A-119 | H | Cl | H | Cl | H | Et | Me |
| A-120 | H | Cl | H | Cl | H | Et | Et |
| A-121 | H | Cl | H | Br | H | Me | H |
| A-122 | H | Cl | H | Br | H | Me | Me |
| A-123 | H | Cl | H | Br | H | Me | Et |
| A-124 | H | Cl | H | Br | H | Et | H |
| A-125 | H | Cl | H | Br | H | Et | Me |
| A-126 | H | Cl | H | Br | H | Et | Et |
| A-127 | H | Cl | H | Me | H | Me | H |
| A-128 | H | Cl | H | Me | H | Me | Me |
| A-129 | H | Cl | H | Me | H | Me | Et |
| A-130 | H | Cl | H | Me | H | Et | H |
| A-131 | H | Cl | H | Me | H | Et | Me |
| A-132 | H | Cl | H | Me | H | Et | Et |
| A-133 | H | Cl | H | Et | H | Me | H |
| A-134 | H | Cl | H | Et | H | Me | Me |
| A-135 | H | Cl | H | Et | H | Me | Et |
| A-136 | H | Cl | H | Et | H | Et | H |
| A-137 | H | Cl | H | Et | H | Et | Me |
| A-138 | H | Cl | H | Et | H | Et | Et |
| A-139 | H | Cl | H | OMe | H | Me | H |
| A-140 | H | Cl | H | OMe | H | Me | Me |
| A-141 | H | Cl | H | OMe | H | Me | Et |
| A-142 | H | Cl | H | OMe | H | Et | H |
| A-143 | H | Cl | H | OMe | H | Et | Me |
| A-144 | H | Cl | H | OMe | H | Et | Et |
| A-145 | H | Cl | H | OCF₃ | H | Me | H |
| A-146 | H | Cl | H | OCF₃ | H | Me | Me |
| A-147 | H | Cl | H | OCF₃ | H | Me | Et |
| A-148 | H | Cl | H | OCF₃ | H | Et | H |
| A-149 | H | Cl | H | OCF₃ | H | Et | Me |
| A-150 | H | Cl | H | OCF₃ | H | Et | Et |
| A-151 | H | Cl | H | CF₃ | H | Me | H |
| A-152 | H | Cl | H | CF₃ | H | Me | Me |
| A-153 | H | Cl | H | CF₃ | H | Me | Et |
| A-154 | H | Cl | H | CF₃ | H | Et | H |
| A-155 | H | Cl | H | CF₃ | H | Et | Me |
| A-156 | H | Cl | H | CF₃ | H | Et | Et |
| A-157 | H | Cl | H | CN | H | Me | H |
| A-158 | H | Cl | H | CN | H | Me | Me |
| A-159 | H | Cl | H | CN | H | Me | Et |
| A-160 | H | Cl | H | CN | H | Et | H |
| A-161 | H | Cl | H | CN | H | Et | Me |
| A-162 | H | Cl | H | CN | H | Et | Et |
| A-163 | H | Br | H | Br | H | Me | H |
| A-164 | H | Br | H | Br | H | Me | Me |
| A-165 | H | Br | H | Br | H | Me | Et |
| A-166 | H | Br | H | Br | H | Et | H |
| A-167 | H | Br | H | Br | H | Et | Me |
| A-168 | H | Br | H | Br | H | Et | Et |
| A-169 | H | Br | H | Me | H | Me | H |
| A-170 | H | Br | H | Me | H | Me | Me |
| A-171 | H | Br | H | Me | H | Me | Et |
| A-172 | H | Br | H | Me | H | Et | H |
| A-173 | H | Br | H | Me | H | Et | Me |
| A-174 | H | Br | H | Me | H | Et | Et |
| A-175 | H | Br | H | Et | H | Me | H |
| A-176 | H | Br | H | Et | H | Me | Me |
| A-177 | H | Br | H | Et | H | Me | Et |
| A-178 | H | Br | H | Et | H | Et | H |
| A-179 | H | Br | H | Et | H | Et | Me |
| A-180 | H | Br | H | Et | H | Et | Et |
| A-181 | H | Br | H | OMe | H | Me | H |
| A-182 | H | Br | H | OMe | H | Me | Me |
| A-183 | H | Br | H | OMe | H | Me | Et |
| A-184 | H | Br | H | OMe | H | Et | H |
| A-185 | H | Br | H | OMe | H | Et | Me |
| A-186 | H | Br | H | OMe | H | Et | Et |
| A-187 | H | Br | H | OCF₃ | H | Me | H |
| A-188 | H | Br | H | OCF₃ | H | Me | Me |
| A-189 | H | Br | H | OCF₃ | H | Me | Et |
| A-190 | H | Br | H | OCF₃ | H | Et | H |
| A-191 | H | Br | H | OCF₃ | H | Et | Me |
| A-192 | H | Br | H | OCF₃ | H | Et | Et |
| A-193 | H | Br | H | CF₃ | H | Me | H |
| A-194 | H | Br | H | CF₃ | H | Me | Me |
| A-195 | H | Br | H | CF₃ | H | Me | Et |
| A-196 | H | Br | H | CF₃ | H | Et | H |
| A-197 | H | Br | H | CF₃ | H | Et | Me |
| A-198 | H | Br | H | CF₃ | H | Et | Et |
| A-199 | H | Br | H | CN | H | Me | H |
| A-200 | H | Br | H | CN | H | Me | Me |
| A-201 | H | Br | H | CN | H | Me | Et |
| A-202 | H | Br | H | CN | H | Et | H |
| A-203 | H | Br | H | CN | H | Et | Me |
| A-204 | H | Br | H | CN | H | Et | Et |
| A-205 | H | Me | H | Me | H | Me | H |
| A-206 | H | Me | H | Me | H | Me | Me |
| A-207 | H | Me | H | Me | H | Me | Et |
| A-208 | H | Me | H | Me | H | Et | H |
| A-209 | H | Me | H | Me | H | Et | Me |
| A-210 | H | Me | H | Me | H | Et | Et |
| A-211 | H | Me | H | Et | H | Me | H |
| A-212 | H | Me | H | Et | H | Me | Me |
| A-213 | H | Me | H | Et | H | Me | Et |
| A-214 | H | Me | H | Et | H | Et | H |
| A-215 | H | Me | H | Et | H | Et | Me |
| A-216 | H | Me | H | Et | H | Et | Et |
| A-217 | H | Me | H | OMe | H | Me | H |
| A-218 | H | Me | H | OMe | H | Me | Me |
| A-219 | H | Me | H | OMe | H | Me | Et |
| A-220 | H | Me | H | OMe | H | Et | H |
| A-221 | H | Me | H | OMe | H | Et | Me |
| A-222 | H | Me | H | OMe | H | Et | Et |
| A-223 | H | Me | H | OCF₃ | H | Me | H |
| A-224 | H | Me | H | OCF₃ | H | Me | Me |
| A-225 | H | Me | H | OCF₃ | H | Me | Et |
| A-226 | H | Me | H | OCF₃ | H | Et | H |
| A-227 | H | Me | H | OCF₃ | H | Et | Me |
| A-228 | H | Me | H | OCF₃ | H | Et | Et |
| A-229 | H | Me | H | CF₃ | H | Me | H |
| A-230 | H | Me | H | CF₃ | H | Me | Me |
| A-231 | H | Me | H | CF₃ | H | Me | Et |
| A-232 | H | Me | H | CF₃ | H | Et | H |
| A-233 | H | Me | H | CF₃ | H | Et | Me |
| A-234 | H | Me | H | CF₃ | H | Et | Et |
| A-235 | H | Me | H | CN | H | Me | H |
| A-236 | H | Me | H | CN | H | Me | Me |
| A-237 | H | Me | H | CN | H | Me | Et |
| A-238 | H | Me | H | CN | H | Et | H |
| A-239 | H | Me | H | CN | H | Et | Me |
| A-240 | H | Me | H | CN | H | Et | Et |
| A-241 | H | Et | H | Et | H | Me | H |
| A-242 | H | Et | H | Et | H | Me | Me |
| A-243 | H | Et | H | Et | H | Me | Et |
| A-244 | H | Et | H | Et | H | Et | H |
| A-245 | H | Et | H | Et | H | Et | Me |
| A-246 | H | Et | H | Et | H | Et | Et |
| A-247 | H | Et | H | OMe | H | Me | H |
| A-248 | H | Et | H | OMe | H | Me | Me |
| A-249 | H | Et | H | OMe | H | Me | Et |
| A-250 | H | Et | H | OMe | H | Et | H |
| A-251 | H | Et | H | OMe | H | Et | Me |
| A-252 | H | Et | H | OMe | H | Et | Et |
| A-253 | H | Et | H | OCF₃ | H | Me | H |
| A-254 | H | Et | H | OCF₃ | H | Me | Me |
| A-255 | H | Et | H | OCF₃ | H | Me | Et |
| A-256 | H | Et | H | OCF₃ | H | Et | H |
| A-257 | H | Et | H | OCF₃ | H | Et | Me |
| A-258 | H | Et | H | OCF₃ | H | Et | Et |
| A-259 | H | Et | H | CF₃ | H | Me | H |
| A-260 | H | Et | H | CF₃ | H | Me | Me |
| A-261 | H | Et | H | CF₃ | H | Me | Et |
| A-262 | H | Et | H | CF₃ | H | Et | H |
| A-263 | H | Et | H | CF₃ | H | Et | Me |
| A-264 | H | Et | H | CF₃ | H | Et | Et |
| A-265 | H | Et | H | CN | H | Me | H |
| A-266 | H | Et | H | CN | H | Me | Me |
| A-267 | H | Et | H | CN | H | Me | Et |
| A-268 | H | Et | H | CN | H | Et | H |
| A-269 | H | Et | H | CN | H | Et | Me |
| A-270 | H | Et | H | CN | H | Et | Et |
| A-271 | H | OMe | H | OMe | H | Me | H |
| A-272 | H | OMe | H | OMe | H | Me | Me |
| A-273 | H | OMe | H | OMe | H | Me | Et |
| A-274 | H | OMe | H | OMe | H | Et | H |
| A-275 | H | OMe | H | OMe | H | Et | Me |
| A-276 | H | OMe | H | OMe | H | Et | Et |
| A-277 | H | OMe | H | OCF₃ | H | Me | H |
| A-278 | H | OMe | H | OCF₃ | H | Me | Me |
| A-279 | H | OMe | H | OCF₃ | H | Me | Et |
| A-280 | H | OMe | H | OCF₃ | H | Et | H |
| A-281 | H | OMe | H | OCF₃ | H | Et | Me |
| A-282 | H | OMe | H | OCF₃ | H | Et | Et |
| A-283 | H | OMe | H | CF₃ | H | Me | H |
| A-284 | H | OMe | H | CF₃ | H | Me | Me |
| A-285 | H | OMe | H | CF₃ | H | Me | Et |
| A-286 | H | OMe | H | CF₃ | H | Et | H |
| A-287 | H | OMe | H | CF₃ | H | Et | Me |
| A-288 | H | OMe | H | CF₃ | H | Et | Et |
| A-289 | H | OMe | H | CN | H | Me | H |
| A-290 | H | OMe | H | CN | H | Me | Me |
| A-291 | H | OMe | H | CN | H | Me | Et |
| A-292 | H | OMe | H | CN | H | Et | H |
| A-293 | H | OMe | H | CN | H | Et | Me |
| A-294 | H | OMe | H | CN | H | Et | Et |
| A-295 | H | OCF₃ | H | OCF₃ | H | Me | H |
| A-296 | H | OCF₃ | H | OCF₃ | H | Me | Me |
| A-297 | H | OCF₃ | H | OCF₃ | H | Me | Et |
| A-298 | H | OCF₃ | H | OCF₃ | H | Et | H |
| A-299 | H | OCF₃ | H | OCF₃ | H | Et | Me |
| A-300 | H | OCF₃ | H | OCF₃ | H | Et | Et |
| A-301 | H | OCF₃ | H | CF₃ | H | Me | H |
| A-302 | H | OCF₃ | H | CF₃ | H | Me | Me |
| A-303 | H | OCF₃ | H | CF₃ | H | Me | Et |
| A-304 | H | OCF₃ | H | CF₃ | H | Et | H |
| A-305 | H | OCF₃ | H | CF₃ | H | Et | Me |
| A-306 | H | OCF₃ | H | CF₃ | H | Et | Et |
| A-307 | H | OCF₃ | H | CN | H | Me | H |
| A-308 | H | OCF₃ | H | CN | H | Me | Me |
| A-309 | H | OCF₃ | H | CN | H | Me | Et |
| A-310 | H | OCF₃ | H | CN | H | Et | H |
| A-311 | H | OCF₃ | H | CN | H | Et | Me |
| A-312 | H | OCF₃ | H | CN | H | Et | Et |
| A-313 | H | CF₃ | H | CF₃ | H | Me | H |
| A-314 | H | CF₃ | H | CF₃ | H | Me | Me |
| A-315 | H | CF₃ | H | CF₃ | H | Me | Et |
| A-316 | H | CF₃ | H | CF₃ | H | Et | H |
| A-317 | H | CF₃ | H | CF₃ | H | Et | Me |
| A-318 | H | CF₃ | H | CF₃ | H | Et | Et |
| A-319 | H | CF₃ | H | CN | H | Me | H |
| A-320 | H | CF₃ | H | CN | H | Me | Me |
| A-321 | H | CF₃ | H | CN | H | Me | Et |
| A-322 | H | CF₃ | H | CN | H | Et | H |
| A-323 | H | CF₃ | H | CN | H | Et | Me |
| A-324 | H | CF₃ | H | CN | H | Et | Et |
| A-325 | H | CN | H | CN | H | Me | H |
| A-326 | H | CN | H | CN | H | Me | Me |
| A-327 | H | CN | H | CN | H | Me | Et |
| A-328 | H | CN | H | CN | H | Et | H |
| A-329 | H | CN | H | CN | H | Et | Me |
| A-330 | H | CN | H | CN | H | Et | Et |
| A-331 | H | H | Et | H | H | Me | H |
| A-332 | H | H | Et | H | H | Me | Me |
| A-333 | H | H | Et | H | H | Me | Et |
| A-334 | Cl | Cl | H | H | Cl | Me | H |
| A-335 | Cl | Cl | H | H | Cl | Me | Me |
| A-336 | Cl | Cl | H | H | Cl | Me | Et |

**Analytische Daten Tabelle A:**

| Nr. | NMR |
|---|---|
| A-1 | [CDCl₃] 3.50 (s,3H); 3.50-3.55 (d,1H), 3.90 (d,1H), 7.45 (m,3H); 7.68 (m,2H). |
| A-2 | [CDCl₃] 3.48 (d,1H); 3.48 (s,3H); 3.83 (d,1H); 3.89 (s,3H); 7.42 (m,3H); 7.68 (m,2H). |
| A-7 | [CDCl₃] 3.49 (d,1H); 3.51 (s,3H); 3.90 (d,1H); 7.18 (m,1H); 7.42-7.51 (m,2H); 7.60 (m,1H). |
| A-8 | [CDCl₃] 3.45 (d,1H), 3.48 (s,3H); 3.80 (d,1H); 3.89 (s,3H); 7.16 (m,1H); 7.37-7.43 (m,3H). |
| A-10 | [CDCl₃] 1.27 (m,3H); 3.50 (d,1H), 3.77 (q,2H); 3.88 (d,1H), 5.74 (s br, 1H); 7.16 (m,1H), 7.40 (m,2H); 7.60 (m,1H). |
| A-12 | [CDCl₃] 1.23 (t,3H); 1.36 (t,3H); 3.42 (d,1H); 3.70-3.85 (m,3H); 4.34 (m,2H); 7.13 (m,1H); 7.40 (m,3H). |
| A-13 | [CDCl₃] 3.49 (d,1H); 3.50 (s,3H), 3.89 (d,1H); 7.39 (t,1H); 7.42 (d,1H); 7.56 (d,1H); 7.69 (s,1H). |
| A-14 | [CDCl₃] 3.43 (d,1H); 3.48 (s,3H); 3.80 (d,1H); 3.89 (s,3H); 7.37 (t,1H); 7.41 (m,1H); 7.54 (m,1H); 7.67 (m,1H). |
| A-25 | [CDCl₃] 2.39 (s,3H); 3.50 (s,3H); 3.54 (d,1H); 3.88 (d,1H); 7.30-7.68 (m,4H). |
| A-26 | [CDCl₃] 2.38 (s,3H); 3.47 (s,3H); 3.44-3.49 (d,1H); 3.83 (d,1H); 3.89 (s,3H); 7.25-7.33 (m,2H), 7.45 (d,1H); 7.51 (s,1H). |
| A-30 | [CDCl₃] 1.22 (t,3H); 1.35 (t,3H); 2.39 (s,3H); 3.49 (d,1H); 3.70-3.81 (m,3H); 4.33 (m,2H); 7.24-7.32 (m,2H); 7.45 (d,1H); 7.52 (s,1H). |
| A-31 | [CDCl₃] 1.25 (m,3H); 2.69 (q,2H), 3.49 (s,3H); 3.50 (d,1H); 3.90 (d,1H), 7.30 (d,1H); 7.33 (t,1H); 7.46 (d,1H); 7.54 (s,1H). |
| A-32 | [CDCl₃] 1.25 (t,3H); 2.68 (q,2H); 3.45-3.50 (d,1H), 3.47 (s,3H); 3.83 (d,1H); 3.88 (s,3H); 7.26-7.35 (m,2H); 7.45 (d,1H); 7.54 (s,1H). |
| A-36 | [CDCl₃] 1.23 (m,6H); 1.35 (t,3H); 2.68 (q,2H); 3.49 (d,1H); 3.70-3.85 (m,3H); 4.43 (m,2H); 7.29-7.35 (m,2H); 7.45 (d,1H); 7.55 (s,1H). |
| A-37 | [CDCl₃] 3.50 (s,3H); 3.51 (d,1H); 3.83 (s,3H); 3.90 (d,1H); 7.01 (d,1H); 7.19 (d,1H); 7.28 (s,1H); 7.33 (t,1H). |
| A-38 | [CDCl₃] 3.44 (d,1H); 3.48 (s,3H); 3.79-3.87 (d,1H); 3.84 (s,3H); 3.89 (s,3H); 6.99 (d,1H); 7.17 (d,1H); 7.26-7.34 (m,2H). |
| A-42 | [CDCl₃] 1.23 (t,3H); 1.36 (t,3H), 3.44 (d,1H); 3.70-3.80 (m,3H); 3.82 (s,3H); 4.32 (m,2H); 6.99 (d,1H); 7.19 (d,1H); 7.28-7.34 (m,2H). |
| A-62 | [CDCl₃] 3.40 (d,1H); 3.48 (s,3H); 3.78 (d,1H); 3.89 (s,3H); 6.90 (t,1H); 7.20 (d,2H). |
| A-61 | [CDCl₃] 3.45 (d,1H); 3.51 (s,3H); 3.86 (d,1H); 6.91 (m,1H); 7.21 (m,2H). |
| A-64 | [CDCl₃] 1.27 (t,3H); 3.45 (d,1H), 3.75 (q,2H); 3.83 (d,1H); 6.90 (m,1H); 7.20 (m,2H). |
| A-66 | [CDCl₃] 1.23 (t,3H); 1.34 (t,3H); 3.40 (d,1H); 3.70-3.77 (m,2H); 3.82 (m,1H); 4.35 (m,2H); 6.89 (m,1H); 7.20 (m,2H). |
| A-79 | [CDCl₃] 2.39 (s,3H); 3.46 (d,1H); 3.49 (s,3H); 3.88 (d,1H); 6.99 (d,1H), 7.20 (d,1H); 7.25 (s,1H). |
| A-80 | [CDCl₃] 2.38 (s,3H); 3.43 (d,1H), 3.47 (s,3H); 3.80 (d,1H), 3.90 (s,3H); 6.97 (d,1H); 7.19 (d,1H); 7.26 (s,1H). |
| A-84 | [CDCl₃] 1.22 (t,3H); 1.35 (t,3H); 2.39 (s,3H); 3.42 (d,1H); 3.72 (m,2H); 3.80 (quin,1H); 4.33 (m,2H); 6.97 (d,1H); 7.20 (d,1H), 7.26 (s,1H). |
| A-91 | [CDCl₃] 3.47 (d,1H); 3.50 (s,3H); 3.83 (s,3H); 3.85 (d,1H); 6.71 (d,1H); 6.96 (d,1H); 7.02 (s,1H). |
| A-92 | [CDCl₃] 3.40 (d,1H); 3.48 (s,3H); 3.78 (d,1H), 3.83 (s,3H); 3.89 (s,3H); 6.70 (d,1H); 6.94 (d,1H); 7.02 (s,1H). |
| A-103 | [CDCl₃] 3.46-3.51 (d,1H); 3.51 (s,3H); 3.92 (d,1H); 7.42 (d,1H); 7.61 (d,1H); 7.69 (s,1H). |
| A-104 | [CDCl₃] 3.45 (d,1H); 3.50 (s,3H); 3.83 (d,1H); 3.90 (s,3H); 7.40 (d,1H); 7.60 (d,1H), 7.68 (s,1H). |
| A-115 | [CDCl₃] 3.35 (d,1H); 3.41 (s,3H); 3.78 (d,1H); 7.38 (s,1H); 7.48 (s,2H). |
| A-116 | [CDCl₃] 3.40 (d,1H); 3.48 (s,3H); 3.78 (d,1H); 3.89 (s,3H); 7.43 (s,1H); 7.55 (s,2H). |
| A-118 | [CDCl₃] 1.27 (t,3H), 3.45 (d,1H); 3.75 (q,2H); 3.85 (d,1H); 7.45 (s,1H); 7.56 (s,2H). |
| A-120 | [CDCl₃] 1.23 (t,3H); 1.37 (t,3H); 3.40 (d,1H); 3.70-3.82 (m,3H); 4.35 (m,2H); 7.43 (s,1H); 7.56 (s,2H). |
| A-145 | [CDCl₃] 3.45 (d,1H); 3.50 (s,3H); 3.87 (d,1H), 7.33 (s,1H), 7.46 (s,1H), 7.59 (s,1H). |
| A-146 | [CDCl₃] 3.41 (d,1H); 3.49 (s,3H); 3.80 (d,1H); 3.89 (s,3H); 7.31 (s,1H); 7.45 (s,1H); 7.58 (s,1H). |
| A-169 | [CDCl₃] 2.37 (s,3H), 3.47 (d,1H), 3.48 (s,3H); 3.87 (d,1H); 7.43 (s, 2H); 7.61 (s,1H). |
| A-170 | [CDCl₃] 2.36 (s,3H); 3.41 (d,1H), 3.47 (s,3H), 3.79 (d,1H); 3.89 (s,3H); 7.42 (d,2H); 7.60 (s,1H). |
| A-187 | [CDCl₃] 3.45 (d,1H); 3.50 (s,3H); 3.87 (d,1H); 7.49 (d,2H); 7.73 (s,1H). |
| A-188 | [CDCl₃] 3.41 (d,1H); 3.49 (s,3H); 3.79 (d,1H); 3.89 (s,3H); 7.47 (s,1H); 7.50 (s,1H); 7.73 (s,1H). |
| A-193 | [CDCl₃] 3.47 (d,1H), 3.50 (s,3H); 3.90 (d,1H); 7.85 (s,2H); 8.02 (s,1H). |
| A-194 | [CDCl₃] 3.45 (d,1H); 3.49 (s,3H); 3.81 (d,1H); 3.90 (s,3H); 7.83 (m,2H); 8.01 (s,1H). |
| A-331 | [CDCl₃] 1.25 (t,3H); 2.69 (q,2H); 3.49 (s,3H); 3.51 (d,1H); 3.89 (d,1H); 7.26 (d,2H); 7.59 (d,2H); 8.0 (s br,1H). |
| A-332 | [CDCl₃] 1.25 (t,3H); 2.68 (q,2H); 3.36 (d,1H); 3.47 (s,3H); 3.82 (d,1H); 3.88 (s,3H); 7.25 (d,2H); 7.58 (d,2H). |
| A-334 | [CDCl₃] 3.39 (d,1H); 3.61 (s,3H); 3.91 (d,1H); 7.36 (d,1H); 7.51 (d,1H). |
| A-335 | [CDCl₃] 3.35 (d,1H); 3.56 (s,3H); 3.82 (d,1H); 3.91 (s,3H); 7.34 (d,1H); 7.50 (d,1H). |

### B. Formulierungsbeispiele

### 1. Stäubemittel

Ein Stäubemittel wird erhalten, indem man 10 Gew.-Teile einer Verbindung der Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der Formel (I), 6 Gew.-Teile

Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man
75 Gew.-Teile einer Verbindung der Formel (I),
10 " ligninsulfonsaures Calcium,
5 " Natriumlaurylsulfat,
3 " Polyvinylalkohol und
7 " Kaolin
mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man 25 Gew.-Teile einer Verbindung der Formel (I),
5 " 2,2'-dinaphthylmethan-6,6'-disulfonsaures Natrium,
2 " oleoylmethyltaurinsaures Natrium,
1 " Polyvinylalkohol,
17 " Calciumcarbonat und
50 " Wasser
auf einer Kolloidmühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen beziehungsweise Rhizomstücke Mono- und dikotyler Schadpflanzen werden in Töpfen von 9 bis 13 cm Durchmesser in sandiger Lehmerde ausgelegt und mit Erde bedeckt. Die als emulgierbare Konzentrate oder Stäubemittel formulierten Herbizide werden in Form wäßriger Dispersionen oder Suspensionen bzw. Emulsionen mit einer Wasseraufwandmenge von umgerechnet 300 bis 8001/ha in unterschiedlichen Dosierungen auf die Oberfläche der Abdeckerde appliziert. Anschließend werden die Töpfe zur weiteren Kultivierung der Pflanzen im Gewächshaus unter optimalen Bedingungen gehalten. Nach 3 bis 4 Wochen Standzeit der Versuchspflanzen im Gewächshaus unter optimalen Wachstumsbedingungen wird die Wirkung der erfindungsgemäßen Verbindungen durch optische Bonitur ermittelt. So zeigen beispielsweise die Verbindungen der Nr. 1.2-7, 1.2-11, 1.2-42, 1.2-46, 1.2-62, 1.2-148, 1.2-202, 1.3-206, 1.3-110, 1.5-212, 1.6-206, 1.7-9, 1.11-9, 1.11-11, 1.11-16, 1.11-21, 1.11-40, 1.11-41 D1, 1.11-42, 1.11-46 D2, 1.11-147, 1.11-48 D 1, 1.11-48 D2, 1.11-53, 1.11-59, 1.11-60, 1.11-110, 1.11-119, 1.11-136, 1.11-137, 1.11-142, 1.11-150, 1.11-197, 1.11-199, 1.11-200, 1.11-201, 1.11-202, 1.11-210, 1.11-212, 1.11-229, 1.14-16, 1.14-212, 1.18-9, 1.18-16, 1.18-96, 1.18-212, 1.20-7, 1.20-19 D2, 1.20-20, 1.20-21, 1.20-42, 1.20-48 D2, 1.20-55, 1.20-74, 1.20-200, 1.20-208, 1.25-206, 1.29-73, 1.29-212, 1.32-206, 1.33-212 und 2.11-212 bei einer Aufwandmenge von 320 Gramm pro Hektar jeweils eine mindestens 90%-ige Wirkung gegen Echinochloa crus galli, Lolium multiflorum, Stellaria media und Veronica persica.

### 2. Herbizide Wirkung gegen Schadpflanzen im Nachauflauf

Samen von mono- und dikotylen Schadpflanzen werden in Papptöpfen in sandigem Lehmboden ausgelegt, mit Erde abgedeckt und im Gewächshaus unter guten Wachstumsbedingungen angezogen. Zwei bis drei Wochen nach der Aussaat werden die Versuchspflanzen im Dreiblattstudium behandelt. Die als Spritzpulver bzw. als Emulsionskonzentrate formulierten erfindungsgemäßen Verbindungen werden mit einer Wasseraufwandmenge von umgerechnet 600 bis 800 l/ha auf die Oberfläche der grünen Pflanzenteile gesprüht. Nach 3 bis 4 Wochen Standzeit der Versuchspflanzen im Gewächshaus unter optimalen Wachstumsbedingungen wird die Wirkung der erfindungsgemäßen Verbindungen durch optische Bonitur ermittelt. So zeigen beispielsweise die Verbindungen der Nr.1.2-11, 1.2-53, 1.20-93, 1.2-102, 1.2-138, 1.2-200, 1.3-9, 1.3-206, 1.6-206, 1.7-96, 1.11-11, 1.11-20, 1.11-40, 1.11-53, 1.11-60, 1.11-93, 1.11-110, 1.11-119, 1.11-200, 1.11-212, 1.11-137, 1.11-142, 1.11-149, 1.14-96, 1.14-136, 1.16-110, 1.18-96, 1.18-206, 1.20-16, 1.20-40, 1.20-58, 1.20-200, 1.20-208, 1.25-102, 1.25-206, 1.29-73, 1.32-96, 1.33-102, 2.2-102, 2.11-96 und 2.11-212 bei einer Aufwandmenge von 320 Gramm pro Hektar jeweils eine mindestens 90%-ige Wirkung gegen Alopecurus myosuroides und Polygonum convolvulus.

### 3. Fungizide Wirkung

### Beispiel: In vivo protektiv Test Sphaerotheca fuliginea (Echter Mehltau an Gurke)

Zur Herstellung einer zweckmäßigen Wirkstoffzubereitung vermischt man Wirkstoff mit Aceton/Tween/ DMSO und verdünnt mit Wasser auf die entsprechende Konzentration.

Gurkenpflanzen (Sorte: Vert petit de Paris) werden in 50/50 Torf Puzzolan-Substrat bei 24 °C angezogen und im Z11 Kotyledonenstadium mit der oben angegebenen Wirkstoffzubereitung besprüht. Pflanzen, welche der Kontrolle dienen, werden mit einer wässrigen Lösung ohne Wirkstoff behandelt.

Nach 24 Stunden werden die Pflanzen mit einer Sporensuspension von Sphaerotheca fuliginea (100 000 Sporen / ml) besprüht. Die Sporen werden von infizierten Pflanzen eingesammelt. Die inokulierten Pflanzen werden bei 20°C / 25°C und bei 60/70 % relativer Feuchtigkeit aufgestellt.

12 Tage nach der Inokulation wird die Auswertung durchgeführt. Dabei bedeutet 0% ein Wirkungsgrad, der demjenigen der Kontrolle entspricht, während 100% bedeutet, dass kein Befall beobachtet wird.

Bei einer Aufwandmenge von 500 ppm werden folgende Wirkungsgrade mit den folgenden Verbindungen festgestellt:

| Beispiel Nr. | Wirkung [%] | | Beispiel Nr. | Wirkung [%] |
|---|---|---|---|---|
| 1.3-206 | 94 | | 1.33-102 | 100 |
| 1.5-206 | 80 | | 1.32-60 | 100 |
| 1.6-206 | 94 | | 1.32-96 | 100 |
| 1.7-9 | 98 | | 1.20-19 D2 | 100 |
| 1.7-206 | 72 | | 1.20-64 | 98 |
| 1.11-19 D1 | 100 | | 1.20-206 | 100 |
| 1.11-110 | 78 | | 1.20-212 | 100 |
| 1.11-238 | 83 | | 1.20-135 | 98 |
| 1.14-16 | 100 | | 1.25-9 | 100 |
| 1.14-206 | 98 | | 1.25-16 | 95 |
| 1.16-60 | 94 | | 1.25-60 | 100 |
| 1.16-96 | 78 | | 1.25-110 | 90 |
| 1.16-110 | 89 | | 1.25-136 | 100 |
| 1.16-136 | 78 | | 1.32-9 | 100 |
| 1.16-212 | 94 | | 1.32-16 | 100 |
| 1.18-110 | 98 | | 1.32-110 | 100 |
| 1.18-212 | 98 | | 1.32-212 | 100 |
| 1.18-206 | 95 | | 1.33-9 | 100 |
| 1.33-60 | 100 | | 1.33-16 | 100 |
| 1.33-96 | 100 | | 1.33-206 | 100 |
| 1.33-212 | 100 | | | |

### Beispiel 2: In vivo protektiv Test Phytophthora infestans (späte Hartfäule an Tomate)

Zur Herstellung einer zweckmäßigen Wirkstoffzubereitung vermischt man Wirkstoff mit Acetone/Tween/ DMSO und verdünnt mit Wasser auf die entsprechende Konzentration.

Tomatenpflanzen (Sorte Rentita) werden in 50/50 Torf Puzzolan-Substrat bei 20-25°C angezogen und im Stadium Z12 mit der oben angegebenen Wirkstoffzubereitung besprüht. Pflanzen, welche der Kontrolle dienen, werden mit einer wässrigen Lösung ohne Wirkstoff behandelt.

Nach 24 Stunden werden die Pflanzen mit einer Sporensuspension von Phytophthora infestans (20 000 Sporen / ml) besprüht. Die Sporen werden von infizierten Pflanzen eingesammelt. Die inokulierten Pflanzen werden für 5 Tage bei 16-18 °C und in einer feuchten Atmosphäre aufgestellt.

5 Tage nach der Infektion wird die Auswertung durchgeführt. Dabei bedeutet 0 % ein Wirkungsgrad, der demjenigen der Kontrolle entspricht, während ein Wirkungsgrad von 100% bedeutet, dass kein Befall beobachtet wird.

Bei einer Aufwandmenge von 500 ppm werden folgende Wirkungsgrade mit den folgenden Verbindungen festgestellt:

| Beispiel Nr. | Wirkung [%] | | Beispiel Nr. | Wirkung [%] |
|---|---|---|---|---|
| 1.11-19 D1 | 100 | | 1.18-212 | 99 |
| 1.11-110 | 100 | | 1.20-19 D2 | 100 |
| 1.11-136 | 100 | | 1.20-64 | 100 |
| 1.11-238 | 99 | | 1.20-206 | 100 |
| 1.11-239 | 100 | | 1.20-212 | 95 |
| 1.11-189 | 100 | | 1.25-212 | 100 |
| 1.14-110 | 100 | | 1.33-212 | 97 |
| 1.14-206 | 100 | | 2.11-206 | 100 |
| 1.16-212 | 100 | | 2.11-212 | 98 |
| 1.18-206 | 90 | | 2.20-206 | 85 |

### Beispiel 3: In vivo protektiv Test Botrytis cinerea (Grauschimmel an Gurken)

Zur Herstellung einer zweckmäßigen Wirkstoffzubereitung vermischt man Wirkstoff mit Acetone/Tween/ DMSO und verdünnt mit Wasser auf die entsprechende Konzentration.

Gurkenpflanzen (Vert petit de Paris) werden in 50/50 Torf Puzzolan-Substrat bei 24°C angezogen und im Stadium Z11 mit der oben angegebenen Wirkstoffzubereitung besprüht. Pflanzen, welche der Kontrolle dienen, werden mit einer wässrigen Lösung ohne Wirkstoff behandelt.

Nach 24 Stunden werden die Pflanzen mit einer Sporensuspension von Botrytis cinerea (50 000 Sporen / ml) besprüht. Die Sporen werden in einer Nährstofflösung bestehend aus 10 g/L PDB, 50 g/L D-Fructose, 2 g/L NH₄NO₃ und 1 g/L KH₂PO₄ suspendiert.

Die inokulierten Gurkenpflanzen werden bei 17°C und bei 80% relativer Luftfeuchte inkubiert.

4-5 Tage nach der Infektion wird die Auswertung durchgeführt. Dabei bedeutet 0% ein Wirkungsgrad, der demjenigen der Kontrolle entspricht, während ein Wirkungsgrad von 100% bedeutet, dass kein Befall beobachtet wird.

Bei einer Aufwandmenge von 500 ppm werden folgende Wirkungsgrade mit den folgenden Verbindungen festgestellt:

| Beispiel Nr. | Wirkung [%] | | Beispiel Nr. | Wirkung [%] |
|---|---|---|---|---|
| 1.3-110 | 98 | | 1.20-110 | 83 |
| 1.5-206 | 100 | | 1.20-135 | 100 |
| 1.6-96 | 95 | | 1.20-206 | 100 |
| 1.6-110 | 100 | | 1.20-212 | 100 |
| 1.6-206 | 100 | | 1.25-9 | 99 |
| 1.7-9 | 95 | | 1.25-16 | 100 |
| 1.7-110 | 100 | | 1.25-60 | 100 |
| 1.7-206 | 99 | | 1.25-110 | 98 |
| 1.11-19 D1 | 100 | | 1.25-136 | 79 |
| 1.11-110 | 90 | | 1.25-206 | 100 |
| 1.11-238 | 100 | | 1.25-212 | 97 |
| 1.11-239 | 98 | | 1.32-9 | 100 |
| 1.14-16 | 99 | | 1.32-16 | 100 |
| 1.14-110 | 100 | | 1.32-60 | 100 |
| 1.14-136 | 88 | | 1.32-94 | 95 |
| 1.14-206 | 100 | | 1.32-110 | 100 |
| 1.16-60 | 100 | | 1.32-102 | 100 |
| 1.16-94 | 75 | | 1.32-206 | 96 |
| 1.16-96 | 85 | | 1.32-212 | 100 |
| 1.16-110 | 100 | | 1.33-9 | 89 |
| 1.16-136 | 96 | | 1.33-16 | 100 |
| 1.16-212 | 100 | | 1.33-60 | 100 |
| 1.18-110 | 91 | | 1.33-96 | 100 |
| 1.18-206 | 100 | | 1.33-110 | 81 |
| 1.18-212 | 100 | | 1.33-206 | 100 |
| 1.20-19 D2 | 100 | | 1.33-212 | 100 |
| 1.20-64 | 70 | | | |

### Beispiel 4: In vivo protektiv Test Septoria tritici (Blattflecken an Weizen)

Zur Herstellung einer zweckmäßigen Wirkstoffzubereitung vermischt man Wirkstoff mit Acetone/Tween/ DMSO und verdünnt mit Wasser auf die entsprechende Konzentration.

Weizenpflanzen (Sorte: Scipion) werden in 50/50 Torf Puzzolan-Substrat bei 22°C (12h) / 20°C (12h) angezogen und im 1-Blatt Stadium (10 cm Höhe) mit der oben angegebenen Wirkstoffzubereitung besprüht. Kontrollpflanzen werden mit einer wässrigen Lösung ohne Wirkstoffzusatz behandelt.

Nach 24 Stunden werden die Pflanzen mit einer Sporensuspension von kryokonservierten Septoria tritici (500 000 Sporen / ml) besprüht. Die inokulierten Pflanzen werden 72 h bei 18°C und bei 100 % relativer Feuchtigkeit aufgestellt und anschließend bei 90 % relativer Luftfeuchtigkeit für weitere 21-28 Tage.

21-28 Tage nach der Inokulation wird die Auswertung im Vergleich mit den Kontrollpflanzen durchgeführt. Dabei bedeutet 0% ein Wirkungsgrad, der demjenigen der Kontrolle entspricht, während ein Wirkungsgrad von 100% bedeutet, dass kein Befall beobachtet wird.

Bei einer Aufwandmenge von 500 ppm werden folgende Wirkungsgrade mit den folgenden Verbindungen festgestellt:

| Beispiel Nr. | Wirkung [%] | | Beispiel Nr. | Wirkung [%] |
|---|---|---|---|---|
| 1.3-206 | 100 | | 2.11-206 | 83 |
| 1.3-110 | 98 | | 2.11-212 | 83 |
| 1.5-206 | 100 | | 2.20-206 | 100 |
| 1.6-96 | 75 | | 1.25-206 | 100 |
| 1.6-110 | 100 | | 1.25-60 | 100 |
| 1.6-206 | 100 | | 1.25-212 | 100 |
| 1.7-9 | 100 | | 1.25-9 | 100 |
| 1.7-110 | 100 | | 1.25-16 | 97 |
| 1.7-206 | 100 | | 1.25-110 | 97 |
| 1.11-19 D1 | 100 | | 1.32-1 | 98 |
| 1.11-110 | 100 | | 1.32-9 | 100 |
| 1.11-136 | 100 | | 1.32-16 | 100 |
| 1.11-238 | 100 | | 1.32-60 | 100 |
| 1.11-239 | 100 | | 1.32-94 | 100 |
| 1.14-16 | 100 | | 1.32-96 | 100 |
| 1.14-110 | 100 | | 1.32-102 | 100 |
| 1.14-206 | 100 | | 1.32-110 | 100 |
| 1.16-60 | 100 | | 1.32-206 | 100 |
| 1.16-96 | 88 | | 1.32-212 | 100 |
| 1.16-110 | 100 | | 1.33-1 | 100 |
| 1.16-212 | 100 | | 1.33-9 | 100 |
| 1.18-110 | 100 | | 1.33-16 | 100 |
| 1.18-212 | 100 | | 1.33-60 | 100 |
| 1.20-19 D2 | 100 | | 1.33-94 | 100 |
| 1.20-64 | 100 | | 1.33-96 | 100 |
| 1.20-110 | 100 | | 1.33-102 | 100 |
| 1.20-135 | 100 | | 1.33-110 | 100 |
| 1.20-206 | 83 | | 1.33-206 | 100 |
| 1.20-212 | 100 | | 1.33-212 | 100 |

### Beispiel 5: Venturia - Test (Apfel) / protektiv

| | |
|---|---|
| Lösungsmittel : | 24,5 Gewichtsteile Aceton |
| | 24,5 Gewichtsteile Dimethylacetamid |
| Emulgator: 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers Venturia inaequalis inokuliert und verbleiben dann 1 Tag bei ca. 20°C und 100% relativer Luftfeuchtigkeit in einer Inkubations-kabine.

Die Pflanzen werden dann im Gewächshaus bei ca. 21 °C und einer relativen Luftfeuchtigkeit von ca. 90% aufgestellt. 10 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die nachfolgenden erfindungsgemäßen Verbindungen bei einer Konzentration an Wirkstoff von 100ppm einen Wirkungsgrad von 70% oder mehr.

| Beispiel Nr. | Wirkung [%] |
|---|---|
| 1.11-212 | 100 |
| 1.20-5 | 96 |
| 1.25-206 | 90 |
| 1.25-212 | 98 |

### Beispiel 6: Plasmopara-Test (Rebe) / protektiv

| | | |
|---|---|---|
| Lösungsmittel : | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von Plasmopara viticola inokuliert und verbleiben dann 1 Tag in einer Inkubationskabine bei ca. 20°C und 100% relativer Luftfeuchtigkeit. Anschließend werden die Pflanzen 4 Tage im Gewächshaus bei ca. 21 °C und ca. 90% Luftfeuchtigkeit aufgestellt. Die Pflanzen werden dann angefeuchtet und 1 Tag in eine Inkubationskabine gestellt. 6 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die nachfolgenden erfindungsgemäßen Verbindungen bei einer Konzentration an Wirkstoff von 100ppm einen Wirkungsgrad von 70% oder mehr.

| Beispiel Nr. | Wirkung [%] |
|---|---|
| 1.20-5 | 99 |
| 1.25-206 | 98 |
| 1.25-212 | 100 |

## Patentansprüche

1. 5-Oxy-substituierte 3-Phenylisoxazolin-5-carboxamide und 5-oxy-substituierte 3-Phenylisoxazolin-5-thioamide der Formel (I), oder deren Salze 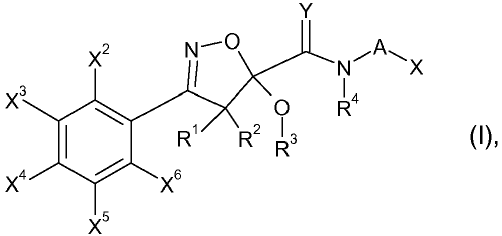 worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy,
oder
R¹ und R² bilden gemeinsam mit dem Kohlenstoffatom, an das sie gebunden sind, einen gesättigten, teilweise oder vollständig ungesättigten drei-, vier- oder fünfgliedrigen Ring, der aus q Kohlenstoffatomen und p Sauerstoffatomen aufgebaut ist;
R³ bedeutet durch jeweils m Reste aus der Gruppe bestehend aus Fluor, Chlor, Brom, Iod, Cyano, (C₁-C₄)-Alkoxy und Hydroxy substituiertes (C₁-C₆)-Alkyl, (C₃-C₆)-Cycloalkyl, (C₂-C₆)-Alkenyl oder (C₂-C₆)-Alkinyl;
R⁴ bedeutet Wasserstoff, Cyano,
oder durch jeweils m Reste aus der Gruppe bestehend aus Fluor, Chlor, Brom, Iod, Cyano, Hydroxy und (C₁-C₆)-Alkoxy substituiertes (C₁-C₈)-Al kyl, (C₃-C₈)-Cycloalkyl, (C₃-C₈)-Alkenyl oder (C₃-C₈)-Alkinyl;
A bedeutet eine Bindung oder eine divalente Einheit aus der Gruppe bestehend aus 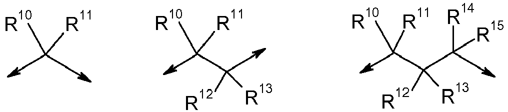 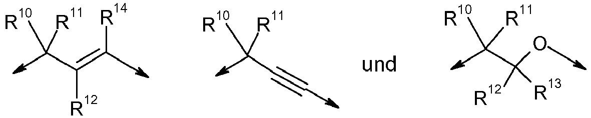
R¹⁰, R¹¹, R¹², R¹³, R¹⁴ und R¹⁵ bedeuten unabhängig voneinander jeweils Wasserstoff, Fluor, Chlor, Brom, Iod, Hydroxy, Cyano, CO₂R⁸, CONR⁶R⁸, R⁵,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
Y bedeutet Sauerstoff oder Schwefel;
X bedeutet Wasserstoff, Cyano, Hydroxy, X¹,
oder
durch jeweils m Reste aus der Gruppe bestehend aus Fluor, Chlor, Brom, Iod, Cyano, Hydroxy, OR⁷, X¹, OX¹, NHX¹, S(O)ₙR⁵, SO₂NR⁶R⁷, SO₂NR⁶COR⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ und C(R⁶)=NOR⁸ substituiertes (C₁-C₁₂)-Alkyl, (C₃-C₈)-Cycloalkyl, (C₂-C₁₂)-Alkenyl oder (C₂-C₁₂)-Alkinyl,
oder
X, A und R⁴ bilden mit dem Stickstoffatom, an das sie gebunden sind, einen gesättigten, teilweise oder vollständig ungesättigten fünf-, sechs- oder siebengliedrigen Ring, der neben diesem Stickstoffatom k Kohlenstoffatome, n Sauerstoffatome, p Schwefelatome und p Elemente aus der Gruppe bestehend aus NR⁷ und NCOR⁷ als Ringatome enthält, wobei ein Kohlenstoffatom p Oxogruppen trägt;
X¹ bedeutet einen drei, vier, fünf- oder sechsgliedrigen gesättigten, teilweise ungesättigten, vollständig ungesättigten oder aromatischen Ring, der aus r Kohlenstoffatomen, s Stickstoffatomen, n Schwefelatomen und n Sauerstoffatomen aufgebaut ist, und der durch s Reste aus der Gruppe bestehend aus R⁶, R^{6a}, R⁸ und R⁹ substituiert ist, wobei die Schwefelatome und Kohlenstoffatome n Oxogruppen tragen;
oder X¹ bedeutet durch m Reste aus der Gruppe bestehend aus R⁶, R^{6a}, R⁸ und R⁹ substituiertes Phenyl;
X², X⁴ und X⁶ bedeuten unabhängig voneinander jeweils Wasserstoff, Fluor, Chlor, Brom, Iod, Cyano, Nitro,
oder jeweils durch m Reste aus der Gruppe bestehend aus Fluor, Chlor, Brom Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Alkylcarbonyl;
X³ und X⁵ bedeuten unabhängig voneinander Wasserstoff, Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, S(O)ₙR⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₃-C₆)-Alkenyloxy oder (C₃-C₆)-Alkinyloxy;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷,
R⁹ bedeutet (C₁-C₃)-Alkyl oder (C₁-C₃)-Alkoxy,
k bedeutet 3, 4, 5 oder 6;
m bedeutet 0, 1, 2, 3, 4 oder 5;
n bedeutet 0, 1 oder 2;
p bedeutet 0 oder 1;
q bedeutet 3, 4 oder 5;
r bedeutet 1, 2, 3, 4 oder 5;
s bedeutet 0, 1, 2, 3 oder 4.

2. 5-Oxy-substituierte 3-Phenylisoxazolin-5-carboxamide und 5-oxy-substituierte 3-Phenylisoxazolin-5-thioamide nach Anspruch 1,
worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy,
oder
R¹ und R² bilden gemeinsam mit dem Kohlenstoffatom, an das sie gebunden sind, einen gesättigten, teilweise oder vollständig ungesättigten drei-, vier- oder fünfgliedrigen Ring, der aus q Kohlenstoffatomen und p Sauerstoffatomen aufgebaut ist;
R³ bedeutet durch jeweils m Reste aus der Gruppe bestehend aus Fluor, Chlor, Brom, Iod, Cyano, (C₁-C₄)-Alkoxy und Hydroxy substituiertes (C₁-C₆)-Alkyl, (C₃-C₆)-Cycloalkyl, (C₂-C₆)-Alkenyl oder (C₂-C₆)-Alkinyl,
R⁴ bedeutet Wasserstoff, Cyano,
oder durch jeweils m Reste aus der Gruppe bestehend aus Fluor, Chlor, Brom, Iod, Cyano, Hydroxy und (C₁-C₆)-Alkoxy substituiertes (C₁-C₈)-Alkyl oder (C₃-C₈)-Cycloalkyl;
A bedeutet eine Bindung oder eine divalente Einheit aus der Gruppe bestehend aus 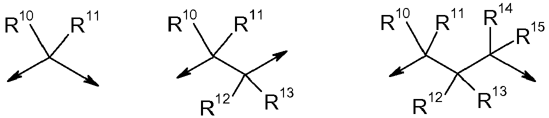 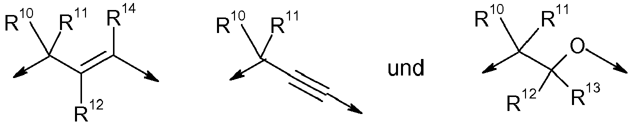
R¹⁰, R¹¹, R¹², R¹³, R¹⁴ und R¹⁵ bedeuten unabhängig voneinander jeweils Wasserstoff, Fluor, Chlor, Brom, Iod, Hydroxy, Cyano, CO₂R⁸, CONR⁶R⁸, R⁵, oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
Y bedeutet Sauerstoff oder Schwefel;
X bedeutet Wasserstoff, Cyano, Hydroxy, X¹,
oder
durch jeweils m Reste aus der Gruppe bestehend aus Fluor, Chlor, Brom, Iod, Cyano, Hydroxy, OR⁷, X¹, OX¹, NHX¹, S(O)ₙR⁵, SO₂NR⁶R⁷, SO₂NCOR⁶R⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ und C(R⁶)=NOR⁸ substituiertes (C₁-C₁₂)-Alkyl, (C₃-C₈)-Cycloalkyl, (C₂-C₁₂)-Alkenyl oder (C₂-C₁₂)-Alkinyl,
oder
X, A und R⁴ bilden mit dem Stickstoffatom, an das sie gebunden sind, einen gesättigten, teilweise oder vollständig ungesättigten fünf-, sechs- oder siebengliedrigen Ring, der neben diesem Stickstoffatom k Kohlenstoffatome, n Sauerstoffatome, p Schwefelatome und p Elemente aus der Gruppe bestehend aus NR⁷ und NCOR⁷ als Ringatome enthält, wobei ein Kohlenstoffatom p Oxogruppen trägt;
X¹ bedeutet einen durch s Reste aus der Gruppe bestehend aus R⁶, R^{6a}, R⁸ und
R⁹ substituierten Ring aus der Gruppe bestehend aus 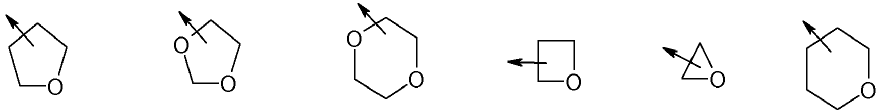 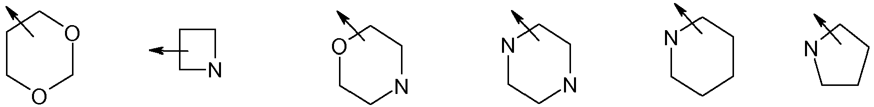 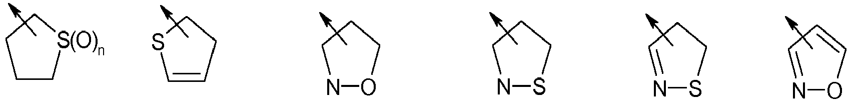 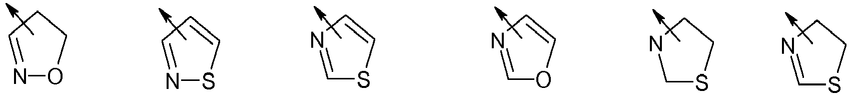 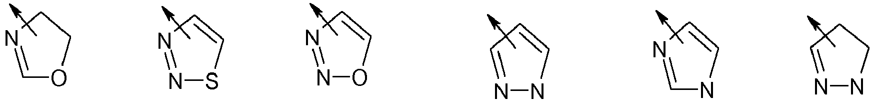 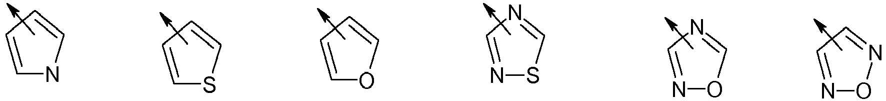 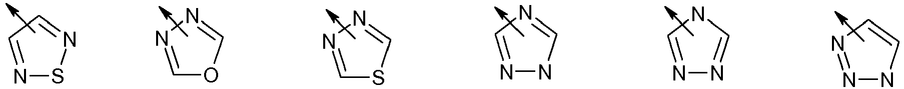 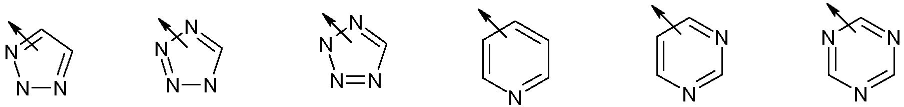 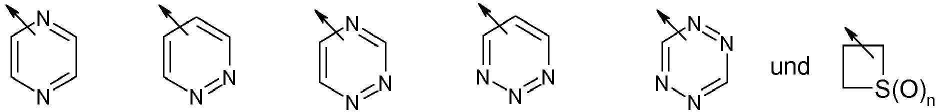
oder X¹ bedeutet durch m Reste aus der Gruppe bestehend aus R⁶, R^{6a}, R⁸ und R⁹ substituiertes Phenyl;
X², X⁴ und X⁶ bedeuten unabhängig voneinander jeweils Wasserstoff, Fluor, Chlor, Brom, Iod, Cyano, Nitro,
oder jeweils durch m Reste aus der Gruppe bestehend aus Fluor, Chlor, Brom, Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Alkylcarbonyl;
X³ und X⁵ bedeuten unabhängig voneinander Wasserstoff Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, S(O)ₙR⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₂-C₆)-Alkenyloxy oder (C₂-C₆)-Alkinyloxy;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷,
R⁹ bedeutet (C₁-C₃)-Alkyl oder (C₁-C₃)-Alkoxy,
k bedeutet 3, 4, 5 oder 6;
m bedeutet 0, 1, 2, 3, 4 oder 5;
n bedeutet 0, 1 oder 2;
p bedeutet 0 oder 1;
q bedeutet 3, 4 oder 5;
s bedeutet 0, 1, 2, 3 oder 4.

3. 5-Oxy-substituierte 3-Phenylisoxazolin-5-carboxamide und 5-oxy-substituierte 3-Phenylisoxazolin-5-thioamide nach Anspruch 1 oder 2,
worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl,
R³ bedeutet durch jeweils m Reste aus der Gruppe bestehend aus Fluor, Chlor, Brom, Cyano, (C₁-C₂)-Alkoxy-substituiertes (C₁-C₄)-Alkyl, (C₃-C₄)-Cycloalkyl, (C₂-C₃)-Alkenyl oder (C₂-C₃)-Alkinyl,
A bedeutet eine Bindung oder eine divalente Einheit aus der Gruppe bestehend aus CH₂, CH₂CH₂, CHCH₃, CH₂CH₂CH₂, CH(CH₂CH₃), CH(CH₃)CH₂, C(CH₃)₂, C(CH₃)₂CH₂, C(iPr)CH₃, CH(CH₂iPr)CH₂, CH₂CH=CH, C(CH₃)₂C=C, CH(CF₃)CH₂, CH(CH₃)CH₂O, CH₂CH₂O, CH(cPr)CH₂O, CH(CH₂OCH₃), CH(CH₂CH₂SCH₃), CH(COOH), CH(COOCH₃), CH(COOH)CH₂, CH(COOCH₃)CH₂, CH₂COH(CF₃), CH(CONHCH₃), CH(CONHCH₃)CH₂ und CH₂CH₂CONHCH₂;
R⁴ bedeutet Wasserstoff oder (C₁-C₈)-Alkyl,
Y bedeutet Sauerstoff oder Schwefel;
X bedeutet Wasserstoff, Cyano, Hydroxy, X¹,
oder
durch jeweils m Reste aus der Gruppe bestehend aus Fluor, Chlor, Cyano, Hydroxy, OR⁷, X¹, OX¹, NHX¹, S(O)ₙR⁵, CO₂R⁸, CONR⁶R⁸, C0NR⁸SO₂R⁵ und POR⁹R⁹ substituiertes (C₁-C₁₂)-Alkyl, (C₃-C₈)-Cycloalkyl, (C₂-C₁₂)-Alkenyl oder (C₂-C₁₂)-Alkinyl;
X¹ bedeutet einen durch s Reste aus der Gruppe bestehend aus R⁶, R^{6a}, R⁸ und R⁹ substituierten Ring aus der Gruppe bestehend aus 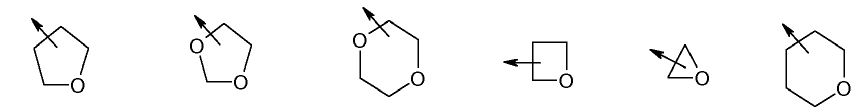 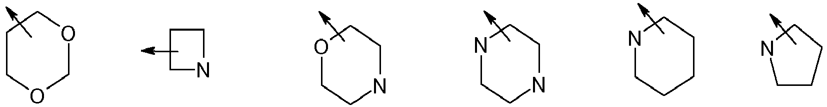 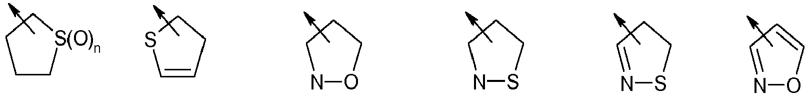 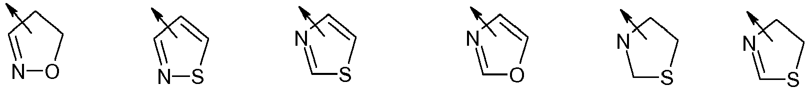 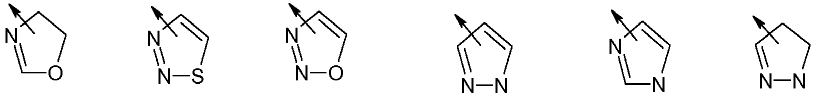 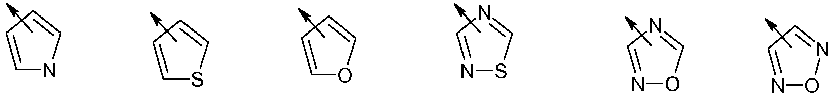 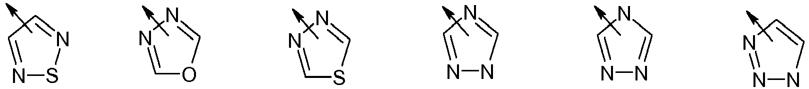 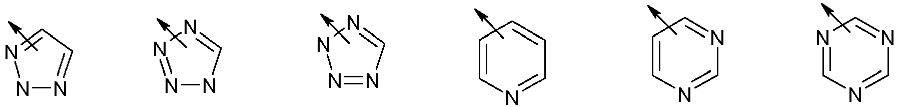 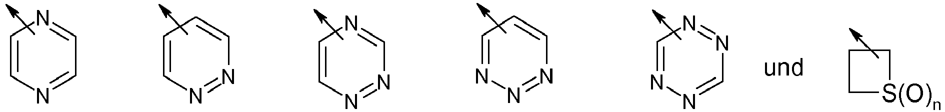
oder X¹ bedeutet durch m Reste aus der Gruppe bestehend aus R⁶, R^{6a}, R⁸ und R⁹ substituiertes Phenyl;
X², X⁴ und X⁶ bedeuten unabhängig voneinander jeweils Wasserstoff, Fluor, oder Chlor,
oder jeweils durch m Reste aus der Gruppe bestehend aus Fluor, Chlor, Cyano und C₁-C₄)-Alkoxy substituiertes (C₁-C₄)-Alkyl oder (C₁-C₄)-Alkoxy,
X³ und X⁵ bedeuten unabhängig voneinander Wasserstoff, Fluor, Chlor, Brom, Cyano,
oder jeweils durch m Reste aus der Gruppe bestehend aus Fluor und Chlor substituiertes (C₁-C₆)-Alkyl,
oder durch m Reste aus der Gruppe bestehend aus Fluor und Chlor substituiertes (C₁-C₆)-Alkoxy,
R⁵ bedeutet Methyl oder Ethyl;
R⁶ bedeutet Wasserstoff oder R⁵;
R^{6a} bedeutet Fluor, Chlor, Brom, Jod, Cyano, Hydroxy, S(O)ₙR⁵ oder durch jeweils m Reste aus der Gruppe bestehend aus Fluor, Chlor, Brom, Cyano und (C₁-C₂)-Alkoxy substituiertes (C₁-C₆)-Alkoxy, (C₂-C₆)-Alkenyloxy oder (C₂-C₆)-Alkinyloxy;
R⁷ bedeutet Wasserstoff oder durch jeweils m Reste aus der Gruppe bestehend aus Fluor und Chlor substituiertes (C₁-C₆)-Alkyl,
R⁸ bedeutet R⁷,
R⁹ bedeutet (C₁-C₃)-Alkoxy,
m bedeutet 0, 1, 2 oder 3;
n bedeutet 0, 1 oder 2;
s bedeutet 0, 1, 2, 3 oder 4.

4. Herbizide Mittel, **gekennzeichnet durch** einen herbizid wirksamen Gehalt an mindestens einer Verbindung der Formel (I) gemäß einem der Ansprüche 1 bis 3.

5. Herbizide Mittel nach Anspruch 4 in Mischung mit Formulierungshilfsmitteln.

6. Herbizide Mittel nach Anspruch 4 oder 5 enthaltend mindestens einen weiteren pestizid wirksame Stoffen aus der Gruppe Insektizide, Akarizide, Herbizide, Fungizide, Safenern und Wachstumsregulatoren.

7. Herbizide Mittel nach Anspruch 6 enthaltend einen Safener.

8. Herbizide Mittel nach Anspruch 7, worin der Safener ausgewählt ist aus der Gruppe bestehend aus Mefenpyr-diethyl, Cyprosulfamid, Isoxadifen-ethyl, Cloquintocet-mexyl, Benoxacor und Dichlormid.

9. Herbizide Mittel nach einem der Ansprüche 6 bis 8 enthaltend ein weiteres Herbizid.

10. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 3 oder eines herbiziden Mittels nach einem der Ansprüche 4 bis 9 auf die Pflanzen oder auf den Ort des unerwünschten Pflanzenwachstums appliziert.

11. Verwendung von Verbindungen der Formel (I) gemäß einem der Ansprüche 1 bis 3 oder von herbiziden Mitteln nach einem der Ansprüche 4 bis 9 zur Bekämpfung unerwünschter Pflanzen.

12. Verwendung nach einem der Ansprüche 10 oder 11, **dadurch gekennzeichnet, daß** die Verbindungen der Formel (I) zur Bekämpfung unerwünschter Pflanzen in Kulturen von Nutzpflanzen eingesetzt werden.

13. Verwendung nach Anspruch 12, **dadurch gekennzeichnet, daß** die Nutzpflanzen transgene Nutzpflanzen sind.

14. Fungizide Mittel, **gekennzeichnet durch** einen fungizid wirksamen Gehalt an mindestens einer Verbindung der Formel (I) gemäß einem der Ansprüche 1 bis 3.

15. Fungizide Mittel nach Anspruch 14 in Mischung mit Formulierungshilfsmitteln.

16. Fungizide Mittel nach Anspruch 14 oder 15 enthaltend mindestens einen weiteren pestizid wirksame Stoffen aus der Gruppe Insektizide, Akarizide, Herbizide, Fungizide, Safenern und Wachstumsregulatoren.

17. Verbindungen der Formel (II) 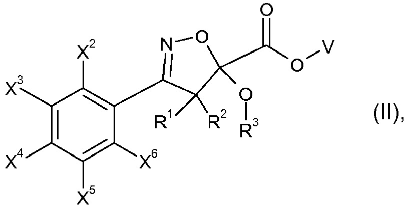 worin
V Wasserstoff oder R⁵ bedeutet, und
X¹, X², X³, X⁴, X⁵, X⁶, R¹, R², R³ und R⁵ wie in einem der Ansprüche 1 bis 3 definiert sind.

## Claims

1. 5-Oxy-substituted 3-phenylisoxazoline-5-carboxamides and 5-oxy-substituted 3-phenylisoxazoline-5-thioamides of the formula (I) or salts thereof 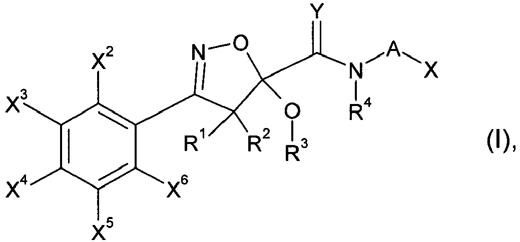 in which
R¹ and R² are each independently hydrogen, fluorine, chlorine, bromine, iodine, cyano, or (C₁-C₄)-alkyl or (C₁-C₄)-alkoxy each substituted by m radicals from the group consisting of fluorine, chlorine, bromine, iodine and cyano,
or
R¹ and R² together with the carbon atom to which they are bonded form a saturated or partly or fully unsaturated three-, four- or five-membered ring formed from q carbon atoms and p oxygen atoms;
R³ is (C₁-C₆)-alkyl, (C₃-C₆)-cycloalkyl, (C₂-C₆)-alkenyl or (C₂-C₆)-alkynyl each substituted by m radicals from the group consisting of fluorine, chlorine, bromine, iodine, cyano, (C₁-C₄)-alkoxy and hydroxyl,
R⁴ is hydrogen, cyano,
or (C₁-C₈)-alkyl, (C₃-C₈) -cycloalkyl, (C₃-C₈) -alkenyl or (C₃-C₈)-alkynyl each substituted by m radicals from the group consisting of fluorine, chlorine, bromine, iodine, cyano, hydroxyl and (C₁-C₆)-alkoxy,
A is a bond or a divalent unit from the group consisting of 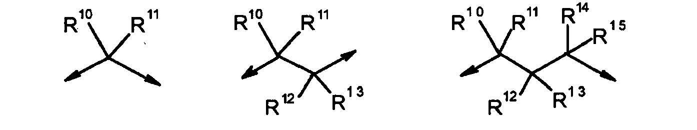 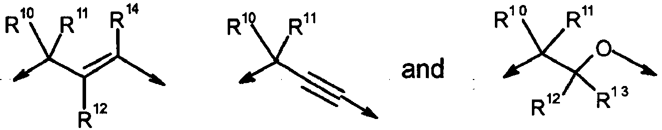
R¹⁰, R¹¹, R¹², R¹³, R¹⁴ and R¹⁵ are each independently hydrogen, fluorine, chlorine, bromine, iodine, hydroxyl, cyano, CO₂R⁸, CONR⁶R⁸, R⁵,
or (C₁-C₆)-alkyl, (C₃-C₅)-cycloalkyl, (C₂-C₆)-alkenyl, (C₂-C₆)-alkynyl each substituted by m radicals from the group consisting of fluorine, chlorine, bromine, iodine, hydroxyl and cyano,
or (C₁-C₆)-alkoxy, (C₃-C₆) -cycloalkoxy, (C₂-C₆)-alkenyloxy or (C₂-C₆)-alkynyloxy each substituted by m radicals from the group consisting of fluorine, chlorine, bromine, iodine, cyano and (C₁-C₂)-alkoxy;
Y is oxygen or sulfur;
X is hydrogen, cyano, hydroxyl, X¹,
or
(C₁-C₁₂)-alkyl, (C₃-C₈)-cycloalkyl, (C₂-C₁₂)-alkenyl or (C₂-C₁₂)-alkynyl each substituted by m radicals from the group consisting of fluorine, chlorine, bromine, iodine, cyano, hydroxyl, OR⁷, X¹, OX¹, NHX¹, S(O)ₙR⁵, SO₂NR⁶R⁷, SO₂NR⁶COR⁸, CO₂R⁸, CONR⁶R⁸, COR⁶, CONR⁸SO₂R⁵,
NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ and C(R⁶)=NOR⁸,
or
X, A and R⁴ together with the nitrogen atom to which they are bonded form a saturated or partly or fully unsaturated five-, six- or seven-membered ring containing, as well as this nitrogen atom, k carbon atoms, n oxygen atoms, p sulfur atoms and p elements from the group consisting of NR⁷ and NCOR⁷ as ring atoms, where one carbon atom bears p oxo groups;
X¹ is a three-, four-, five- or six-membered saturated, partly unsaturated, fully unsaturated or aromatic ring which is formed from r carbon atoms, s nitrogen atoms, n sulfur atoms and n oxygen atoms, and which is substituted by s radicals from the group consisting of R⁶, R^{6a}, R⁸ and R⁹, where the sulfur atoms and carbon atoms bear n oxo groups;
or X¹ is phenyl substituted by m radicals from the group consisting of R⁶, R^{6a}, R⁸ and R⁹;
X², X⁴ and X⁶ are each independently hydrogen, fluorine, chlorine, bromine, iodine, cyano, nitro,
or (C₁-C₄)-alkyl, (C₃-C₅)-cycloalkyl, (C₂-C₄)-alkenyl, (C₂-C₄)-alkynyl, (C₁-C₄)-alkoxy, (C₂-C₄)-alkenyloxy, (C₂-C₄)-alkynyloxy or (C₁-C₄)-alkylcarbonyl each substituted by m radicals from the group consisting of fluorine, chlorine, bromine, iodine, cyano and (C₁-C₄)-alkoxy;
X³ and X⁵ are each independently hydrogen, fluorine, chlorine, bromine, iodine, hydroxyl, cyano, nitro, SF₅, C0NR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, S(O)ₙR⁵, SO₂NR⁶R⁸, OSO₂NR⁶R⁸,
or (C₁-C₆)-alkyl, (C₃-C₅)-cycloalkyl, (C₂-C₆)-alkenyl, (C₂-C₆)-alkynyl each substituted by m radicals from the group consisting of fluorine, chlorine, bromine, iodine, hydroxyl and cyano,
or (C₁-C₆)-alkoxy, (C₃-C₆) -cycloalkoxy, (C₂-C₆)-alkenyloxy or (C₂-C₆)-alkynyloxy each substituted by m radicals from the group consisting of fluorine, chlorine, bromine, iodine, cyano and (C₁-C₂)-alkoxy;
R⁵ is (C₁-C₆)-alkyl or (C₃-C₆)-cycloalkyl each substituted by m radicals from the group consisting of fluorine, chlorine, bromine, iodine, cyano and hydroxyl;
R⁶ is hydrogen or R⁵;
R^{6a} is fluorine, chlorine, bromine, iodine, cyano, hydroxyl, S(O)ₙR⁵, or (C₁-C₆)-alkoxy, (C₃-C₆)-alkenyloxy or (C₃-C₆)-alkynyloxy each substituted by m radicals from the group consisting of fluorine, chlorine, bromine, cyano and (C₁-C₂)-alkoxy;
R⁷ is hydrogen or (C₁-C₆)-alkyl, (C₃-C₆) -cycloalkyl, (C₂-C₄)-alkenyl or (C₂-C₄)-alkynyl each substituted by m radicals from the group consisting of fluorine, chlorine, bromine, cyano and (C₁-C₂)-alkoxy;
R⁸ is R⁷,
R⁹ is (C₁-C₃)-alkyl or (C₁-C₃)-alkoxy;
k is 3, 4, 5 or 6;
m is 0, 1, 2, 3, 4 or 5;
n is 0, 1 or 2;
p is 0 or 1;
q is 3, 4 or 5;
r is 1, 2, 3, 4 or 5;
s is 0, 1, 2, 3 or 4.

2. 5-Oxy-substituted 3-phenylisoxazoline-5-carboxamides and 5-oxy-substituted 3-phenylisoxazoline-5-thioamides according to Claim 1,
in which
R¹ and R² are each independently hydrogen, fluorine, chlorine, bromine, iodine, cyano, or (C₁-C₄)-alkyl or (C₁-C₄)-alkoxy each substituted by m radicals from the group consisting of fluorine, chlorine, bromine, iodine and cyano,
or
R¹ and R² together with the carbon atom to which they are bonded form a saturated or partly or fully unsaturated three-, four- or five-membered ring formed from q carbon atoms and p oxygen atoms;
R³ is (C₁-C₆)-alkyl, (C₃-C₆)-cycloalkyl, (C₂-C₆)-alkenyl or (C₂-C₆)-alkynyl each substituted by m radicals from the group consisting of fluorine, chlorine, bromine, iodine, cyano, (C₁-C₄)-alkoxy and hydroxyl,
R⁴ is hydrogen, cyano,
or (C₁-C₈)-alkyl or (C₃-C₈)-cycloalkyl each substituted by m radicals from the group consisting of fluorine, chlorine, bromine, iodine, cyano, hydroxyl and (C₁-C₆)-alkoxy;
A is a bond or a divalent unit from the group consisting of 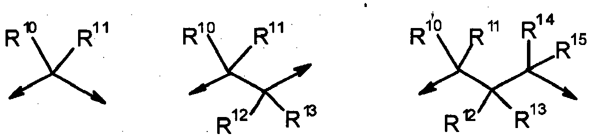 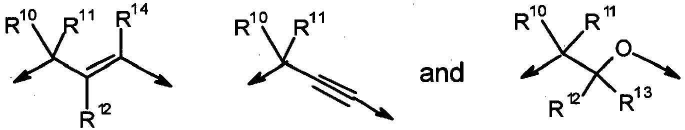
R¹⁰, R¹¹, R¹², R¹³, R¹⁴ and R¹⁵ are each independently hydrogen, fluorine, chlorine, bromine, iodine, hydroxyl, cyano, CO₂R⁸, CONR⁶R⁸, R⁵,
or (C₁-C₆)-alkyl, (C₃-C₅)-cycloalkyl, (C₂-C₆)-alkenyl, (C₂-C₆)-alkynyl each substituted by m radicals from the group consisting of fluorine, chlorine, bromine, iodine, hydroxyl and cyano,
or (C₁-C₆) -alkoxy, (C₃-C₆) -cycloalkoxy, (C₂-C₆)-alkenyloxy or (C₂-C₆)-alkynyloxy each substituted by m radicals from the group consisting of fluorine, chlorine, bromine, iodine, cyano and (C₁-C₂)-alkoxy;
Y is oxygen or sulfur;
X is hydrogen, cyano, hydroxyl, X¹,
or
(C₁-C₁₂)-alkyl, (C₃-C₈)-cycloalkyl, (C₂-C₁₂)-alkenyl or (C₂-C₁₂)-alkynyl each substituted by m radicals from the group consisting of fluorine, chlorine, bromine, iodine, cyano, hydroxyl, OR⁷, X¹, OX¹, NHX¹, S(O)R⁵, SO₂NR⁶R⁷, SO₂NR⁶COR⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ and C(R⁶)=NOR⁸,
or
X, A and R⁴ together with the nitrogen atom to which they are bonded form a saturated or partly or fully unsaturated five-, six- or seven-membered ring containing, as well as this nitrogen atom, k carbon atoms, n oxygen atoms, p sulfur atoms and p elements from the group consisting of NR⁷ and NCOR⁷ as ring atoms, where one carbon atom bears p oxo groups;
X¹ is a ring, substituted by s radicals from the group consisting of R⁶, R^{6a}, R⁸ and R⁹, from the group consisting of 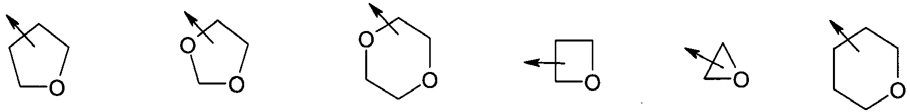 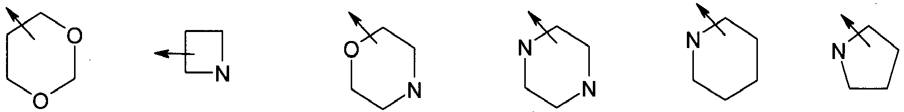 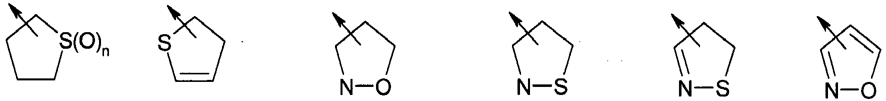 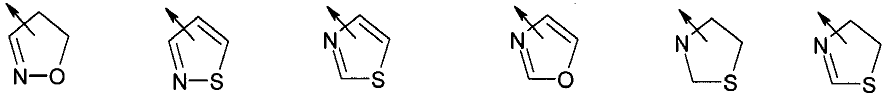 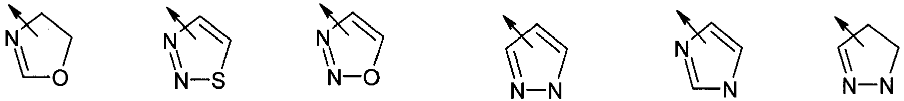 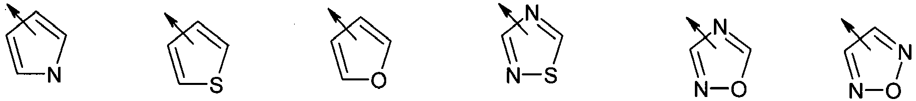 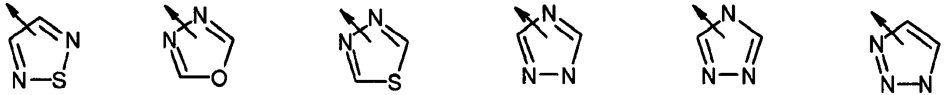 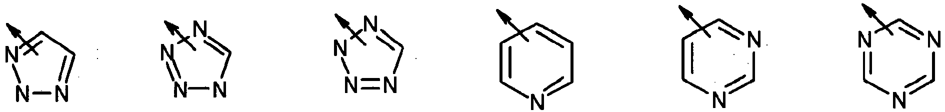 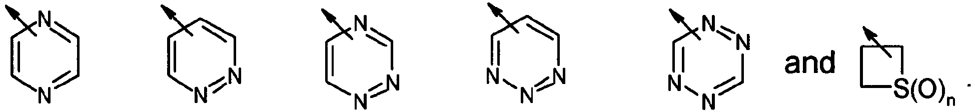
or X¹ is phenyl substituted by m radicals from the group consisting of R⁶, R^{6a}, R⁸ and R⁹;
X², X⁴ and X⁶ are each independently hydrogen, fluorine, chlorine, bromine, iodine, cyano, nitro,
or (C₁-C₄)-alkyl, (C₃-C₅)-cycloalkyl, (C₂-C₄)-alkenyl, (C₂-C₄)-alkynyl, (C₁C₄)-alkoxy, (C₂-C₄)-alkenyloxy, (C₂-C₄)-alkynyloxy or (C₁-C₄)-alkylcarbonyl each substituted by m radicals from the group consisting of fluorine, chlorine, bromine, iodine, cyano and (C₁-C₄)-alkoxy;
X³ and X⁵ are each independently hydrogen, fluorine, chlorine, bromine, iodine, hydroxyl, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, S(O)ₙR⁵, SO₂NR⁶R⁸, OSO₂NR⁶R⁸,
or (C₁-C₆)-alkyl, (C₃-C₅)-cycloalkyl, (C₂-C₆)-alkenyl, (C₂-C₆)-alkynyl each substituted by m radicals from the group consisting of fluorine, chlorine, bromine, iodine, hydroxyl and cyano,
or (C₁-C₆)-alkoxy, (C₃-C₆) -cycloalkoxy, (C₂-C₆)-alkenyloxy or (C₂-C₆)-alkynyloxy each substituted by m radicals from the group consisting of fluorine, chlorine, bromine, iodine, cyano and (C₁-C₂)-alkoxy;
R⁵ is (C₁-C₆)-alkyl or (C₃-C₆) -cycloalkyl each substituted by m radicals from the group consisting of fluorine, chlorine, bromine, iodine, cyano and hydroxyl;
R⁶ is hydrogen or R⁵;
R^{6a} is fluorine, chlorine, bromine, iodine, cyano, hydroxyl, S(O)ₙR⁵, or (C₁-C₆)-alkoxy, (C₂-C₆)-alkenyloxy or (C₂-C₆)-alkynyloxy each substituted by m radicals from the group consisting of fluorine, chlorine, bromine, cyano and (C₁-C₂)-alkoxy;
R⁷ is hydrogen or (C₁-C₆)-alkyl, (C₃-C₆)-cycloalkyl, (C₂-C₄)-alkenyl or (C₂-C₄)-alkynyl each substituted by m radicals from the group consisting of fluorine, chlorine, bromine, cyano and (C₁-C₂)-alkoxy;
R⁸ is R⁷,
R⁹ is (C₁-C₃)-alkyl or (C₁-C₃)-alkoxy;
k is 3, 4, 5 or 6;
m is 0, 1, 2, 3, 4 or 5;
n is 0, 1 or 2;
p is 0 or 1;
q is 3, 4 or 5;
s is 0, 1, 2, 3 or 4.

3. 5-Oxy-substituted 3-phenylisoxazoline-5-carboxamides and 5-oxy-substituted 3-phenylisoxazoline-5-thioamides according to Claim 1 or 2,
in which
R¹ and R² are each independently hydrogen, fluorine, chlorine, bromine, iodine, cyano, or (C₁-C₄)-alkyl substituted by m radicals from the group consisting of fluorine, chlorine, bromine, iodine and cyano;
R³ is (C₁-C₄)-alkyl, (C₃-C₄)-cycloalkyl, (C₂-C₃)-alkenyl or (C₂-C₃)-alkynyl each substituted by m radicals from the group consisting of fluorine, chlorine, bromine, cyano, (C₁-C₂)-alkoxy,
A is a bond or a divalent unit from the group consisting of CH₂, CH₂CH₂, CHCH₃, CH₂CH₂CH₂, CH(CH₂CH₃), CH(CH₃)CH₂, C(CH₃)₂, C(CH₃)₂CH₂, C(iPr)CH₃, CH(CH₂iPr)CH₂, CH₂CH=CH, C(CH₃)₂C≡C, CH(CF₃)CH₂, CH(CH₃)CH₂O, CH₂CH₂O, CH(cPr)CH₂O, CH(CH₂OCH₃) CH(CH₂CH₂SCH₃), CH(COOH), CH(COOCH₃), CH(COOH)CH₂, CH (COOCH₃) CH₂, CH₂COH(CF₃), CH(CONHCH₃), CH (CONHCH₃) CH₂ and CH₂CH₂CONHCH₂;
R⁴ is hydrogen or (C₁-C₈)-alkyl;
Y is oxygen or sulfur;
X is hydrogen, cyano, hydroxyl, X¹,
or (C₁-C₁₂)-alkyl, (C₃-C₈) -cycloalkyl, (C₂-C₁₂)-alkenyl or (C₂-C₁₂)-alkynyl each substituted by m radicals from the group consisting of fluorine, chlorine, cyano, hydroxyl, OR⁷, X¹, OX¹, NHX¹, S(O)ₙR⁵, CO₂R⁸, CONR⁶R⁸, CONR⁸SO₂R⁵ and POR⁹R⁹;
X¹ is a ring, substituted by s radicals from the group consisting of R⁶, R^{6a}, R⁸ and R⁹, from the group consisting of 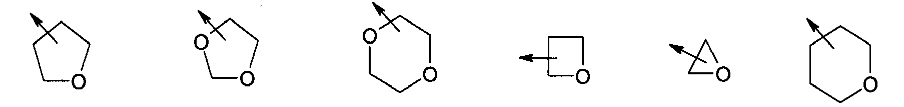 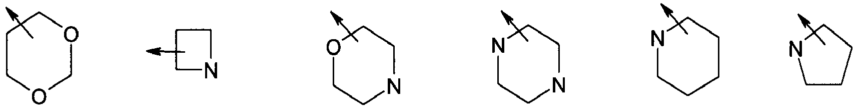 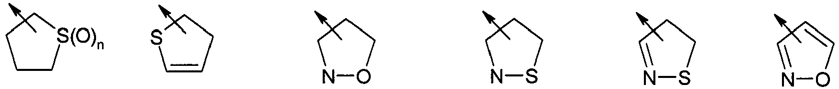 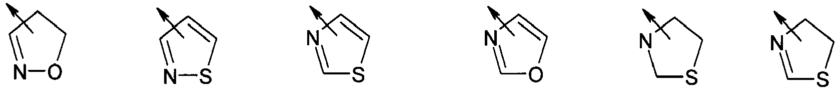 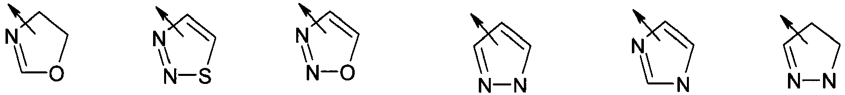 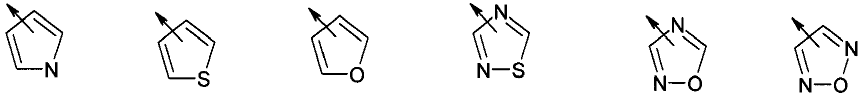 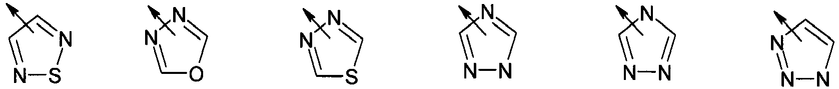 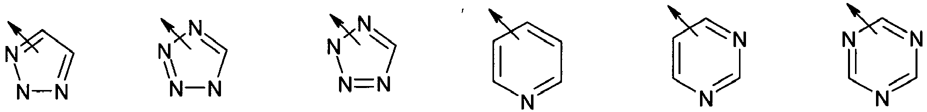 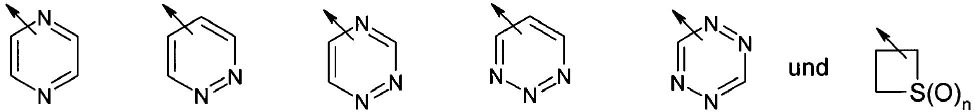
or X¹ is phenyl substituted by m radicals from the group consisting of R⁶, R^{6a}, R⁸ and R⁹;
X², X⁴ and X⁶ are each independently hydrogen, fluorine, or chlorine,
or (C₁-C₄)-alkyl or (C₁-C₄)-alkoxy each substituted by m radicals from the group consisting of fluorine, chlorine, cyano and (C₁-C₄)-alkoxy;
X³ and X⁵ are each independently hydrogen, fluorine, chlorine, bromine, cyano,
or (C₁-C₆)-alkyl substituted by m radicals from the group consisting of fluorine and chlorine, or (C₁-C₆)-alkoxy substituted by m radicals from the group consisting of fluorine and chlorine;
R⁵ is methyl or ethyl;
R⁶ is hydrogen or R⁵;
R^{6a} is fluorine, chlorine, bromine, iodine, cyano, hydroxyl, S(O)ₙR⁵, or (C₁-C₆)-alkoxy, (C₂-C₆)-alkenyloxy or (C₂-C₆)-alkynyloxy each substituted by m radicals from the group consisting of fluorine, chlorine, bromine, cyano and (C₁-C₂)-alkoxy;
R⁷ is hydrogen or (C₁-C₆)-alkyl substituted by m radicals from the group consisting of fluorine and chlorine;
R⁸ is R⁷,
R⁹ is (C₁-C₃)-alkoxy;
m is 0, 1, 2 or 3;
n is 0, 1 or 2;
s is 0, 1, 2, 3 or 4.

4. Herbicidal compositions, **characterized by** a herbicidally active content of at least one compound of the formula (I) according to any of Claims 1 to 3.

5. Herbicidal compositions according to Claim 4 in a mixture with formulation auxiliaries.

6. Herbicidal compositions according to Claim 4 or 5, comprising at least one further pesticidally active substance from the group of insecticides, acaricides, herbicides, fungicides, safeners and growth regulators.

7. Herbicidal compositions according to Claim 6, comprising a safener.

8. Herbicidal compositions according to Claim 7, in which the safener is selected from the group consisting of mefenpyr-diethyl, cyprosulfamide, isoxadifen-ethyl, cloquintocet-mexyl, benoxacor and dichlormid.

9. Herbicidal compositions according to any of Claims 6 to 8, comprising a further herbicide.

10. Method for controlling unwanted plants, **characterized in that** an effective amount of at least one compound of the formula (I) according to any of Claims 1 to 3 or of a herbicidal composition according to any of Claims 4 to 9 is applied to the plants or the site of the unwanted vegetation.

11. Use of compounds of the formula (I) according to any of Claims 1 to 3 or of herbicidal compositions according to any of Claims 4 to 9 for controlling unwanted plants.

12. Use according to any of Claims 10 or 11, **characterized in that** the compounds of the formula (I) are used for controlling unwanted plants in crops of useful plants.

13. Use according to Claim 12, **characterized in that** the useful plants are transgenic useful plants.

14. Fungicidal compositions, **characterized by** a fungicidally active amount of at least one compound of the formula (I) according to any of Claims 1 to 3.

15. Fungicidal compositions according to Claim 14 in a mixture with formulation auxiliaries.

16. Fungicidal compositions according to Claim 14 or 15, comprising at least one further pesticidally active substance from the group of insecticides, acaricides, herbicides, fungicides, safeners and growth regulators.

17. Compounds of the formula (II) 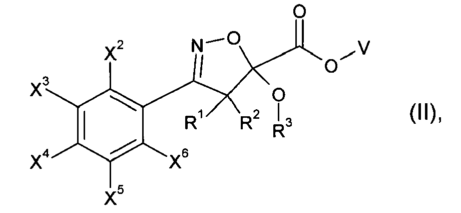 in which
V is hydrogen or R⁵, and
X¹, X², X³, X⁴, X⁵, X⁶, R¹, R², R³ and R⁵ are each as defined in one of Claims 1 to 3.

## Revendications

1. 3-Phénylisoxazoline-5-carboxamides à substitution 5-oxy et 3-phénylisoxazoline-5-thioamides à substitution 5-oxy de formule (I), ou leurs sels 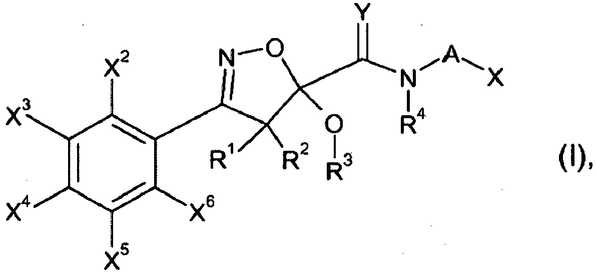 dans lesquels
R¹ et R² signifient chacun indépendamment l'un de l'autre hydrogène, fluor, chlore, brome, iode, cyano, ou alkyle en (C₁-C₄) ou alcoxy en (C₁-C₄), chacun substitué par m radicaux du groupe constitué par fluor, chlore, brome, iode et cyano,
ou
R¹ et R² forment ensemble avec l'atome de carbone auquel ils sont reliés un cycle saturé, partiellement ou entièrement insaturé, à trois, quatre ou cinq éléments, qui est formé par q atomes de carbone et p atomes d'oxygène ;
R³ signifie alkyle en (C₁-C₆), cycloalkyle en (C₃-C₆), alcényle en (C₂-C₆)ou alcynyle en (C₂-C₆), chacun substitué par m radicaux du groupe constitué par fluor, chlore, brome, iode, cyano, alcoxy en (C₁-C₄) et hydroxy ;
R⁴ signifie hydrogène, cyano,
ou alkyle en (C₁-C₈), cycloalkyle en (C₃-C₈), alcényle en (C₃-C₈) ou alcynyle en (C₃-C₈), chacun substitué par m radicaux du groupe constitué par fluor, chlore, brome, iode, cyano, hydroxy et alcoxy en (C₁-C₆) ;
A signifie une liaison ou une unité bivalente du groupe constitué par 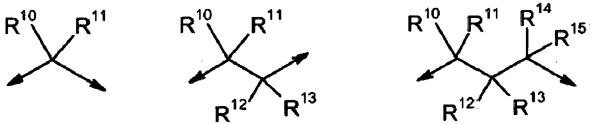 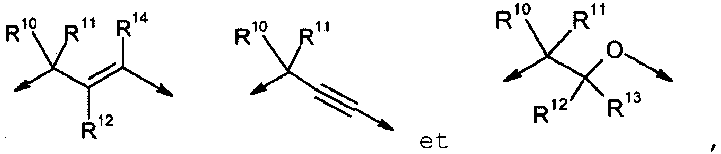
R¹⁰, R¹¹, R¹², R¹³, R¹⁴ et R¹⁵ signifient chacun indépendamment les uns des autres hydrogène, fluor, chlore, brome, iode, hydroxy, cyano, CO₂R⁸, CONR⁶R⁸, R⁵, ou alkyle en (C₁-C₆), cycloalkyle en (C₃-C₈), alcényle en (C₂-C₆), alcynyle en (C₂-C₆), chacun substitué par m radicaux du groupe constitué par fluor, chlore, brome, iode, hydroxy et cyano,
ou alcoxy en (C₁-C₆), cycloalcoxy en (C₃-C₆), alcényloxy en (C₂-C₆)ou alcynyloxy en (C₂-C₆), chacun substitué par m radicaux du groupe constitué par fluor, chlore, brome, iode, cyano et alcoxy en (C₁-C₂) ;
Y signifie oxygène ou soufre ;
X signifie hydrogène, cyano, hydroxy, X¹,
ou
alkyle en (C₁-C₁₂), cycloalkyle en (C₃-C₈), alcényle en (C₂-C₁₂) ou alcynyle en (C₂-C₁₂), chacun substitué par m radicaux du groupe constitué par fluor, chlore, brome, iode, cyano, hydroxy, OR⁷, X¹, OX¹, NHX¹, S(O)ₙR⁵, SO₂NR⁶R⁷, SO₂NR⁶COR⁸, CO₂R⁸, CONR⁶R⁸, COR⁶, CONR⁸SO₂R⁵,
NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ et C(R⁶)=NOR⁸,
ou
X, A et R⁴ forment avec l'atome d'azote auquel ils sont reliés un cycle saturé, partiellement ou entièrement insaturé, à cinq, six ou sept éléments, qui contient en plus de cet atome d'azote k atomes de carbone, n atomes d'oxygène, p atomes de soufre et p éléments du groupe constitué par NR⁷ et NCOR⁷ en tant qu'atomes de cycle, un atome de carbone portant p groupes oxo ;
X¹ signifie un cycle à trois, quatre, cinq ou six éléments, saturé, partiellement insaturé, entièrement insaturé ou aromatique, qui est formé par r atomes de carbone, s atomes d'azote, n atomes de soufre et n atomes d'oxygène, et qui est substitué par s radicaux du groupe constitué par R⁶, R^{6a}, R⁸ et R⁹, les atomes de soufre et les atomes de carbone portant n groupes oxo ; ou X¹ signifie phényle substitué par m radicaux du groupe constitué par R⁶, R^{6a}, R⁸ et R⁹ ;
X², X⁴ et X⁶ signifient chacun indépendamment les uns des autres hydrogène, fluor, chlore, brome, iode, cyano, nitro,
ou alkyle en (C₁-C₄), cycloalkyle en (C₃-C₅), alcényle en (C₂-C₄), alcynyle en (C₂-C₄), alcoxy en (C₁-C₄), alcényloxy en (C₂-C₄), alcynyloxy en (C₂-C₄)ou alkylcarbonyle en (C₁-C₄), chacun substitué par m radicaux du groupe constitué par fluor, chlore, brome, iode, cyano et alcoxy en (C₁-C₄) ;
X³ et X⁵ signifient indépendamment l'un de l'autre hydrogène, fluor, chlore, brome, iode, hydroxy, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, S(O)ₙR⁵, SO₂NR⁶R⁸, OSO₂NR⁶R⁸, ou alkyle en (C₁-C₆), cycloalkyle en (C₃-C₅), alcényle en (C₂-C₆), alcynyle en (C₂-C₆), chacun substitué par m radicaux du groupe constitué par fluor, chlore, brome, iode, hydroxy et cyano,
ou alcoxy en (C₁-C₆), cycloalcoxy en (C₃-C₆), alcényloxy en (C₂-C₆)ou alcynyloxy en (C₂-C₆), chacun substitué par m radicaux du groupe constitué par fluor, chlore, brome, iode, cyano et alcoxy en (C₁-C₂) ;
R⁵ signifie alkyle en (C₁-C₆) ou cycloalkyle en (C₃-C₆), chacun substitué par m radicaux du groupe constitué par fluor, chlore, brome, iode, cyano et hydroxy ;
R⁶ signifie hydrogène ou R⁵;
R^{6a} signifie fluor, chlore, brome, iode, cyano, hydroxy, S(O)ₙR⁵, ou alcoxy en (C₁-C₆), alcényloxy en (C₃-C₆) ou alcynyloxy en (C₃-C₆), chacun substitué par m radicaux du groupe constitué par fluor, chlore, brome, cyano et alcoxy en (C₁-C₂) ;
R⁷ signifie hydrogène ou alkyle en (C₁-C₆), cycloalkyle en (C₃-C₆), alcényle en (C₂-C₄) ou alcynyle en (C₂-C₄), chacun substitué par m radicaux du groupe constitué par fluor, chlore, brome, cyano et alcoxy en (C₁-C₂) ;
R⁸ signifie R⁷ ;
R⁹ signifie alkyle en (C₁-C₃) ou alcoxy en (C₁-C₃) ;
k signifie 3, 4, 5 ou 6 ;
m signife 0, 1, 2, 3, 4 ou 5 ;
n signifie 0, 1 ou 2 ;
p signifie 0 ou 1 ;
q signifie 3, 4 ou 5 ;
r signifie 1, 2, 3, 4 ou 5 ;
s signifie 0, 1, 2, 3 ou 4.

2. 3-Phénylisoxazoline-5-carboxamides à substitution 5-oxy et 3-phénylisoxazoline-5-thioamides à substitution 5-oxy selon la revendication 1,
dans lesquels
R¹ et R² signifient chacun indépendamment l'un de l'autre hydrogène, fluor, chlore, brome, iode, cyano, ou alkyle en (C₁-C₄) ou alcoxy en (C₁-C₄), chacun substitué par m radicaux du groupe constitué par fluor, chlore, brome, iode et cyano,
ou
R¹ et R² forment ensemble avec l'atome de carbone auquel ils sont reliés un cycle saturé, partiellement ou entièrement insaturé, à trois, quatre ou cinq éléments, qui est formé par q atomes de carbone et p atomes d'oxygène ;
R³ signifie alkyle en (C₁-C₆), cycloalkyle en (C₃-C₆), alcényle en (C₂-C₆)ou alcynyle en (C₂-C₆), chacun substitué par m radicaux du groupe constitué par fluor, chlore, brome, iode, cyano, alcoxy en (C₁-C₄) et hydroxy ;
R⁴ signifie hydrogène, cyano,
ou alkyle en (C₁-C₈) ou cycloalkyle en (C₃-C₈), chacun substitué par m radicaux du groupe constitué par fluor, chlore, brome, iode, cyano, hydroxy et alcoxy en (C₁-C₆) ;
A signifie une liaison ou une unité bivalente du groupe constitué par 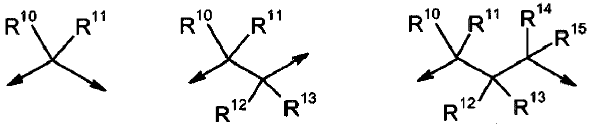 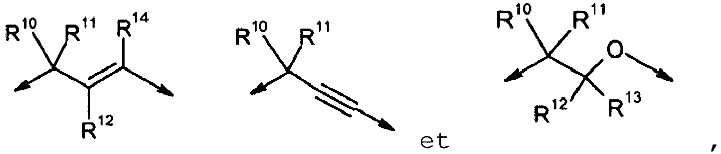
R¹⁰, R¹¹, R¹², R¹³, R¹⁴ et R¹⁵ signifient chacun indépendamment les uns des autres hydrogène, fluor, chlore, brome, iode, hydroxy, cyano, CO₂R⁸, CONR⁶R⁸, R⁵, ou alkyle en (C₁-C₆), cycloalkyle en (C₃-C₅), alcényle en (C₂-C₆), alcynyle en (C₂-C₆), chacun substitué par m radicaux du groupe constitué par fluor, chlore, brome, iode, hydroxy et cyano,
ou alcoxy en (C₁-C₆), cycloalcoxy en (C₃-C₆), alcényloxy en (C₂-C₆)ou alcynyloxy en (C₂-C₆), chacun substitué par m radicaux du groupe constitué par fluor, chlore, brome, iode, cyano et alcoxy en (C₁-C₂) ;
Y signifie oxygène ou soufre ;
X signifie hydrogène, cyano, hydroxy, X¹,
ou
alkyle en (C₁-C₁₂), cycloalkyle en (C₃-C₈), alcényle en (C₂-C₁₂) ou alcynyle en (C₂-C₁₂), chacun substitué par m radicaux du groupe constitué par fluor, chlore, brome, iode, cyano, hydroxy, OR⁷, X¹, OX¹, NHX¹, S(O)ₙR⁵, SO₂NR⁶R⁷, SO₂NCOR⁶R⁸, CO₂R⁸, CONR⁶R⁸, COR⁶, CONR⁸SO₂R⁵, NR⁶R⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OCSNR⁶R⁸, POR⁹R⁹ et C(R⁶)=NOR⁸,
ou
X, A et R⁴ forment avec l'atome d'azote auquel ils sont reliés un cycle saturé, partiellement ou entièrement insaturé, à cinq, six ou sept éléments, qui contient en plus de cet atome d'azote k atomes de carbone, n atomes d'oxygène, p atomes de soufre et p éléments du groupe constitué par NR⁷ et NCOR⁷ en tant qu'atomes de cycle, un atome de carbone portant p groupes oxo ;
X¹ signifie un cycle substitué par s radicaux du groupe constitué par R⁶, R^{6a}, R⁸ et R⁹, du groupe constitué par 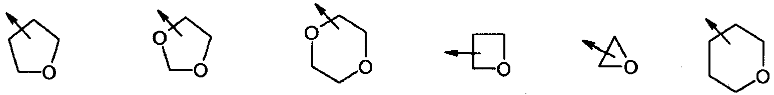 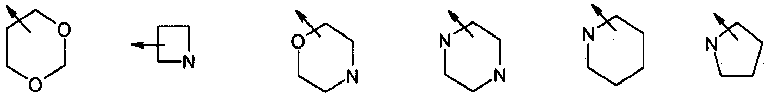 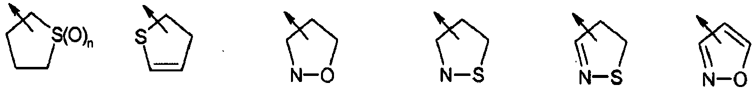 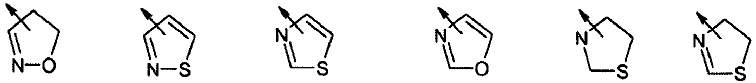 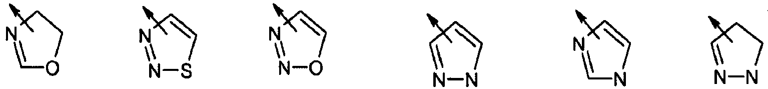 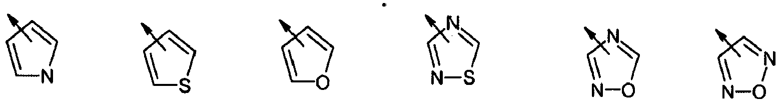 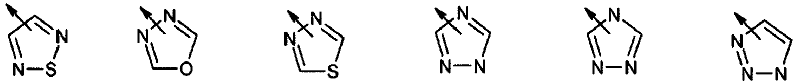 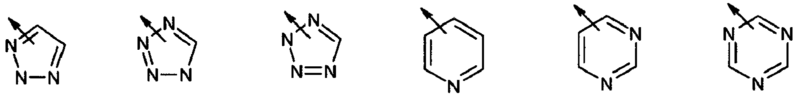 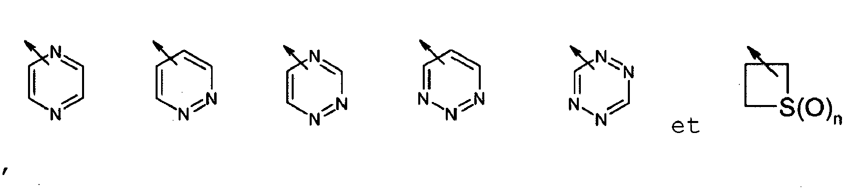
ou X¹ signifie phényle substitué par m radicaux du groupe constitué par R⁶, R^{6a}, R⁸ et R⁹ ;
X², X⁴ et X⁶ signifient chacun indépendamment les uns des autres hydrogène, fluor, chlore, brome, iode, cyano, nitro,
ou alkyle en (C₁-C₄), cycloalkyle en (C₃-C₅), alcényle en (C₂-C₄), alcynyle en (C₂-C₄), alcoxy en (C₁-C₄), alcényloxy en (C₂-C₄), alcynyloxy en (C₂-C₄) ou alkylcarbonyle en (C₁-C₄), chacun substitué par m radicaux du groupe constitué par fluor, chlore, brome, iode, cyano et alcoxy en (C₁-C₄) ;
X³ et X⁵ signifient indépendamment l'un de l'autre hydrogène, fluor, chlore, brome, iode, hydroxy, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, S(O)ₙR⁵, SO₂NR⁶R⁸, OSO₂NR⁶R⁸, ou alkyle en (C₁-C₆), cycloalkyle en (C₃-C₅), alcényle en (C₂-C₆), alcynyle en (C₂-C₆), chacun substitué par m radicaux du groupe constitué par fluor, chlore, brome, iode, hydroxy et cyano,
ou alcoxy en (C₁-C₆), cycloalcoxy en (C₃-C₆), alcényloxy en (C₂-C₆) ou alcynyloxy en (C₂-C₆), chacun substitué par m radicaux du groupe constitué par fluor, chlore, brome, iode, cyano et alcoxy en (C₁-C₂) ;
R⁵ signifie alkyle en (C₁-C₆) ou cycloalkyle en (C₃-C₆), chacun substitué par m radicaux du groupe constitué par fluor, chlore, brome, iode, cyano et hydroxy ;
R⁶ signifie hydrogène ou R⁵;
R^{6a} signifie fluor, chlore, brome, iode, cyano, hydroxy, S(O)ₙR⁵, ou alcoxy en (C₁-C₆), alcényloxy en (C₂-C₆) ou alcynyloxy en (C₂-C₆), chacun substitué par m radicaux du groupe constitué par fluor, chlore, brome, cyano et alcoxy en (C₁-C₂) ;
R⁷ signifie hydrogène ou alkyle en (C₁-C₆), cycloalkyle en (C₃-C₆), alcényle en (C₂-C₄) ou alcynyle en (C₂-C₄), chacun substitué par m radicaux du groupe constitué par fluor, chlore, brome, cyano et alcoxy en (C₁-C₂) ;
R⁸ signifie R⁷ ;
R⁹ signifie alkyle en (C₁-C₃) ou alcoxy en (C₁-C₃) ;
k signifie 3, 4, 5 ou 6 ;
m signife 0, 1, 2, 3, 4 ou 5 ;
n signifie 0, 1 ou 2 ;
p signifie 0 ou 1 ;
q signifie 3, 4 ou 5 ;
s signifie 0, 1, 2, 3 ou 4.

3. 3-Phénylisoxazoline-5-carboxamides à substitution 5-oxy et 3-phénylisoxazoline-5-thioamides à substitution 5-oxy selon la revendication 1 ou 2,
dans lesquels
R¹ et R² signifient chacun indépendamment l'un de l'autre hydrogène, fluor, chlore, brome, iode, cyano, ou alkyle en (C₁-C₄) substitué par m radicaux du groupe constitué par fluor, chlore, brome, iode et cyano ;
R³ signifie alkyle en (C₁-C₄), cycloalkyle en (C₃-C₄), alcényle en (C₂-C₃) ou alcynyle en (C₂-C₃), chacun substitué par m radicaux du groupe constitué par fluor, chlore, brome, cyano, alcoxy en (C₁-C₂) ;
A signifie une liaison ou une unité bivalente du groupe constitué par CH₂, CH₂CH₂, CHCH₃, CH₂CH₂CH₂, CH(CH₂CH₃), CH(CH₃)CH₂, C(CH₃)₂, C(CH₃)₂CH₂, C(iPr)CH₃, CH(CH₂iPr)CH₂, CH₂CH=CH, C(CH₃)₂C≡C, CH(CF₃)CH₂, CH(CH₃)CH₂O, CH₂CH₂O, CH(cPr)CH₂O, CH(CH₂OCH₃), CH (CH₂CH₂SCH₃) CH(COOH), CH(COOCH₃), CH(COOH)CH₂, CH (COOCH₃) CH₂, CH₂COH(CF₃), CH(CONHCH₃), CH(CONHCH₃)CH₂ et CH₂CH₂CONHCH₂ ;
R⁴ signifie hydrogène ou alkyle en (C₁-C₈) ;
Y signifie oxygène ou soufre ;
X signifie hydrogène, cyano, hydroxy, X¹,
ou
alkyle en (C₁-C₁₂), cycloalkyle en (C₃-C₈), alcényle en (C₂-C₁₂) ou alcynyle en (C₂-C₁₂), chacun substitué par m radicaux du groupe constitué par fluor, chlore, cyano, hydroxy, OR⁷, X¹, OX¹, NHX¹, S(O)ₙR⁵, CO₂R⁸, CONR⁶R⁸, CONR⁸SO₂R⁵ et POR⁹R⁹,
X¹ signifie un cycle substitué par s radicaux du groupe constitué par R⁶, R^{6a}, R⁸ et R⁹, du groupe constitué par 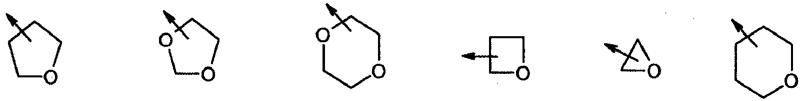 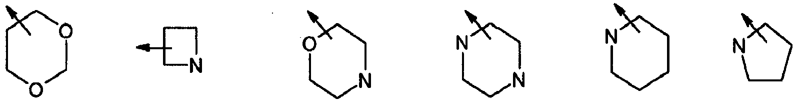 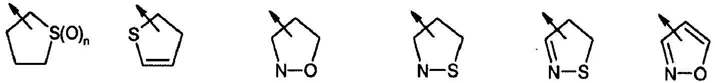 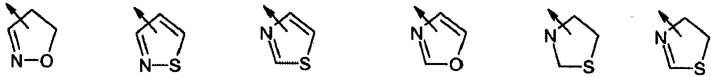 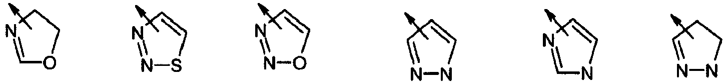 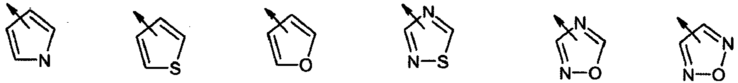 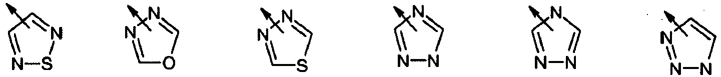 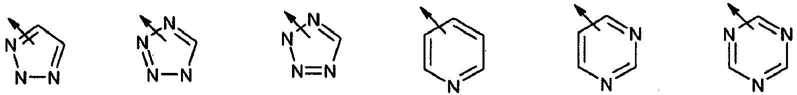 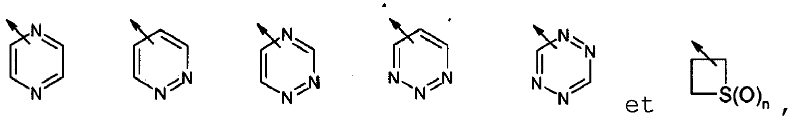
ou X¹ signifie phényle substitué par m radicaux du groupe constitué par R⁶, R^{6a}, R⁸ et R⁹ ;
X², X⁴ et X⁶ signifient chacun indépendamment les uns des autres hydrogène, fluor ou chlore,
ou alkyle en (C₁-C₄) ou alcoxy en (C₁-C₄), chacun substitué par m radicaux du groupe constitué par fluor, chlore, cyano et alcoxy en (C₁-C₄) ;
X³ et X⁵ signifient indépendamment l'un de l'autre hydrogène, fluor, chlore, brome, cyano,
ou alkyle en (C₁-C₆) substitué par m radicaux du groupe constitué par fluor et chlore,
ou alcoxy en (C₁-C₆) substitué par m radicaux du groupe constitué par fluor et chlore ;
R⁵ signifie méthyle ou éthyle ;
R⁶ signifie hydrogène ou R⁵;
R^{6a} signifie fluor, chlore, brome, iode, cyano, hydroxy, S(O)ₙR⁵, ou alcoxy en (C₁-C₆), alcényloxy en (C₂-C₆)ou alcynyloxy en (C₂-C₆), chacun substitué par m radicaux du groupe constitué par fluor, chlore, brome, cyano et alcoxy en (C₁-C₂) ;
R⁷ signifie hydrogène ou alkyle en (C₁-C₆) substitué par m radicaux du groupe constitué par fluor et chlore ;
R⁸ signifie R⁷ ;
R⁹ signifie alcoxy en (C₁-C₃) ;
m signife 0, 1, 2 ou 3 ;
n signifie 0, 1 ou 2 ;
s signifie 0, 1, 2, 3 ou 4.

4. Agents herbicides, **caractérisés par** une teneur herbicide efficace en au moins un composé de formule (I) selon l'une quelconque des revendications 1 à 3.

5. Agents herbicides selon la revendication 4, en mélange avec des adjuvants de formulation.

6. Agents herbicides selon la revendication 4 ou 5, contenant au moins une autre substance à effet pesticide du groupe constitué par les insecticides, les acaricides, les herbicides, les fongicides, les agents protecteurs et les régulateurs de croissance.

7. Agents herbicides selon la revendication 6, contenant un agent protecteur.

8. Agents herbicides selon la revendication 7, dans lesquels l'agent protecteur est choisi dans le groupe constitué par le méfenpyr-diéthyle, le cyprosulfamide, l'isoxadifène-éthyle, le cloquintocet-mexyle, le benoxacor et le dichlormid.

9. Agents herbicides selon l'une quelconque des revendications 6 à 8, contenant un herbicide supplémentaire.

10. Procédé de lutte contre des plantes indésirables, **caractérisé en ce qu'**une quantité efficace d'au moins un composé de formule (I) selon l'une quelconque des revendications 1 à 3 ou d'un agent herbicide selon l'une quelconque des revendications 4 à 9 est appliquée sur les plantes ou à l'emplacement de la végétation indésirable.

11. Utilisation de composés de formule (I) selon l'une quelconque des revendications 1 à 3 ou d'agents herbicides selon l'une quelconque des revendications 4 à 9 pour lutter contre des plantes indésirables.

12. Utilisation selon l'une quelconque des revendications 10 ou 11, **caractérisée en ce que** les composés de formule (I) sont utilisés pour lutter contre des plantes indésirables dans des cultures de plantes utiles.

13. Utilisation selon la revendication 12, **caractérisée en ce que** les plantes utiles sont des plantes utiles transgéniques.

14. Agents fongicides, **caractérisés par** une teneur fongicide efficace en au moins un composé de formule (I) selon l'une quelconque des revendications 1 à 3.

15. Agents fongicides selon la revendication 14, en mélange avec des adjuvants de formulation.

16. Agents fongicides selon la revendication 14 ou 15, contenant au moins une autre substance à effet pesticide du groupe constitué par les insecticides, les acaricides, les herbicides, les fongicides, les agents protecteurs et les régulateurs de croissance.

17. Composés de formule (II) 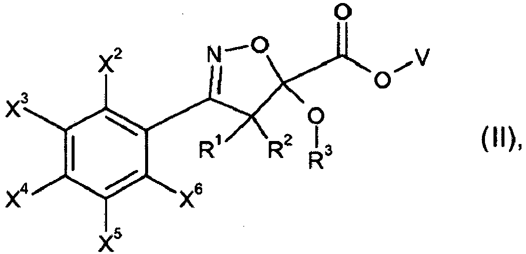 dans lesquels
V signifie hydrogène ou R⁵, et
X¹, X², X³, X⁴, X⁵, X⁶, R¹, R², R³ et R⁵ sont tels que définis dans l'une quelconque des revendications 1 à 3.
